# EUROPEAN PATENT SPECIFICATION

(11) **EP 0 953 052 B1**
(45) Date of publication and mention of the grant of the patent: **04.03.2009**
(21) Application number: 97924603.0
(22) Date of filing: 05.05.1997
(51) Int. Cl.: C12N 15/86

(54) **CROSSLESS RETROVIRAL VECTORS**
REKOMBINATIONSUNFÄHIGE RETROVIRALE VEKTOREN
VECTEURS RETROVIRAUX SANS CROISEMENT

(30) Priority: 06.05.1996 US 643411; 26.09.1996 US 721327
(43) Date of publication of application: 03.11.1999
(73) Proprietor: Oxford BioMedica (UK) Limited, Oxford Science Park Oxford OX4 4GA (GB)
(72) Inventor: RESPESS, James, G., San Diego, CA 92109 (US); DePOLO, Nicholas, J., Solana Beach, CA 92075 (US); CHADA, Sunil, Vista, CA 92083 (US); SAUTER, Sybille, Del Mar, CA 92014 (US); BODNER, Mordechai, San Diego, CA 92129 (US); DRIVER, David, A., San Diego, CA 92117 (US)
(74) Representative: Mallalieu, Catherine Louise
(86) International application number: PCT/US1997/007697
(87) International publication number: WO 1997/042338

(56) References cited:
- WO-A-89/07150
- WO-A-92/05266
- WO-A-94/29438
- WO-A-95/30763
- WO-A-95/31566
- WO-A-96/07749
- BIOTECHNIQUES, vol. 7, no. 9, October 1989, pages 980-990, XP000606889 MILLER A D ET AL: "IMPROVED RETROVIRAL VECTORS FOR GENE TRANSFER AND EXPRESSION"
- J. VIROLOGY, vol. 61, no. 5, 19 May 1987, AM.SOC.MICROBIOL.,WASHINGTON,US, pages 1639-1646, XP002036903 M.A. BENDER ET AL.: "Evidence that the packaging signal of Moloney Murine Leukemia Virus extends into the gag region"
- MOLECULAR AND CELLULAR BIOLOGY, vol. 7, no. 5, 1 May 1987, pages 1797-1806, XP002036904 BOSSELMAN R A ET AL: "REPLICATION-DEFICTIVE CHIMERIC HELPER PROVIRUSES AND FACTORS AFFECTING GENERATION OF COMPETENT VIRUS: EXPRESSION OF MOLONEY MURINA LEUKEMIA VIRUS STRUCTURAL GENES VIA THE METALLOTHIONEIN PROMOTER" cited in the application
- PROCEEDINGS OF THE NATIONAL ACADEMY OF SCIENCES OF USA, vol. 85, no. 17, 1 September 1988, pages 6460-6464, XP002036905 DANOS O ET AL: "SAFE AND EFFICIENT GENERATION OF RECOMBINANT RETROVIRUSES WITH AMPHOTROPIC AND ECOTROPIC HOST RANGES" cited in the application
- JOURNAL OF VIROLOGY, vol. 62, no. 4, 1 April 1988, pages 1120-1124, XP000562362 MARKOWITZ D ET AL: "A SAFE PACKAGING LINE FOR GENE TRANSFER: SEPARATING VIRAL GENES ON TWO DIFFERENT PLASMIDS" cited in the application

### SEQUENCE LISTING

(1) GENERAL INFORMATION:
   (i) APPLICANTS: Respess. Jams G.
      De Polo. Nicholas J.
      Chada, Sunil
      Sauter. Sybille
      Bodner. Mordechai
      Driver, David A.
   (ii) TITLE OF INVENTION: CROSSLESS RETROVIRAL VECTORS
   (iii) NUMBER OF SEQUENCES: 45
   (iv) CORRESPONDENCE ADDRESS:
      (A) ADDRESSEE: CHIRON CORPORATION
      (B) STREET: INTELLECTUAL.PROPERTY-R440 P.O. BOX 8097
      (C) CITY: EMERYVILLE
      (D) STATE: CA
      (E) COUNTRY: USA
      (F) ZIP: 94662-8097
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. Version #1.25
   (vi) CURRENT APPLICATION DATA:
      (A) APPLICATION NUMBER:
      (B) FILING DATE: 06-MAY-1997
      (C) CLASSIFICATION:
   (viii) ATTORNEY/AGENT INFORMATION:
      (A) NAME: KRUSE. NORMAN J.
      (B) REGISTRATION NUMBER: 35.235
      (C) REFERENCE/DOCKET NUMBER: 930049.42402PC
   (ix) TELECOMMUNICATION INFORMATION:
      (A) TELEPHONE: (206)622-4900
      (B) TELEFAX: (206)682-6031
(2) INFORMATION FOR SEQ ID NO:1:
   (i) SEQUENCE CHARACTERISTICS:
      (A) LENGTH: 8332 base pairs
      (B) TYPE: nucleic acid
      (C) STRANDEDNESS: single
      (D) TOPOLOGY: linear
   (xi) SEQUENCE DESCRIPTION: SEQ ID NO:1:
   GCGCCAGTCC TCCGATTGAC TGAGTCGCCC GGGTACCCGT GTATCCAATA AACCCTCTTG 60
   CAGTTGCATC CGACTTGTGG TCTCGCTGTT CCTTGGGAGG GTCTCCTCTG AGTGATTGAC 120
   TACCCGTCAG CGGGGGTCTT TCATTTGGGG GCTCGTCCGG GATCGGGAGA CCCCTGCCCA 180
   GGGACCACCG ACCCACCACC GGGAGGTAAG CTGGCCAGCA ACTTATCTGT GTCTGTCCGA 240
   TTGTCTAGTG TCTATGACTG ATTTTATGCG CCTGCGTCGG TACTAGTTAG CTAACTAGCT 300
   CTGTATCTGG CGGACCCGTG GTGGAACTGA CGAGTTCGGA ACACCCGGCC GCAACCCTGG 360
   GAGACGTCCC AGGGACTTCG GGGGCCGTTT TTGTGGCCCG ACCTGAGTCC AAAAATCCCG 420
   ATCGTTTTGG ACTCTTTGGT GCACCCCCCT TAGAGGAGGG ATATGTGGTT CTGGTAGGAG 480
   ACGAGAACCT AAAACAGTTC CCGCCTCCGT CTGAATTTTT GCTTTCGGTT TGGGACCGAA 540
   GCCGCGCCGC GCGTCTTGTC TGCTGCAGCA TCGTTCTGTG TTGTCTCTGT CTGACTGTGT 600
   TTCTGTATTT GTCTGAGAAT ATGGGCCAGA CTGTTACCAC TCCCTTAAGT TTGACCTTAG 660
   GTCACTGGAA AGATGTCGAG CGGATCGCTC ACAACCAGTC GGTAGATGTC AAGAAGAGAC 720
   GTTGGGTTAC CTTCTGCTCT GCAGAATGGC CAACCTTTAA CGTCGGATGG CCGCGAGACG 780
   GCACCTTTAA CCGAGACCTC ATCACCCAGG TTAAGATCAA GGTCTTTTCA CCTGGCCCGC 840
   ATGGACACCC AGACCAGGTC CCCTACATCG TGACCTGGGA AGCCTTGGCT TTTGACCCCC 900
   CTCCCTGGGT CAAGCCCTTT GTACACCCTA AGCCTCCGCC TCCTCTTCCT CCATCCGCCC 960
   CGTCTCTCCC CCTTGAACCT CCTCGTTCGA CCCCGCCTCG ATCCTCCCTT TATCCAGCCC 1020
   TCACTCCTTC TCTAGGCGCC AAACCTAAAC CTCAAGTTCT TTCTGACAGT GGGGGGCCGC 1080
   TCATCGACCT ACTTACAGAA GACCCCCCGC CTTATAGGGA CCCAAGACCA CCCCCTTCCG 1140
   ACAGGGACGG AAATGGTGGA GAAGCGACCC CTGCGGGAGA GGCACCGGAC CCCTCCCCAA 1200
   TGGCATCTCG CCTACGTGGG AGACGGGAGC CCCCTGTGGC CGACTCCACT ACCTCGCAGG 1260
   CATTCCCCCT CCGCGCAGGA GGAAACGGAC AGCTTCAATA CTGGCCGTTC TCCTCTTCTG 1320
   ACCTTTACAA CTGGAAAAAT AATAACCCTT CTTTTTCTGA AGATCCAGGT AAACTGACAG 1380
   CTCTGATCGA GTCTGTTCTC ATCACCCATC AGCCCACCTG GGACGACTGT CAGCAGCTGT 1440
   TGGGGACTCT GCTGACCGGA GAAGAAAAAC AACGGGTGCT CTTAGAGGCT AGAAAGGCGG 1500
   TGCGGGGCGA TGATGGGCGC CCCACTCAAC TGCCCAATGA AGTCGATGCC GCTTTTCCCC 1560
   TCGAGCGCCC AGACTGGGAT TACACCACCC AGGCAGGTAG GAACCACCTA GTCCACTATC 1620
   GCCAGTTGCT CCTAGCGGGT CTCCAAAACG CGGGCAGAAG CCCCACCAAT TTGGCCAAGG 1680
   TAAAAGGAAT AACACAAGGG CCCAATGAGT CTCCCTCGGC CTTCCTAGAG AGACTTAAGG 1740
   AAGCCTATCG CAGGTACACT CCTTATGACC CTGAGGACCC AGGGCAAGAA ACTAATGTGT 1800
   CTATGTCTTT CATTTGGCAG TCTGCCCCAG ACATTGGGAG AAAGTTAGAG AGGTTAGAAG 1860
   ATTTAAAAAA CAAGACGCTT GGAGATTTGG TTAGAGAGGC AGAAAAGATC TTTAATAAAC 1920
   GAGAAACCCC GGAAGAAAGA GAGGAACGTA TCAGGAGAGA AACAGAGGAA AAAGAAGAAC 1980
   GCCGTAGGAC AGAGGATGAG CAGAAAGAGA AAGAAAGAGA TCGTAGGAGA CATAGAGAGA 2040
   TGAGCAAGCT ATTGGCCACT GTCGTTAGTG GACAGAAACA GGATAGACAG GGAGGAGAAC 2100
   GAAGGAGGTC CCAACTCGAT CGCGACCAGT GTGCCTACTG CAAAGAAAAG GGGCACTGGG 2160
   CTAAAGATTG TCCCAAGAAA CCACGAGGAC CTCGGGGACC AAGACCCCAG ACCTCCCTCC 2220
   TGACCCTAGA TGACTAGGGA GGTCAGGGTC AGGAGCCCCC CCCTGAACCC AGGATAACCC 2280
   TCAAAGTCGG GGGGCAACCC GTCACCTTCC TGGTAGATAC TGGGGCCCAA CACTCCGTGC 2340
   TGACCCAAAA TCCTGGACCC CTAAGTGATA AGTCTGCCTG GGTCCAAGGG GCTACTGGAG 2400
   GAAAGCGGTA TCGCTGGACC ACGGATCGCA AAGTACATCT AGCTACCGGT AAGGTCACCC 2460
   ACTCTTTCCT CCATGTACCA GACTGTCCCT ATCCTCTGTT AGGAAGAGAT TTGCTGACTA 2520
   AACTAAAAGC CCAAATCCAC TTTGAGGGAT CAGGAGCTCA GGTTATGGGA CCAATGGGGC 2580
   AGCCCCTGCA AGTGTTGACC CTAAATATAG AAGATGAGCA TCGGCTACAT GAGACCTCAA 2640
   AAGAGCCAGA TGTTTCTCTA GGGTCCACAT GGCTGTCTGA TTTTCCTCAG GCCTGGGCGG 2700
   AAACCGGGGG CATGGGACTG GCAGTTCGCC AAGCTCCTCT GATCATACCT CTGAAAGCAA 2760
   CCTCTACCCC CGTGTCCATA AAACAATACC CCATGTCACA AGAAGCCAGA CTGGGGATCA 2820
   AGCCCCACAT ACAGAGACTG TTGGACCAGG GAATACTGGT ACCCTGCCAG TCCCCCTGGA 2880
   ACACGCCCCT GCTACCCGTT AAGAAACCAG GGACTAATGA TTATAGGCCT GTCCAGGATC 2940
   TGAGAGAAGT CAACAAGCGG GTGGAAGACA TCCACCCCAC CGTGCCCAAC CCTTACAACC 3000
   TCTTGAGCGG GCTCCCACCG TCCCACCAGT GGTACACTGT GCTTGATTTA AAGGATGCCT 3060
   TTTTCTGCCT GAGACTCCAC CCCACCAGTC AGCCTCTCTT CGCCTTTGAG TGGAGAGATC 3120
   CAGAGATGGG AATCTCAGGA CAATTGACCT GGACCAGACT CCCACAGGGT TTCAAAAACA 3180
   GTCCCACCCT GTTTGATGAG GCACTGCACA GAGACCTAGC AGACTTCCGG ATCCAGCACC 3240
   CAGACTTGAT CCTGCTACAG TACGTGGATG ACTTACTGCT GGCCGCCACT TCTGAGCTAG 3300
   ACTGCCAACA AGGTACTCGG GCCCTGTTAC AAACCCTAGG GAACCTCGGG TATCGGGCCT 3360
   CGGCCAAGAA AGCCCAAATT TGCCAGAAAC AGGTCAAGTA TCTGGGGTAT CTTCTAAAAG 3420
   AGGGTCAGAG ATGGCTGACT GAGGCCAGAA AAGAGACTGT GATGGGGCAG CCTACTCCGA 3480
   AGACCCCTCG ACAACTAAGG GAGTTCCTAG GGACGGCAGG CTTCTGTCGC CTCTGGATCC 3540
   CTGGGTTTGC AGAAATGGCA GCCCCCTTGT ACCCTCTCAC CAAAACGGGG ACTCTGTTTA 3600
   ATTGGGGCCC AGACCAACAA AAGGCCTATC AAGAAATCAA GCAAGCTCTT CTAACTGCCC 3660
   CAGCCCTGGG GTTGCCAGAT TTGACTAAGC CCTTTGAACT CTTTGTCGAC GAGAAGCAGG 3720
   GCTACGCCAA AGGTGTCCTA ACGCAAAAAC TGGGACCTTG GCGTCGGCCG GTGGCCTACC 3780
   TGTCCAAAAA GCTAGACCCA GTAGCAGCTG GGTGGCCCCC TTGCCTACGG ATGGTAGCAG 3840
   CCATTGCCGT ACTGACAAAG GATGCAGGCA AGCTAACCAT GGGACAGCCA CTAGTCATTC 3900
   TGGCCCCCCA TGCAGTAGAG GCACTAGTCA AACAACCCCC CGACCGCTGG CTTTCCAACG 3960
   CCCGGATGAC TCACTATCAG GCCTTGCTTT TGGACACGGA CCGGGTCCAG TTCGGACCGG 4020
   TGGTAGCCCT GAACCCGGCT ACGCTGCTCC CACTGCCTGA GGAAGGGCTG CAACACAACT 4080
   GCCTTGATAT CCTGGCCGAA GCCCACGGAA CCCGACCCGA CCTAACGGAC CAGCCGCTCC 4140
   CAGACGCCGA CCACACCTGG TACACGGATG GAAGCAGTCT CTTACAAGAG GGACAGCGTA 4200
   AGGCGGGAGC TGCGGTGACC ACCGAGACCG AGGTAATCTG GGCTAAAGCC CTGCCAGCCG 4260
   GGACATCCGC TCAGCGGGCT GAACTGATAG CACTCACCCA GGCCCTAAAG ATGGCAGAAG 4320
   GTAAGAAGCT AAATGTTTAT ACTGATAGCC GTTATGCTTT TGCTACTGCC CATATCCATG 4380
   GAGAAATATA CAGAAGGCGT GGGTTGCTCA CATCAGAAGG CAAAGAGATC AAAAATAAAG 4440
   ACGAGATCTT GGCCCTACTA AAAGCCCTCT TTCTGCCCAA AAGACTTAGC ATAATCCATT 4500
   GTCCAGGACA TCAAAAGGGA CACAGCGCCG AGGCTAGAGG CAACCGGATG GCTGACCAAG 4560
   CGGCCCGAAA GGCAGCCATC ACAGAGACTC CAGACACCTC TACCCTCCTC ATAGAAAATT 4620
   CATCACCCTA CACCTCAGAA CATTTTCATT ACACAGTGAC TGATATAAAG GACCTAACCA 4680
   AGTTGGGGGC CATTTATGAT AAAACAAAGA AGTATTGGGT CTACCAAGGA AAACCTGTGA 4740
   TGCCTGACCA GTTTACTTTT GAATTATTAG ACTTTCTTCA TCAGCTGACT CACCTCAGCT 4800
   TCTCAAAAAT GAAGGCTCTC CTAGAGAGAA GCCACAGTCC CTACTACATG CTGAACCGGG 4860
   ATCGAACACT CAAAAATATC ACTGAGACCT GCAAAGCTTG TGCACAAGTC AACGCCAGCA 4920
   AGTCTGCCGT TAAACAGGGA ACTAGGGTCC GCGGGCATCG GCCCGGCACT CATTGGGAGA 4980
   TCGATTTCAC CGAGATAAAG CCCGGATTGT ATGGCTATAA ATATCTTCTA GTTTTTATAG 5040
   ATACCTTTTC TGGCTGGATA GAAGCCTTCC CAACCAAGAA AGAAACCGCC AAGGTCGTAA 5100
   CCAAGAAGCT ACTAGAGGAG ATCTTCCCCA GGTTCGGCAT GCCTCAGGTA TTGGGAACTG 5160
   ACAATGGGCC TGCCTTCGTC TCCAAGGTGA GTCAGACAGT GGCCGATCTG TTGGGGATTG 5220
   ATTGGAAATT ACATTGTGCA TACAGACCCC AAAGCTCAGG CCAGGTAGAA AGAATGAATA 5280
   GAACCATCAA GGAGACTTTA ACTAAATTAA CGCTTGCAAC TGGCTCTAGA GACTGGGTGC 5340
   TCCTACTCCC CTTAGCCCTG TACCGAGCCC GCAACACGCC GGGCCCCCAT GGCCTCACCC 5400
   CATATGAGAT CTTATATGGG GCACCCCCGC CCCTTGTAAA CTTCCCTGAC CCTGACATGA 5460
   CAAGAGTTAC TAACAGCCCC TCTCTCCAAG CTCACTTACA GGCTCTCTAC TTAGTCCAGC 5520
   ACGAAGTCTG GAGACCTCTG GCGGCAGCCT ACCAAGAACA ACTGGACCGA CCGGTGGTAC 5580
   CTCACCCTTA CCGAGTCGGC GACACAGTGT GGGTCCGCCG ACACCAGACT AAGAACCTAG 5640
   AACCTCGCTG GAAAGGACCT TACACAGTCC TGCTGACCAC CCCCACCGCC CTCAAAGTAG 5700
   ACGGCATCGC AGCTTGGATA CACGCCGCCC ACGTGAAGGC TGCCGACCCC GGGGGTGGAC 5760
   CATCCTCTAG ACTGACATGG CGCGTTCAAC GCTCTCAAAA CCCCTTAAAA ATAAGGTTAA 5820
   CCCGCGAGGC CCCCTAATCC CCTTAATTCT TCTGATGCTC AGAGGGGTCA GTACTGCTTC 5880
   GCCCGGCTCC AGTCCTCATC AAGTCTATAA TATCACCTGG GAGGTAACCA ATGGAGATCG 5940
   GGAGACGGTA TGGGCAACTT CTGGCAACCA CCCTCTGTGG ACCTGGTGGC CTGACCTTAC 6000
   CCCAGATTTA TGTATGTTAG CCCACCATGG ACCATCTTAT TGGGGGCTAG AATATCAATC 6060
   CCCTTTTTCT TCTCCCCCGG GGCCCCCTTG TTGCTCAGGG GGCAGCAGCC CAGGCTGTTC 6120
   CAGAGACTGC GAAGAACCTT TAACCTCCCT CACCCCTCGG TGCAACACTG CCTGGAACAG 6180
   ACTCAAGCTA GACCAGACAA CTCATAAATC AAATGAGGGA TTTTATGTTT GCCCCGGGCC 6240
   CCACCGCCCC CGAGAATCCA AGTCATGTGG GGGTCCAGAC TCCTTCTACT GTGCCTATTG 6300
   GGGCTGTGAG ACAACCGGTA GAGCTTACTG GAAGCCCTCC TCATCATGGG ATTTCATCAC 6360
   AGTAAACAAC AATCTCACCT CTGACCAGGC TGTCCAGGTA TGCAAAGATA ATAAGTGGTG 6420
   CAACCCCTTA GTTATTCGGT TTACAGACGC CGGGAGACGG GTTACTTCCT GGACCACAGG 6480
   ACATTACTGG GGCTTACGTT TGTATGTCTC CGGACAAGAT CCAGGGCTTA CATTTGGGAT 6540
   CCGACTCAGA TACCAAAATC TAGGACCCCG CGTCCCAATA GGGCCAAACC CCGTTCTGGC 6600
   AGACCAACAG CCACTCTCCA AGCCCAAACC TGTTAAGTCG CCTTCAGTCA CCAAACCACC 6660
   CAGTGGGACT CCTCTCTCCC CTACCCAACT TCCACCGGCG GGAACGGAAA ATAGGCTGCT 6720
   AAACTTAGTA GACGGAGCCT ACCAAGCCCT CAACCTCACC AGTCCTGACA AAACCCAAGA 6780
   GTGCTGGTTG TGTCTAGTAG CGGGACCCCC CTACTACGAA GGGGTTGCCG TCCTGGGTAC 6840
   CTACTCCAAC CATACCTCTG CTCCAGCCAA CTGCTCCGTG GCCTCCCAAC ACAAGTTGAC 6900
   CCTGTCCGAA GTGACCGGAC AGGGACTCTG CATAGGAGCA GTTCCCAAAA CACATCAGGC 6960
   CCTATGTAAT ACCACCCAGA CAAGCAGTCG AGGGTCCTAT TATCTAGTTG CCCCTACAGG 7020
   TACCATGTGG GCTTGTAGTA CCGGGCTTAC TCCATGCATC TCCACCACCA TACTGAACCT 7080
   TACCACTGAT TATTGTGTTC TTGTCGAACT CTGGCCAAGA GTCACCTATC ATTCCCCCAG 7140
   CTATGTTTAC GGCCTGTTTG AGAGATCCAA CCGACACAAA AGAGAACCGG TGTCGTTAAC 7200
   CCTGGCCCTA TTATTGGGTG GACTAACCAT GGGGGGAATT GCCGCTGGAA TAGGAACAGG 7260
   GACTACTGCT CTAATGGCCA CTCAGCAATT CCAGCAGCTC CAAGCCGCAG TACAGGATGA 7320
   TCTCAGGGAG GTTGAAAAAT CAATCTCTAA CCTAGAAAAG TCTCTCACTT CCCTGTCTGA 7380
   AGTTGTCCTA CAGAATCGAA GGGGCCTAGA CTTGTTATTT CTAAAAGAAG GAGGGCTGTG 7440
   TGCTGCTCTA AAAGAAGAAT GTTGCTTCTA TGCGGACCAC ACAGGACTAG TGAGAGACAG 7500
   CATGGCCAAA TTGAGAGAGA GGCTTAATCA GAGACAGAAA CTGTTTGAGT CAACTCAAGG 7560
   ATGGTTTGAG GGACTGTTTA ACAGATCCCC TTGGTTTACC ACCTTGATAT CTACCATTAT 7620
   GGGACCCCTC ATTGTACTCC TAATGATTTT GCTCTTCGGA CCCTGCATTC TTAATCGATT 7680
   AGTCCAATTT GTTAAAGACA GGATATCAGT GGTCCAGGCT CTAGTTTTGA CTCAACAATA 7740
   TCACCAGCTG AAGCCTATAG AGTACGAGCC ATAGATAAAA TAAAAGATTT TATTTAGTCT 7800
   CCAGAAAAAG GGGGGAATGA AAGACCCCAC CTGTAGGTTT GGCAAGCTAG CTTAAGTAAC 7860
   GCCATTTTGC AAGGCATGGA AAAATACATA ACTGAGAATA GAGAAGTTCA GATCAAGGTC 7920
   AGGAACAGAT GGAACAGCTG AATATGGGCC AAACAGGATA TCTGTGGTAA GCAGTTCCTG 7980
   CCCCGGCTCA GGGCCAAGAA CAGATGGAAC AGCTGAATAT GGGCCAAACA GGATATCTGT 8040
   GGTAAGCAGT TCCTGCCCCG GCTCAGGGCC AAGAACAGAT GGTCCCCAGA TGCGGTCCAG 8100
   CCCTCAGCAG TTTCTAGAGA ACCATCAGAT GTTTCCAGGG TGCCCCAAGG ACCTGAAATG 8160
   ACCCTGTGCC TTATTTGAAC TAACCAATCA GTTCGCTTCT CGCTTCTGTT CGCGCGCTTC 8220
   TGCTCCCCGA GCTCAATAAA AGAGCCCACA ACCCCTCACT CGGGGCGCCA GTCCTCCGAT 8280
   TGACTGAGTC GCCCGGGTAC CCGTGTATCC AATAAACCCT CTTGCAGTTG CA 8332
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:2:
      GGTAACAGTC TGGCCCGAAT TCTCAGACAA ATACAG 36
(2) INFORMATION FOR SEQ ID NO:3:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:3:
      CTGTATTTGT CTGAGAATTA AGGCTAGACT GTTACCAC 38
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:4:
      ATATATATAT ATCGATACCA TG 22
(2) INFORMATION FOR SEQ ID NO:5:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:5:
      GGCGCCAAAC CTAAAC 16
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (xi) SEQUENCE DESCRIPTION: SEQ ID NO:6:
      Ser Lys Asn Tyr Pro 5
(2) INFORMATION FOR SEQ ID NO:7:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:7:
      ACCATCCTCT GGACGGACAT G 21
(2) INFORMATION FOR SEQ ID NO:8:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:8:
      ACCCGGCCGT GGACGGACAT G 21
(2) INFORMATION FOR SEQ ID NO:9:
   (i) SEQUENCE CHARACTERISTICS:
      (A) LENGTH: 449 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 20..442
   (xi) SEQUENCE DESCRIPTION: SEQ ID NO:9:
   ATATATATAT ATCGATACC ATG GGG CAA ACC GTG ACT ACC CCT CTG TCC CTC      52
                        Met Gly Gln Thr Val Thr Thr Pro Leu Ser Leu
                         1               5                  10
   ACA CTG GGC CAT TGG AAG GAC GTG GAA AGA ATT GCC CAT AAT CAA AGC      100
   Thr Leu Gly His Trp Lys Asp Val Glu Arg Ile Ala His Asn Gln Ser
               15                  20                  25
   GTG GAC GTC AAA AAA CGC AGG TGG GTG ACA TTT TGT AGC GCC GAG TGG      148
   Val Asp Val Lys Lys Arg Arg Trp Val Thr Phe Cys Ser Ala Glu Trp
           30                  35                  40
   CCC ACA TTC AAT GTT GGC TGG CCT AGG GAT GGA ACT TTC AAT CGC GAT      196
   Pro Thr Phe Asn Val Gly Trp Pro Arg Asp Gly Thr Phe Asn Arg Asp
       45                  50                  55
   CTG ATT ACT CAA GTG AAA ATT AAA GTG TTC AGC CCC GGA CCC CAC GGC      244
   Leu Ile Thr Gln Val Lys Ile Lys Val Phe Ser Pro Gly Pro His Gly
    60                  65                  70                  75
   CAT CCC GAT CAA GTT CCT TAT ATT GTC ACA TGG GAG GCT CTC GCT TTC      292
   His Pro Asp Gln Val Pro Tyr Ile Val Thr Trp Glu Ala Leu Ala Phe
                   80                  85                  90
   GAT CCA CCA CCT TGG GTG AAA CCA TTC GTG CAT CCC AAA CCA CCT CCA      340
   Asp Pro Pro Pro Trp Val Lys Pro Phe Val His Pro Lys Pro Pro Pro
               95                 100                 105
   CCC CTC CCA CCC AGC GCT CCT AGC CTG CCC TTG GAG CCC CCA CGA AGC      388
   Pro Leu Pro Pro Ser Ala Pro Ser Leu Pro Leu Glu Pro Pro Arg Ser
           110                 115                 120
   ACA CCA CCC AGG AGC AGC TTG TAC CCT GCT CTG ACC CCC AGC CTC GGC      436
   Thr Pro Pro Arg Ser Ser Leu Tyr Pro Ala Leu Thr Pro Ser Leu Gly
       125                 130                 135
   GCC AAA CCTAAAC                                                      449
   Ala Lys
   140
(2) INFORMATION FOR SEQ ID NO:10:
   (i) SEQUENCE CHARACTERISTICS:
      (A) LENGTH: 141 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Gly Gln Thr Val Thr Thr Pro Leu Ser Leu Thr Leu Gly His Trp
   1 5 10 15
   Lys Asp Val Glu Arg Ile Ala His Asn Gln Ser Val Asp Val Lys Lys
   20 25 30
   Arg Arg Trp Val Thr Phe Cys Ser Ala Glu Trp Pro Thr Phe Asn Val
   35 40 45
   Gly Trp Pro Arg Asp Gly Thr Phe Asn Arg Asp Leu Ile Thr Gln Val
   50 55 60
   Lys Ile Lys Val Phe Ser Pro Gly Pro His Gly His Pro Asp Gln Val
   65 70 75 80
   Pro Tyr Ile Val Thr Trp Glu Ala Leu Ala Phe Asp Pro Pro Pro Trp
   85 90 95
   Val Lys Pro Phe Val His Pro Lys Pro Pro Pro Pro Leu Pro Pro Ser
   100 105 110
   Ala Pro Ser Leu Pro Leu Glu Pro Pro Arg Ser Thr Pro Pro Arg Ser
   115 120 125
   Ser Leu Tyr Pro Ala Leu Thr Pro Ser Leu Gly Ala Lys
   130 135 140
(2) INFORMATION FOR SEQ ID NO:11:
   (i) SEQUENCE CHARACTERISTICS:
      (A) LENGTH: 420 base pairs
      (B) TYPE: nucleic acid
      (C) STRANDEDNESS: single
      (D) TOPOLOGY: linear
   (ix) FEATURE:
      (A) NAME/KEY: CDS
      (B) LOCATION: 1..420
   (xi) SEQUENCE DESCRIPTION: SEQ ID NO:11:
   ATG GGC CAG ACT GTT ACC ACT CCC TTA AGT TTG ACC TTA GGT CAC TGG       48
   Met Gly Gln Thr Val Thr Thr Pro Leu Ser Leu Thr Leu Gly His Trp
    1               5                  10                  15
   AAA GAT GTC GAG CGG ATC GCT CAC AAC CAG TCG GTA GAT GTC AAG AAG       96
   Lys Asp Val Glu Arg Ile Ala His Asn Gln Ser Val Asp Val Lys Lys
               20                  25                  30
   AGA CGT TGG GTT ACC TTC TGC TCT GCA GAA TGG CCA ACC TTT AAC GTC      144
   Arg Arg Trp Val Thr Phe Cys Ser Ala Glu Trp Pro Thr Phe Asn Val
           35                  40                  45
   GGA TGG CCG CGA GAC GGC ACC TTT AAC CGA GAC CTC ATC ACC CAG GTT      192
   Gly Trp Pro Arg Asp Gly Thr Phe Asn Arg Asp Leu Ile Thr Gln Val
       50                  55                  60
   AAG ATC AAG GTC TTT TCA CCT GGC CCG CAT GGA CAC CCA GAC CAG GTC      240
   Lys Ile Lys Val Phe Ser Pro Gly Pro His Gly His Pro Asp Gln Val
   65                  70                  75                  80
   CCC TAC ATC GTG ACC TGG GAA GCC TTG GCT TTT GAC CCC CCT CCC TGG      288
   Pro Tyr Ile Val Thr Trp Glu Ala Leu Ala Phe Asp Pro Pro Pro Trp
                   85                  90                  95
   GTC AAG CCC TTT GTA CAC CCT AAG CCT CCG CCT CCT CTT CCT CCA TCC      336
   Val Lys Pro Phe Val His Pro Lys Pro Pro Pro Pro Leu Pro Pro Ser
               100                 105                 110
   GCC CCG TCT CTC CCC CTT GAA CCT CCT CGT TCG ACC CCG CCT CGA TCC      384
   Ala Pro Ser Leu Pro Leu Glu Pro Pro Arg Ser Thr Pro Pro Arg Ser
           115                 120                 125
   TCC CTT TAT CCA GCC CTC ACT CCT TCT CTA GGC GCC                      420
   Ser Leu Tyr Pro Ala Leu Thr Pro Ser Leu Gly Ala
       130                 135                 140
(2) INFORMATION FOR SEQ ID NO:12:
   (i) SEQUENCE CHARACTERISTICS:
      (A) LENGTH: 140 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Gly Gln Thr Val Thr Thr Pro Leu Ser Leu Thr Leu Gly His Trp
   1               5                   10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
           35                  40                  45
   Gly Trp Pro Arg Asp Gly Thr Phe Asn Arg Asp Leu Ile Thr Gln Val
       50                  55                  60
   Lys Ile Lys Val Phe Ser Pro Gly Pro His Gly His Pro Asp Gln Val
   65                  70                  75                  80
   Pro Tyr Ile Val Thr Trp Glu Ala Leu Ala Phe Asp Pro Pro Pro Trp
                   85                  90                  95
   Val Lys Pro Phe Val His Pro Lys Pro Pro Pro Pro Leu Pro Pro Ser
               100                 105                 110
   Ala Pro Ser Leu Pro Leu Glu Pro Pro Arg Ser Thr Pro Pro Arg Ser
           115                 120                 125
   Ser Leu Tyr Pro Ala Leu Thr Pro Ser Leu Gly Ala
       130                 135                 140
(2) INFORMATION FOR SEQ ID NO:13:
   (i) SEQUENCE CHARACTERISTICS:
      (A) LENGTH: 2001 base pairs
      (B) TYPE: nucleic acid
      (C) STRANDEDNESS: single
      (D) TOPOLOGY: linear
   (xi) SEQUENCE DESCRIPTION: SEQ ID NO:13:
   GGCCGACACC CAGAGTGGAC CATCCTCTGG ACGGACATGG CGCGTTCAAC GCTCTCAAAA      60
   CCCCCTCAAG ATAAGATTAA CCCGTGGAAG CCCTTAATAG TCATGGGAGT CCTGTTAGGA     120
   GTAGGGATGG CAGAGAGCCC CCATCAGGTC TTTAATGTAA CCTGGAGAGT CACCAACCTG     180
   ATGACTGGGC GTACCGCCAA TGCCACCTCC CTCCTGGGAA CTGTACAAGA TGCCTTCCCA     240
   AAATTATATT TTGATCTATG TGATCTGGTC GGAGAGGAGT GGGACCCTTC AGACCAGGAA     300
   CCGTATGTCG GGTATGGCTG CAAGTACCCC GCAGGGAGAC AGCGGACCCG GACTTTTGAC 360
   TTTTACGTGT GCCCTGGGCA TACCGTAAAG TCGGGGTGTG GGGGACCAGG AGAGGGCTAC 420
   TGTGGTAAAT GGGGGTGTGA AACCACCGGA CAGGCTTACT GGAAGCCCAC ATCATCGTGG 480
   GACCTAATCT CCCTTAAGCG CGGTAACACC CCCTGGGACA CGGGATGCTC TAAAGTTGCC 540
   TGTGGCCCCT GCTACGACCT CTCCAAAGTA TCCAATTCCT TCCAAGGGGC TACTCGAGGG 600
   GGCAGATGCA ACCCTCTAGT CCTAGAATTC ACTGATGCAG GAAAAAAGGC TAACTGGGAC 660
   GGGCCCAAAT CGTGGGGACT GAGACTGTAC CGGACAGGAA CAGATCCTAT TACCATGTTC 720
   TCCCTGACCC GGCAGGTCCT TAATGTGGGA CCCCGAGTCC CCATAGGGCC CAACCCAGTA 780
   TTACCCGACC AAAGACTCCC TTCCTCACCA ATAGAGATTG TACCGGCTCC ACAGCCACCT 840
   AGCCCCCTCA ATACCAGTTA CCCCCCTTCC ACTACCAGTA CACCCTCAAC CTCCCCTACA 900
   AGTCCAAGTG TCCCACAGCC ACCCCCAGGA ACTGGAGATA GACTACTAGC TCTAGTCAAA 960
   GGAGCCTATC AGGCGCTTAA CCTCACCAAT CCCGACAAGA CCCAAGAATG TTGGCTGTGC 1020
   TTAGTGTCGG GACCTCCTTA TTACGAAGGA GTAGCGGTCG TGGGCACTTA TACCAATCAT 1080
   TCCACCGCTC CGGCCAACTG TACGGCCACT TCCCAACATA AGCTTACCCT ATCTGAAGTG 1140
   ACAGGACAGG GCCTATGCAT GGGGGCAGTA CCTAAAACTC ACCAGGCCTT ATGTAACACC 1200
   ACCCAAAGCG CCGGCTCAGG ATCCTACTAC CTTGCAGCAC CCGCCGGAAC AATGTGGGCT 1260
   TGCAGCACTG GATTGACTCC CTGCTTGTCC ACCACGGTGC TCAATCTAAC CACAGATTAT 1320
   TGTGTATTAG TTGAACTCTG GCCCAGAGTA ATTTACCACT CCCCCGATTA TATGTATGGT 1380
   CAGCTTGAAC AGCGTACCAA ATATAAAAGA GAGCCAGTAT CATTGACCCT GGCCCTTCTA 1440
   CTAGGAGGAT TAACCATGGG AGGGATTGCA GCTGGAATAG GGACGGGGAC CACTGCCTTA 1500
   ATTAAAACCC AGCAGTTTGA GCAGCTTCAT GCCGCTATCC AGACAGACCT CAACGAAGTC 1560
   GAAAAGTCAA TTACCAACCT AGAAAAGTCA CTGACCTCGT TGTCTGAAGT AGTCCTACAG 1620
   AACCGCAGAG GCCTAGATTT GCTATTCCTA AAGGAGGGAG GTCTCTGCGC AGCCCTAAAA 1680
   GAAGAATGTT GTTTTTATGC AGACCACACG GGGCTAGTGA GAGACAGCAT GGCCAAATTA 1740
   AGAGAAAGGC TTAATCAGAG ACAAAAACTA TTTGAGACAG GCCAAGGATG GTTCGAAGGG 1800
   CTGTTTAATA GATCCCCCTG GTTTACCACC TTAATCTCCA CCATCATGGG ACCTCTAATA 1860
   GTACTCTTAC TGATCTTACT CTTTGGACCT TGCATTCTCA ATCGATTGGT CCAATTTGTT 1920
   AAAGACAGGA TCTCAGTGGT CCAGGCTCTG GTTTTGACTC AGCAATATCA CCAGCTAAAA 1980
   CCCATAGAGT ACGAGCCATG A 2001
(2) INFORMATION FOR SEQ ID NO:14:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (xi) SEQUENCE DESCRIPTION: SEQ ID NO:14:
      CTAGCTAGCT AG 12
(2) INFORMATION FOR SEQ ID NO:15:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (xi) SEQUENCE DESCRIPTION: SEQ ID NO:15:
   ATATATATAT ATCGATACCA TGGGGCAAAC CGTGACTACC CCTCTGTCCC TCACACTGGC 60
   CCAA 64
(2) INFORMATION FOR SEQ ID NO:16:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:16:
      TTGATTATGG GCAATTCTTT CCACGTCCTT CCAATGGCCC AGTGTGAGGG AC 52
(2) INFORMATION FOR SEQ ID NO:17:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:17:
   AGAATTGCCC ATAATCAAAG CGTGGACGTC AAAAAACGCA GGTGGGTGAC ATTTTGTAGC 60
   GCCGAGTGGC CC 72
(2) INFORMATION FOR SEQ ID NO:18:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:18:
      AAGTTCCATC CCTAGGCCAG CCAACATTGA ATGTGGGCCA CTCGGCGCTA CA 52
(2) INFORMATION FOR SEQ ID NO:19:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:19:
   GGCCTAGGGA TGGAACTTTC AATCGCGATC TGATTACTCA AGTGAAAATT AAAGTGTTCA 60
   GCCCCGGACC CC 72
(2) INFORMATION FOR SEQ ID NO:20:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:20:
      GTGACAATAT AAGGAACTTG ATCGGGATGG CCGTGGGGTC CGGGGCTGAA CA 52
(2) INFORMATION FOR SEQ ID NO:21:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:21:
   AGTTCCTTAT ATTGTCACAT CGGAGGCTCT CGCTTTCGAT CCACCACCTT GGGTGAAACC 60
   ATTCGTGCAT CC 72
(2) INFORMATION FOR SEQ ID NO:22:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:22
      AGGAGCGCTG GGTGGGAGGG GTGGAGGTGG TTTGGGATGC ACGAATGGTT TC 52
(2) INFORMATION FOR SEQ ID NO:23
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO:23:
   CTCCCACCCA GCGCTCCTAG CCTGCCCTTG GAGCCCCCAC GAAGCACACC ACCCAGGAGC 60
   AGCTTGTACC CT 72
(2) INFORMATION FOR SEQ ID NO:24:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (xi) SEQUENCE DESCRIPTION: SEQ ID NO:24:
      GTTTAGGTTT GGCGCCGAGG CTGGGGGTCA GAGCAGGGTA CAAGCTGCTC CT 52
(2) INFORMATION FOR SEQ ID NO:25:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (xi) SEQUENCE DESCRIPTION: SEQ ID NO:25:
      ATATATATAT ATCGATACC 19
(2) INFORMATION FOR SEQ ID NO:26:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:26:
      GTTTAGGTTT GGCGCCGAGG 20
(2) INFORMATION FOR SEQ ID NO:27:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:27:
      GGGAGTGGTA ACAGTCTGGC CTTAATTCTC AG 32
(2) INFORMATION FOR SEQ ID NO:28:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:28:
      CGGTCGACCT CGAGAATTAA TAC 23
(2) INFORMATION FOR SEQ ID NO:29:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:29:
      CTGGGAGACG TCCCAGGGAC TTC 23
(2) INFORMATION FOR SEQ ID NO:30:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:30:
      GGCCAGACTG TTACCACTCC CTGAAGTTTG AC 32
(2) INFORMATION FOR SEQ ID NO:31:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (x) SEQUENCE DESCRIPTION: SEQ ID NO:31:
      CATCGATAAA ATAAAAGATT TTATTTAGTC 30
(2) INFORMATION FOR SEQ ID NO:32:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:32:
      CAAATGAAAG ACCCCCGCTG AC 22
(2) INFORMATION FOR SEQ ID NO:33:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:33:
      CCTATGAGCT CGCCTTCTAG TTGCCAGC 28
(2) INFORMATION FOR SEQ ID NO:34:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:34:
      CCTATGAATT CGCGGCCGCC ATAGAGCCCA CCGCATCC 38
(2) INFORMATION FOR SEQ ID NO:35:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (xi) SEQUENCE DESCRIPTION: SEQ ID NO:35:
      TATATATGAG CTCTAATAAA ATGAGGAAAT TGCATCGCAT TGTC 44
(2) INFORMATION FOR SEQ ID NO:36:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (xi) SEQUENCE DESCRIPTION: SEQ ID NO:36:
      CCTATGAATT CGCGGCCGCA TAGAATGACA CCTACTCAGA CAATGCGA 48
(2) INFORMATION FOR SEQ ID NO:37:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:37:
      GCTCGTTTAG TGAACCGTCA G 21
(2) INFORMATION FOR SEQ ID NO:38:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:38:
      TATCCGAGCT CATGGCTCGT ACTCTATGG 29
(2) INFORMATION FOR SEQ ID NO:39:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:39:
      CACCTATGCT AGCCACCATG GCGCGTTCAA CGCTCTC 37
(2) INFORMATION FOR SEQ ID NO:40:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:40:
      CACCTATGCG GCCGCTCATG GCTCGTACTC TATGGG 36
(2) INFORMATION FOR SEQ ID NO:41:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:41:
      CACCTATGCG GCCGCCACCA TGGCGCGTTC AACGCTCTC 39
(2) INFORMATION FOR SEQ ID NO:42:
   (i) SEQUENCE CHARACTERISTICS:
      (A) LENGTH: 429 base pairs
      (B) TYPE: nucleic acid
      (C) STRANDEDNESS: single
      (D) TOPOLOGY: linear
   (xi) SEQUENCE DESCRIPTION: SEQ ID NO:42:
   ATCGATACCA TGGGGCAAAC CGTGACTACC CCTCTGTCCC TCACACTGGG CCATTGGAAG 60
   GACGTGGAAA GAATTGCCCA TAATCAAAGC GTGGACGTCA AAAAACGCAG GTGGGTGACA 120
   TTTTGTAGCG CCGAGTGGCC CACATTCAAT GTTGGCTGGC CTAGGGATGG AACTTTCAAT 180
   CGCGATCTGA TTACTCAAGT GAAAATTAAA GTGTTCAGCC CCGGACCCCA CGGCCATCCC 240
   GATCAAGTTC CTTATATTGT CACATGGGAG GCTCTCGCTT TCGATCCACC ACCTTGGGTG 300
   AAACCATTCG TGCATCCCAA ACCACCTCCA CCCCTCCCAC CCAGCGCTCC TAGCCTGCCC 360
   TTGGAGCCCC CACGAAGCAC ACCACCCAGG AGCAGCTTGT ACCCTGCTCT GACCCCCAGC 420
   CTCGGCGCC 429
(2) INFORMATION FOR SEQ ID NO:43:
   (i) SEQUENCE CHARACTERISTICS:
      (A) LENGTH: 32 base pairs
      (B) TYPE nucleic acid
      (C) STRANDEDNESS: single
      (D) TOPOLOGY: linear
   (xi) SEQUENCE DESCRIPTION: SEQ ID NO:43:
      CGAATCGATA CCATGGGCCA GACTGTTACC AC 32
(2) INFORMATION FOR SEQ ID NO:44:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:44:
      CATTCTGCAG AGCAGAAGGT AAC 23
(2) INFORMATION FOR SEQ ID NO:45:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (xi) SEQUENCE DESCRIPTION: SEQ ID NO:45:
      TAAGCGGCCG CTTA 14

## Description

### Technical Field

The present invention relates generally to producer cell lines comprising retroviral vectors for use in gene transfer, and more specifically, retroviral vectors which are constructed such that the formation of replication competent virus by recombination is precluded.

### Background of the Invention

Retroviruses are RNA viruses which can replicate and integrate into a host cell's genome through a DNA intermediate. This DNA intermediate, or provirus, may be stably integrated into the host's cellular DNA. Retroviruses are known to be responsible for a wide variety of diseases in both man and animals, including for example AIDS and a wide variety of cancers.

Although retroviruses can cause disease, they also have a number of properties that lead them to be considered as one of the most promising techniques for genetic therapy of disease. These properties include: (1) efficient entry of genetic material (the vector genome) into cells; (2) an active efficient process of entry into the target cell nucleus; (3) relatively high levels of gene expression; (4) minimal pathological effects on target cells; and (5) the potential to target particular cellular subtypes through control of the vector-target cell binding and tissue-specific control of gene expression. In using a retrovirus for genetic therapy, a foreign gene of interest may be incorporated into the retrovirus in place of normal retroviral RNA. When the retrovirus injects its RNA into a cell, the foreign gene is also introduced into the cell, and may then be integrated into the host's cellular DNA as if it were the retrovirus itself. Expression of this foreign gene within the host results in expression of foreign protein by the host cell.

Most retroviral vector systems which have been developed for gene therapy are based on murine retroviruses. Briefly, these retroviruses exist in two forms, as proviruses integrated into a host's cellular DNA, or as free virions. The virion form of the virus contains the structural and enzymatic proteins of the retrovirus (including reverse transcriptase), two RNA copies of the viral genome, and portions of the cell's plasma membrane in which is embedded the viral envelope glycoprotein. The genome is organized into four main regions: the Long Terminal Repeat (LTR), and the *gag*, *pol,* and *env* genes. The LTR may be found at both ends of the proviral genome, is a composite of the 5' and 3' ends of the RNA genome, and contains *cis*-acting elements necessary for the initiation and termination of transcription. The three genes *gag*, *pol,* and *env* are located between the terminal LTRs. The *gag* and *pol* genes encode, respectively, internal viral structures and enzymatic proteins (such as integrase). The *env* gene encodes the envelope glycoprotein (designated gp70 and p15e) which confers infectivity and host range specificity of the virus, as well as the "R" peptide of undetermined function.

An important consideration in using retroviruses for gene therapy is the availability of "safe" retroviruses. Packaging cell lines and vector producing cell lines have been developed to meet this concern. Briefly, this methodology employs the use of two components, a retroviral vector and a packaging cell line (PCL). The retroviral vector contains long terminal repeats (LTRs), the foreign DNA to be transferred and a packaging sequence (y). This retroviral vector will not reproduce by itself because the genes which encode structural and envelope proteins arc not included within the vector genome. The PCL contains genes encoding the *gag*, *pol,* and *env* proteins, but does not contain the packaging signal "y". Thus, a PCL can only form empty virion particles by itself Within this general method, the retroviral vector is introduced into the PCL, thereby creating a vector-producing cell line (VCL). This VCL manufactures virion particles containing only the retroviral vector's (foreign) genome, and therefore has previously been considered to be a safe retrovirus vector for therapeutic use.

There are, however, several shortcomings with the current use of VCLs. One issue involves the generation of "live virus" (*i.e.*, replication competent retrovirus; RCR) by the VCL. Briefly, RCR can be produced in conventional producer cells when: (1) The vector genome and the helper genomes recombine with each other; (2) The vector genome or helper genome recombines with homologous cryptic endogenous retroviral elements in the producer cell; or (3) Cryptic endogenous retroviral elements reactivate (*e.g.*, xenotropic retroviruses in mouse cells).

Another issue is the propensity of mouse based VCLs to package endogenous retrovirus-like elements (which can contain oncogenic gene sequences) at efficiencies close to that with which they package the desired retroviral vector. Such elements, because of their retrovirus-like structure, are transmitted to the target cell to be treated at frequencies that parallel its transfer of the desired retroviral vector sequence.

A third issue is the ability to make sufficient retroviral vector particles at a suitable concentration to: (1) treat a large number of cells (*e.g.*, 10⁸ - 10¹⁰); and (2) manufacture vector particles at a commercially viable cost.

In order to construct safer PCLs, researchers have generated deletions of the 5' LTR and portions of the 3' LTR of helper elements (*see*, Miller and Buttimore, Mol. Cell. Biol. 6:2895-2902, 1986). When such cells are used, two recombination events are necessary to form the wild-type, replication competent genome. Nevertheless, results from several laboratories have indicated that even when several deletions are present, RCR may still be generated (*see*, Bosselman et al., Mol. Cell. Biol 7:1797-1806, 1987; Danos and Mulligan, Proc. Nat'l. Acad. Sci. USA 81:6460-6464, 1988). In addition, cell lines containing both 5' and 3' LTR deletions which have been constructed have thus far not proven useful since they produce relatively low titers (Dougherty et art, J. Virol. 63:3209-3212, 1989).

One of the more recent approaches to constructing safer packaging cell lines involves the use of complementary portions of helper virus elements, divided among two separate plasmids, one containing *gag* and *pol,* and the other containing *env* (*see,* Markowitz et al., J. Virol. 62:1120-1124; and Markowitz et al., Virology 167:600-606, 1988. One benefit of this double-plasmid system is that three recombination events arc required to generate a replication competent genome. Nevertheless, these double-plasmid vectors have also suffered from the drawback of including portions of the retroviral LTRs, and therefore remain capable of producing infectious virus.

The present invention overcomes the difficulties of recombination and low titer associated with many of the prior packaging cell lines, and further provides other related advantages.

### Summary of the Invention

Briefly stated, the present invention provides a producer cell line, comprising a *gag*/*pol* expression cassette, an *env* expression cassette and a retroviral vector construct, wherein there is no sequence overlap between the *gag*/*pol* expression cassette and the *env* expression cassette, and no sequence overlap between the *env* expression cassette and the retroviral vector construct, with the proviso that said retroviral vector construct overlaps with at least 4 nucleotides of a 5' terminal end of a *gag*/*pol* gene encoded within said *gag*/*pol* expression cassette.

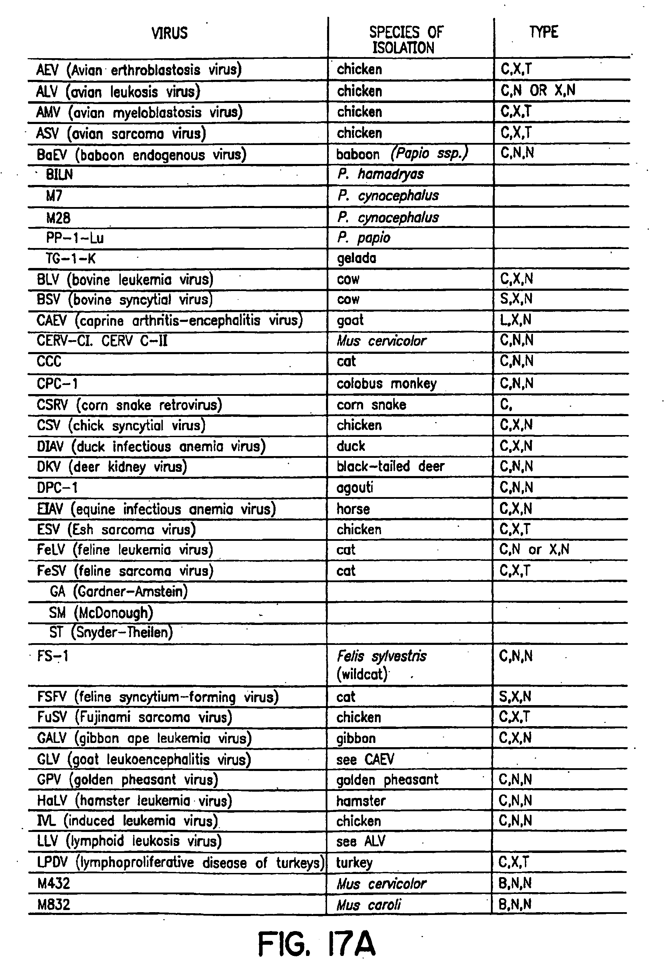

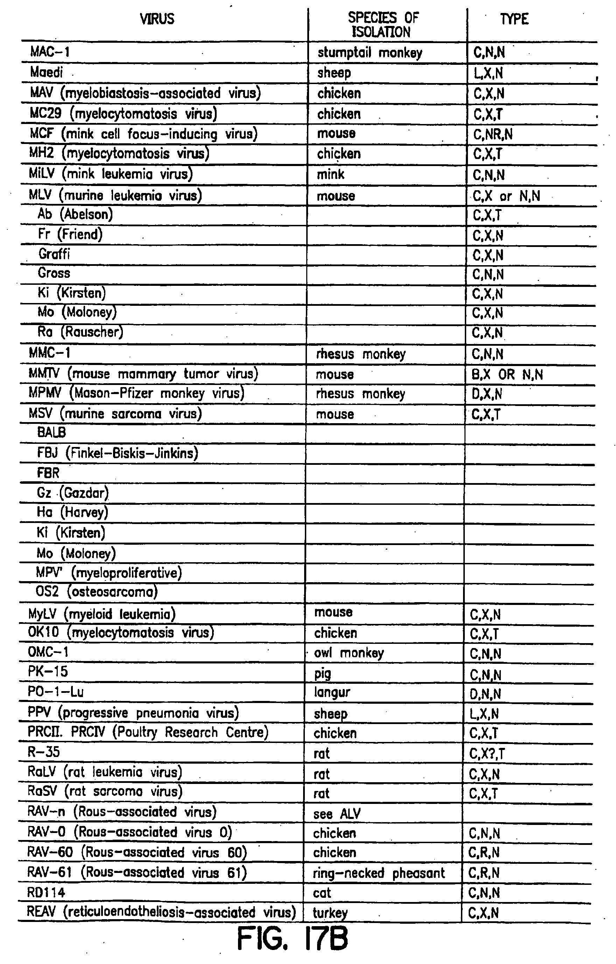

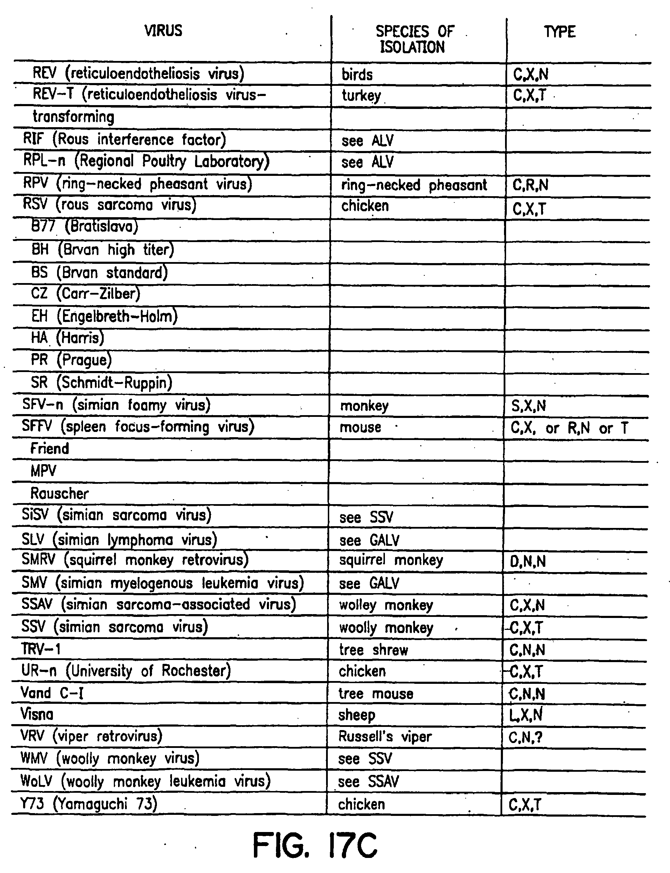

Retroviral vector constructs used in the present invention may be constructed from one or more retroviruses, including, for example, a wide variety of amphotropic, ecotropic, xenotropic, and polytropic viruses (*see e.g.*, Figures 17A ,B and C).

As noted above, retroviral vector constructs used in the present invention include one or more heterologous sequences. The retroviral vector construct may further comprise a heterologous sequence that is at least x kb in length, wherein x is selected from the group consisting of 1, 2, 3, 4,5, 6, 7 and 8. The heterologous sequence may be gene encoding a cytotoxic protein, such as, for example, ricin; abrin, diphtheria toxin, cholera toxin, gelonin, pokeweed, antiviral protein, tritin, Shigella toxin, and Pseudomonas exotoxin A. The heterologous sequence may be an antisense sequence, or an immune accessory molecule. Representative examples of immune accessory molecules include IL-1, IL-3, IL-4, IL-5, IL-6, IL-7, IL-8, IL-9, IL-10, IL-11. IL-13, and IL-14. Particularly preferred immune accessory molecules may be selected from the group consisting of IL-2, IL-12, IL-15 and gamma-interferon, or the group consisting of ICAM-1, ICAM-2, b-microglobin, LFA3. HLA class I and HLA class II molecules.

Furthermore, the heterologous sequence may encode a gene product that activates a compound with little or no cytotoxicity into a toxic product. Representative examples of such gene products include type thymidine kinases such as HSVTK and VZVTK as well as other prodrug-converting enzymes such as cytosine deaminase. Furthermore, the heterologous sequence may be a ribozyme. Furthermore, the heterologous sequence is a replacement gene, which encode proteins such as Factor VIII, ADA, HPRT, CF and the LDL Receptor. Furthermore, the heterologous sequence encodes an immunogenic portion of a virus selected from the group consisting of HBV, HCV, HPV, EBV, FeLV, FIV, and HIV.

Furthermore, *gag*/*pol* expression cassettes are described, comprising a promoter operably linked to a *gaglpol* gene, and a polyadenylation sequence, wherein the *gag*/*pol* gene has been modified to contain codons which are degenerate for gag. Furthermore, the 5' terminal end of the *gaglpol* gene may lack a retroviral packaging signal sequence. *gag*/*pol* expression cassettes are described comprising a promoter operably linked to a *gaglpol* gene, and a polyadenylation sequence, wherein the expression cassette does not co-encapsidate with a replication competent virus.

Furthermore, *gag*/*pol* expression cassettes are described comprising a promoter operably linked to a *gaglpol* gene, and a polyadenylation sequence, wherein a 3' terminal end of the *gag*/*pol* gene has been deleted without effecting the biological activity of integrase. A 5' terminal end of the *gaglpol* gene may be modified to contain codons which are degenerate for gag. The 5' terminal end of the *gag*/*pol* gene may lack a retroviral packaging signal sequence. The 3' terminal end may have deleted so that nucleotides downstream of nucleotide 5751 or any nucleotide between nucleotide 5751 and 5777 of SEQ ID NO: 1 are deleted.

Furthermore, *env* expression cassettes are described comprising a promoter operably linked to an *env* gene, and a polyadenylation sequence, wherein no more than 6 retroviral nucleotides are included upstream of the *env* gene. *env* expression cassettes are described comprising a promoter operably linked to an *env* gene, and a polyadenylation sequence, wherein the *env* expression cassette does not contain a consecutive sequence of more than 8 nucleotides which are found in *gag*/*pol* gene. *env* expression cassettes are described comprising a promoter operably linked to an *env* gene, and a polyadenylation sequence, wherein a 3' terminal end of the *env* gene has been deleted without effecting the biological activity of env. The 3' terminal end of the gene may have been deleted such that a complete R peptide is not produced by the expression cassette. The *env* gene may be derived from a type C retrovirus, and the 3' terminal end has been deleted such that the *env* gene includes less than 18 nucleic acids which encode the R peptide. The 3' terminal end may have been deleted downstream from nucleotide 7748 of SEQ ID NO:1.

Furthermore the promoters of the *gaglpol* and *env* expression cassettes described above are heterologous promoters, such as CMV IE, the HVTK promoter, RSV promoter, Adenovirus major-later promoter and the SV40 promoter. The polyadenylation sequence may be a heterologous polyadenylation sequence, such as the SV40 late poly A Signal and the SV40 early poly A Signal.

Furthermore packaging cell lines are described, comprising a *gaglpol* expression cassette and an *env* expression cassette wherein the *gaglpol* expression cassette lacks a consecutive sequence of greater than 20. preferably greater than 15, more preferably greater than 10, and most preferably greater than 8 consecutive nucleotides which are found in the *env* expression cassette. Producer cell lines are described comprising a *gaglpol* expression cassette, *env* expression cassette, and a retroviral vector construct, wherein the *gag*/*pol* expression cassette, *env* expression cassette and retroviral vector construct lack a consecutive sequence of greater than 20, preferably greater than 15, more preferably greater than 10, and most preferably greater than 8 nucleotides in common. Representative examples of such retroviral vector constructs, *gaglpol* and *env* expression cassettes are described more detail below.

Furthermore, producer cell lines are described comprising a packaging cell line as described above, and a retroviral vector construct. Producer cell lines are described comprising a *gaglpol* expression cassette, *env* expression cassette and a retroviral vector construct, wherein the *gaglpol* expression cassette, *env* expression cassette and retroviral vector construct lack a consecutive sequence of greater than eight nucleotides in common.

Furthermore packaging cell lines are described which 'mix and match' various elements of the above described retroviral vector constructs, *gag*/*pol* expression cassettes, and *env* expression cassettes. Briefly, many previous packaging cell lines have three areas of overlap: (1) between the retroviral vector construct and the *gaglpol* expression cassette; (2) between the *gag*/*pol* expression cassette and the *env* expression cassette: and/or (3) between the *env* expression cassette and the retroviral vector. As described herein, packaging cell lines and producer cell lines with reduced sequence overlap can be produced with no sequence overlap in area 1, area 2, or, area 3 only, a combination of any two (*e.g.*. no sequence overlap in areas 1 and 2 only, no sequence overlap in areas 1 and 3 only, or no sequence overlap in areas 2 and 3 only), or no sequence overlap in any of the three areas. For example, producer cell lines are described a *gag*/*pol* expression cassette, an env expression cassette and a retroviral vector construct, wherein a 3' terminal end of a *gag*/*pol* gene encoded within said *gag*/*pol* expression cassette lacks homology with a 5' terminal end of an *env* gene encoded within said *env* expression cassette, and wherein a 3' terminal end of said env gene lacks homology with said retroviral vector construct, with the proviso that said retroviral vector construct overlaps with at least 4 nucleotides (and as many as 8, 10, 15, 20, or more nucleotides) of a 5' terminal end of said *gaglpol* gene encoded within said *gaglpol* expression cassette. As utilized herein, the phase "lack homology" means that the two cassettes or cassette and construct lack at least 3 or 4, and preferably more than 8, 10, 15 or 20 consecutive nucleotides in common.

Furthermore methods of producing a packaging cell line are described comprising the steps of (a) introducing a *gaglpol* expression cassette as described above into an animal cell; (b) selecting a cell containing a *gaglpol* expression cassette which expresses high levels of *gaglpol,* (c) introducing an *env* expression cassette into said selected cell, and (d) selecting a cell which expresses high levels of *env* and thereby producing the packaging cell. The *env* expression cassette may be introduced into the cell first, followed by, the *gag*/*pol* expression cassette. Methods are described for producing recombinant retroviral particles comprising the step of introducing a retroviral vector construct into a packaging cell as described above. For example, the retroviral vector construct is one of the retroviral vector constructs described above. As noted above, within any of the methods described herein not all areas of sequence overlap must be eliminated. Thus, within certain methods sequence overlap is not eliminated, for example, between the retroviral vector construct and the *gaglpol* expression cassette.

These and other aspects of the present invention will become evident upon reference to the following detailed description and attached drawings. In addition, various references are set forth below which describe in more detail certain procedures or compositions (*e.g.*. plasmids, etc.),

### Brief Description of the Drawings

Figure 1 is a schematic illustration of pKS2+*Eco*57I-LTR(+).
Figure 2 is a schematic illustration of pKS2+*Eco*57I-LTR(-).
Figure 3 is a schematic illustration of pKS2+LTR-*Eco*RI.
Figure 4 is a schematic illustration of pR1.
Figure 5 is a schematic illustration of pR2.
Figure 6 is a schematic illustration of pKT1.
Figure 7 is a schematic illustration of pRI-HIVenv.
Figure 8 is a schematic illustration of pR2-HIVenv.
Figure 9 is a representative "prewobble" sequence for a MoMLV *gag*/*pol* (*see also* SEQ I.D. Nos. 11 and 12).
Figure 10 is a representative "wobble" sequence for a MoMLV *gaglpol* (*see also* SEQ. I.D. Nos. 9 and 10).
Figure 11 is a schematic illustration of pHCMV-PA.
Figure 12 is a schematic illustration of pCMV *gaglpol.*

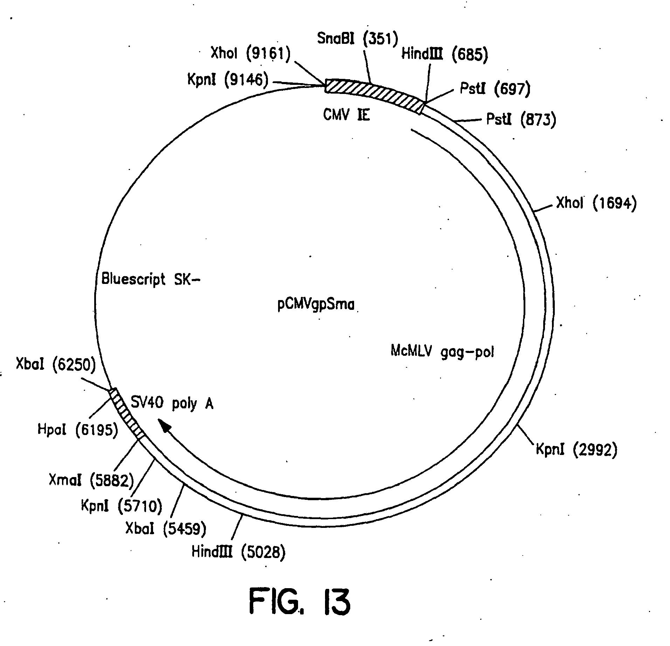

Figure 13 is a schematic illustration of pCMVgpSma.
Figure 14 is a schematic illustration otpCMVgp-X.
Figure 15 is a schematic illustration of pCMV env-X.
Figure 16 is a schematic illustration of pRgpNeo.
Figures 17A, B and C comprise a table which sets forth a variety of retroviruses which may be utilized to construct the retroviral vector constructs, *gag*/*pol* expression cassettes and *env* expression cassettes of the present invention.
Figure 18 is a schematic illustration of pCMV Envam-Eag-X-less.
Figure 19A is a diagrammatic illustration of a "wobble" *-gag* construct. Figure 19B is a diagrammatic illustration of a "normal" *-gag* constructs.
Figure 20 is a description of all modifications carried out on retroviral vector as shown in A), resulting in the cross-less retroviral vector shown in B). The cross-less retroviral backbone cloned into a prokaryotic vector is called pBA-5.

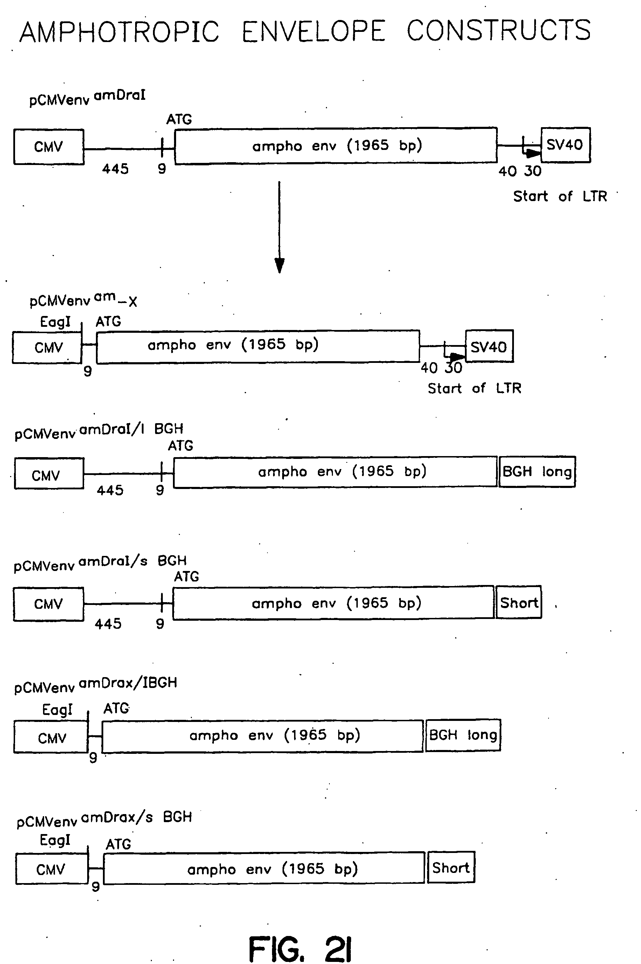

Figure 21 depicts retroviral amphotropic envelope constructs starting with the pCMVenvAMDraI at the top of the page and modifications thereof. The exact modifications in the envelope constructs are described in the examples.
Figures 22A, 22B and 22C are schematics showing retrovirus with three regions (A), one region (B) and no region (C) of sequence overlap.

### Detailed Description of the Invention

Prior to setting forth the invention, it may be helpful to an understanding thereof to first set forth definitions of certain terms that will be used hereinafter.

"Retroviral vector construct" refers to an assembly which is, capable of directing the expression of a sequence(s) or gene(s) of interest. Briefly, the retroviral vector construct must include a 5' LTR a tRNA binding site, a packaging signal, an origin of second strand DNA synthesis and a 3' LTR. A wide variety of heterologous sequences may be included within the vector construct, including for example, sequences which encode a protein (*e.g.*. cytotoxic protein, disease-associated antigen, immune accessory molecule, or replacement gene), or which are useful as a molecule itself (*e.g.*, as a ribozyme or antisense sequence). Alternatively, the heterologous sequence may merely be a "stuffer" or "filler" sequence, which is of a size sufficient to allow production of viral particles containing the RNA genome. Preferably, the heterologous sequence is at least 1. 2, 3, 4. 5, 6. 7 or 8 kB in length.

The retroviral vector construct may also include transcriptional promoter/enhancer or locus defining element(s), or other elements which control gene expression by means such as alternate splicing, nuclear RNA export, post-translational modification of messenger, or post-transcriptional modification of protein. Optionally, the retroviral vector construct may also include selectable markers such as Nco, TK, hygromycin, phleomycin, histidinol, human placental Alkaline Phosphatase, NGFR or DHFR, as well as one or more specific restriction sites and a translation termination sequence.

"Expression cassette" refers to an assembly which is capable of directing the expression of the sequence(s) or gene(s) of interest. The expression cassette must include a promoter which, when transcribed, is operably linked to the sequence(s) or gene(s) of interest, as well at a polyadenylation sequence. Furthermore, both the promoter and the polyadenylation sequence are from a source which is heterologous to the helper elements (*i.e.. gaglpol* and *env*). Expression cassettes used in the present invention may be utilized to express a *gaglpol* gene or an *env* gene. In addition, the expression cassettes may also be utilized to express one or more heterologous sequences either from a *gaglpol* and/or *env* expression cassette, or from a entirely different expression cassette.

Furthermore the expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA (*e.g.*, a M13 origin of replication), a multiple cloning site, and a "mammalian" origin of replication (*e.g.,* a SV40 or adenovirus origin of replication).

### Preparation of Retroviral vector constructs. GaglPol Expression Cassettes and Env Expression Cassettes

The following sections describe the preparation of retroviral vector constructs, *gaglpol* expression cassettes, and *env* expression cassettes.

### 1. Construction of retroviral vector constructs

Retroviral vector constructs are described comprising a 5' LTR, a tRNA binding site, a packaging signal, an origin of second strand DNA synthesis and a 3' LTR, wherein the vector construct lacks *gaglpol* or *env* coding sequences. Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTR's may be readily identified in the provirus due to their precise duplication at either end of the genome.

The tRNA binding site and origin of second strand DNA synthesis are also important for a retrovirus to be biologically active, and may be readily identified by one of skill in the art. For example, tRNA binds to a retroviral tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5' LTR. Similarly, the origin of second strand DNA synthesis is, as its name implies, important for the second strand DNA synthesis of a retrovirus. This region, which is also referred to as the poly-purine tract, is located just upstream of the 3' LTR.

In addition to 5' and 3' LTRs, a tRNA binding site, and an origin of second strand DNA synthesis, retroviral vector constructs of the present invention also comprise a packaging signal, as well as one or more heterologous sequences, each of which is discussed in more detail below.

Retroviral vector constructs useful in the present invention may be readily constructed from a wide variety of retroviruses, including for example, B, C, and D type retroviruses as well as spumaviruses and lentiviruses *(see* RNA Tumor Viruses. Second Edition. Cold Spring Harbor Laboratory. 1985). Briefly, viruses are often classified according to their morphology as seen under electron microscopy. Type "B" retroviruses appear to have an eccentric core, while type "C" retroviruses have a central core. Type "D" retroviruses have a morphology intermediate between type B and type C retroviruses. Representative examples of suitable retroviruses include those set forth below in Figures 17A, B and C (*see* RNA Tumor Viruses at pages 2-7), as well as a variety of xenotropic retroviruses (*e.g.*, NZB-X1, NZB-X2 and NZB₉₋₁ (*see* O'Neill et al., J. Vir. 53:100-106. 1985)) and polytropic retroviruses (*e.g.*, MCF and MCF-MLV *(see* Kelly et al., J. Vir. 45(1):291-299, 1983)). Such retroviruses may be readily obtained from depositories or collections such as the American Type Culture Collection ("ATCC"; Rockville. Maryland), or isolated from known sources using commonly available techniques.

Particularly preferred retroviruses for the preparation or construction of retroviral vector constructs used in the present invention include retroviruses selected from the group consisting of Avian Leukosis Virus, Bovine Leukemia Virus, Murine Leukemia Virus. Mink-Cell Focus-inducing Virus. Murine Sarcoma Virus. Reticuloendotheliosis virus, Gibbon Ape Leukemia Virus, Mason Pfizer Monkey Virus, and Rous Sarcoma Virus. Particularly preferred Murine Leukemia Viruses include 4070A and 1504A (Hartley and Rowe, J. Virol. 19:19-25, 1976), Abelson (ATCC No. VR-999), Friend (ATCC No. VR-245), Graffi, Gross (ATCC No. VR-590), Kirsten, Harvey Sarcoma Virus and Rauscher (ATCC No. VR-998), and Moloney Murine Leukemia Virus (ATCC No. VR-190). Particularly preferred Rous Sarcoma Viruses include Bratislava, Bryan high titer (*e.g.*, ATCC Nos. VR-334. VR-657, VR-726. VR-659, and VR-728), Bryan standard, Carr-Zilber, Engelbreth-Holm, Harris, Prague (*e.g.*, ATCC Nos. VR-772, and 45033), and Schmidt-Ruppin (*e.g.* ATCC Nos. VR-724, VR-725, VR-354).

Any of the above retroviruses may be readily utilized in order in assemble or construct retroviral vector constructs, packaging cells, or producer cells useful in the present invention given the disclosure provided herein, and standard recombinant techniques (*e.g..* Sambrook et al, Molecular Cloning: A Laboratory Manual. 2d ed., Cold Spring Harbor Laboratory Press, 1989; Kunkle, PNAS 82:488, 1985). Further, within certain embodiments of the invention, portions of the retroviral vector construct may be derived from different retroviruses. For example, the retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus. Similarly, portions of a packaging cell line may be derived from different viruses (*e.g.*, a *gaglpol* expression cassette may be constructed from a Moloney Murine Leukemia Virus, and an *env* expression cassette from a Mason Pfizer Monkey virus).

The retroviral vector constructs wich are described herein have packaging signals, and which lack both *gaglpol* and *env* coding sequences. As utilized within the context of the present invention, a packaging signal should be understood to refer to that sequence of nucleotides which is not required for synthesis, processing or translation of viral RNA or assembly of virions, but which is required in *cis* for encapsidation of genomic RNA (*see* Mann et al.. Cell 33:153-159. 1983; RNA Tumor Viruses, Second Edition, *supra*). Further, as utilized herein, the phrase "lacks *gag*/*pol* or *env* coding sequences" should be understood to refer to retrovectors which contain less than 20. preferably less than 15, more preferably less than 10, and most preferably less than 8 consecutive nucleotides which are found in *gag*/*pol* or *env* genes, and in particular, within *gag*/*pol* or *env* expression cassettes that are used to construct packaging cell lines for the retroviral vector construct. Representative examples of such retroviral vector constructs are set forth in more detail below and in Example 1.

As an illustration, within one embodiment of the invention construction of retroviral vector constructs which lack *gaglpol* or *env* sequences may be accomplished by preparing retroviral vector constructs which lack an extended packaging signal. As utilized herein, the phrase "extended packaging signal" refers to a sequence of nucleotides beyond the minimum core sequence which is required for packaging, that allows increased viral titer due to enhanced packaging. As an example, for the Murine Leukemia Virus MoMLV, the minimum core packaging signal is encoded by the sequence (counting from the 5' LTR cap site) from approximately nucleotide 144 of SEQ. I.D. No. 1, up through the *Pst* I site (nucleotide 567 of SEQ. I.D. No. 1). The extended packaging signal of MoMLV includes the sequence beyond nucleotide 567 up through the start of the *gaglpol* gene (nucleotide 621), and beyond nucleotide 1040. Thus, retroviral vector constructs which lack extended packaging signal may be constructed from the MoMLV by deleting or truncating the packaging signal downstream of nucleotide 567.

Herewith retroviral vector constructs are described wherein the packaging signal that extends into, or overlaps with, retroviral *gaglpol* sequence is deleted or truncated. For example, in the representative case of MoMLV: the packaging signal is deleted or truncated downstream of the start of the *gag*/*pol* gene (nucleotide 621 of SEQ ID NO: 1). For example the packaging signal is terminated at nucleotide 570. 575, 580, 585, 590, 595, 600, 610, 615 or 617 of SEQ ID NO: 1.

Herewith retroviral vector constructs are described which include a packaging signal that extends beyond the start of the *gag*/*pol* gene (*e.g.*. for MoMLV, beyond nucleotide 621 of SEQ ID NO: 1). When such retroviral vector constructs are utilized, it is preferable to utilize packaging cell lines for the production of recombinant viral particles wherein the 5' terminal end of the *gaglpol* gene in a *gaglpol* expression cassette has been modified to contain codons which are degenerate for *gag.* Such *gag*/*pol* expression cassettes are described in more detail below in section 2, and in Example 3.

Within certain retroviral vector constructs, the packaging signal that extends beyond the start of the *gag*/*pol* gene was modified in order to contain one, two or more stop codons within the *gag*/*pol* reading frame. Most preferably, one of the stop codons eliminates the start site and/or has two or three base pair substitutions. One representative example of such modifications is provided below in Example 9.

Herewith retroviral vector constructs are described comprising a 5' LTR, a tRNA binding site, a packaging signal, an origin of second strand DNA synthesis and a 3' LTR. wherein the retrovector plasmid construct does not contain a retroviral nucleic acid sequence upstream of the 5' LTR. As utilized within the context of the present invention, the phrase "does not contain a retroviral nucleic acid sequence upstream of the 5' LTR should be understood to mean that the retrovector plasmid construct contains less than 20, preferably less than 15, more preferably less-than 10, and most preferably less than 8 consecutive nucleotides which are found in a retrovirus, and more specifically, in a retrovirus which is homologous to the retroviral vector construct, upstream of and/or contiguous with the 5' LTR. For example the retrovector plasmid constructs do not contain an *env* coding sequence (as discussed below) upstream of the 5' LTR. An example of such retrovector plasmid constructs is set forth in more detail below in Example 1.

Herewith, retrovector plasmid constructs are described comprising a 5' LTR, a tRNA binding site, a packaging signal, an origin of second strand DNA synthesis and a 3' LTR, wherein the retrovector plasmid construct does not contain a retroviral packaging signal sequence downstream of the 3' LTR. As utilized herein, the term "packaging signal sequence" should be understood to mean a sequence sufficient to allow packaging of the RNA genome. A representative example of such a retroviral vector construct is set forth in more detail below in Example 1.

Herewith, retrovector plasmid constructs are described comprising a 5' LTR. a tRNA binding site, a packaging signal, an origin of second strand DNA synthesis and a 3' LTR, wherein the retrovector plasmid construct does not contain envelope sequences upstream of the 3' LTR. As utilized within this context, the term "envelope sequence" should be understood to mean envelope coding as well as flanking untranslated sequences. A representative example of such a retroviral vector construct is set forth in more detail below in Example 9.

### 2. Construction of gag/pol expression cassettes

Herewith it is described a variety of *gag*/*pol* expression cassettes which, in combination with the retroviral vector constructs and *env* expression cassettes useful in the present invention, enable the construction of packaging cell lines and producer cell lines which preclude the formation of replication competent virus. Briefly, retroviral *gag*/*pol* genes contain a *gag* region which encodes a variety of structural proteins that make up the core matrix and nucleocapsid, and a *pol* region which contains genes which encode (1) a protease for the processing of *gaglpol* and *env* proteins, (2) a reverse transcriptase polymerase. (3) an RNase H, and (4) an integrase, which is necessary for integration of the retroviral provector into the host genome. Although retroviral *gag*/*pol* genes may be utilized to construct the *gag*/*pol* expression cassettes useful in the present invention, a variety of other non-retroviral (and non-viral) genes may also be utilized to construct the *gag*/*pol* expression cassette. For example, a gene which encodes retroviral RNase H may be replaced with genes which encode bacterial (*e.g., E. coli* or *Thermus thermophilus)* RNase H. Similarly, a retroviral integrate gene may be replaced by other genes with similar function (*e.g.,* yeast retrotransposon TY3 integrase).

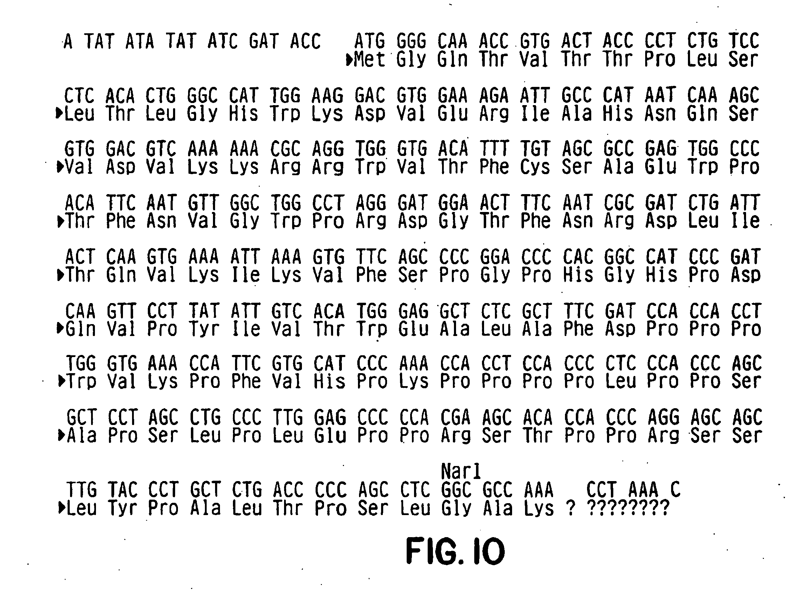

Herewith *gag*/*pol* expression cassettes are described comprising a promoter operably linked to a *gag*/*pol* gene, and a polyadenylation sequence, wherein the *gag*/*pol* gene has been modified to contain codons which are degenerate for gag. Briefly, as noted above, in wild-type retrovirus the extended packaging signal of the retrovirus overlaps with sequences which encode gag and pol. Thus, in order to eliminate the potential of crossover between the retroviral vector construct and the *gag*/*pol* expression cassette, as well as to eliminate the possiblity of co-encapsidation of the *gag*/*pol* expression cassette and replication competent virus or retroviral vector constructs, sequences of overlap should be eliminated. Elimination of such overlap may be accomplished by modifying the *gaglpol* gene (and more specifically, regions which overlap with the retroviral vector construct such as the extended packaging signal) to contain codons that are degenerate (*i.e.*, that "wobble") for gag. In particular codons may be selected wich encode biologically active gag/pol protein (*i.e.*, capable of producing a competent retroviral particle, in combination with an *env* expressing element, and a RNA genome), and which lack any packaging signal sequence, including in particular, extended packaging signal sequence. As utilized herein, the phrase "lacks any retroviral packaging signal sequence" should be understood to mean that the *gaglpol* expression cassette contains less than 20, preferably less than 15, more preferably less than 10, and most preferably less than 8 consecutive nucleotides which are identical to a sequence found in a retroviral packaging signal (*e.g.,* in the case of MoMLV, extending up and through the *Xho* 1 site at approximately nucleotide number 1561). A particularly preferred example of such modified codons which are degenerate for gag is shown in Figure 10. and in Example 3, although the present invention should not be so limited. In particular at least 25, 50, 75, 100, 125 or 135 *gag* codons may be modified or "wobbled" from the native *gag* sequence within the *gag*/*pol* expression cassettes of the present invention.

In addition to eliminating overlap between the retroviral vector construct and the *gag*/*pol* gene, it is also preferable to eliminate any potential overlap between the *gag*/*pol* gene and the *env* gene in order to prohibit the possibility of homologous recombination. This may be accomplished in at least two principal ways: (1) by deleting a portion of the *gaglpol* gene which encodes the integrase protein, and in particular, that portion of the gene which encodes the integrase protein which overlaps with the *env* coding sequence, or (2) by selecting codons which are degenerate for integrase and/or env.

Thus, *gag*/*pol* expression cassettes are described comprising a promoter operably linked to a *gag*/*pol* gene, and a polyadenylation sequence or signal, wherein a 3' terminal end of the gene has been deleted without effecting the biological activity of the integrase. (The biological activity of integrase may be readily determined by detection of an integration event, either by DNA analysis or by expression of a transduced gene; *see* Roth et al., J. Vir. 65(4):2141-2145,1991.) As an example, in the Murine Leukemia Virus MoMLV (SEQ ID. NO.1), the *gaglpol* gene is encoded by nucleotides 621 through 5834. Within this sequence, the protein integrase is encoded by nucleotides 4610 through nucleotide 5834. A portion of the *gag*/*pol* sequence which encodes integrase also encodes env (which begins at nucleotide 5776). Thus, the 3' terminal end of the *gag*/*pol* gene may be deleted or truncated in order to prevent crossover with the *env* gene, without effecting the biological activity of the integrase. The *gag*/*pol* gene may be deleted at any nucleotide downstream (3') from the beginning of the integrase coding sequence, and preferably prior to the start of the *env* gene sequence. The sequence encoding gag/pol is a MoMLV sequence; and the *gag*/*pol* gene may be deleted at any nucleotide between nucleotides 4610 and 5776 (of SEQ. I.D. No. 1), including for example, at nucleotides 5775. 5770; 5765. 5760, 5755, 5750.

The *gag*/*pol* expression cassette may contain sequences encoding gag/pol (and including integrase), while lacking any sequence found in an *env* gene. The phrase "lacking any sequence found in an *env* gene" should be understood to mean that the *gaglpol* expression cassette does not contain at least 20. preferably at least 15, more preferably at least 10, and most preferably less than 8 consecutive nucleotides which are identical to an *env* sequence, and preferably which are found in an *env* expression cassette which will he utilized along with the *gaglpol* expression cassette to form a packaging cell. Such expression cassettes may be readily prepared by selecting codons which are degenerate for integrase, and which do not encode biologically active env. (*See* Morgenstern and Land, Nuc. Acids Res. 18:3587-3596, 1990.)

The *gaglpol* expression cassette may contain a heterologous promoter, and/or heterologous polyadenylation sequence. As utilized herein, "heterologous" promoters or polyadenylation sequences refers to promoters or polyadenylation sequences which are from a different source from which the *gag*/*pol* gene (and preferably the *env* gene and retroviral vector construct) is derived from. Representative examples of suitable promoters include the Cytomegalovirus Immediate Early ("CMV IE") promoter, the Herpes Simplex Virus Thymidine Kinase ("HSVTK") promoter, the Rous Sarcoma Virus ("RSV") promoter, the Adenovirus major-late promoter and the SV 40 promoter. Representative examples of suitable polyadenylation signals include the SV 40 late polyadenylation signal and the SV40 early polyadenylation signal.

Some *gag*/*pol* expression cassettes such as those described above will not co-encapsidate along with a replication competent virus. One representative method for determination of co-encapsidation is set forth below in Example 8.

### 3. Construction of env expression cassettes

Herewith, *env* expression cassettes are described which, in combination with the *gag*/*pol* expression cassettes and retroviral vector constructs described above, preclude formation of replication competent virus by homologous recombination, as well as to confer a particular specificity of the resultant vector particle (*e.g.*, amphotropic, ecotropic, xenotropic or polytropic; *see* Figure 17, as well as the discussion above). Briefly, in a wild-type retrovirus the *env* gene encodes two principal proteins, the surface glycoprotein "SU" and the transmembrane protein "TM", which are translated as a polyprotein, and subsequently separated by proteolytic cleavage. Representative examples of the SU and TM proteins are the gp120 protein and gp41 protein in HIV, and the gp70 protein and p15c protein in MoMLV. In some retroviruses, a third protein designated the "R" peptide" of undetermined function, is also expressed from the *env* gene and separated from the polyprotein by proteolytic cleavage. In the Murine Leukemia Virus MoMLV, the R peptide is designated "p2".

A wide variety of *env* expression cassettes may be constructed given the disclosure provided herein, and utilized within the present invention to preclude homologous recombination. Herewith *env* expression cassettes are described comprising a promoter operably linked to an *env* gene, wherein no more than 6. 8, 10, 15, or 20 consecutive retroviral nucleotides are included upstream (5') of and/or contiguous with said *env* gene. Furthermore *env* expression cassettes are described comprising a promoter operably linked to an *env* gene, wherein the *env* expression cassette does not contain a consecutive sequence of greater than 20, preferably less than 15, more preferably less than 10. and most preferably less than 8 or 6 consecutive nucleotides which are found in a *gag*/*pol* gene, and in particular, in a *gaglpol* expression cassette that will be utilized along with the *env* expression cassette to create a packaging cell line.

Herewith *env* expression cassettes are described comprising a promoter operably linked to an *env* gene, and a polyadenylation sequence, wherein a 3' terminal end of the *env* gene has been deleted without effecting the biological activity of env. As utilized herein, the phrase "biological activity of env" refers to the ability of envelope protein to be expressed on the surface of a virus or vector particle, and to allow for a successful infection of a host cell. One practical method for assessing biological activity is to transiently transfect the *env* expression cassette into a cell containing a previously determined functional *gaglpol* expression cassette, and a retroviral vector construct which expresses a selectable marker. Another method for assessing biological activity is to either stably transfect the *env* expression cassette together with a retroviral vector construct coding for a selectable marker into a cell containing a previously determined functional *gag*/*pol* expression cassette, or, transducing this gag/pol expressing cell in a transient and/or stable manner with a retroviral vector coding for the env gene and a selectable marker. A biologically functional *env* expression cassette will allow vector particles produced in that transfected cell, to transmit the selectable marker to a naive sensitive cell such that it becomes resistant to the marker drug selection. Within a preferred embodiment of the invention, the 3' terminal end of the *env* gene is deleted or truncated such that a complete R peptide is not produced by the expression cassette. In the representative example of MoMLV, sequence encoding the R peptide (which begins at nucleotide 7734) is deleted, truncated, or, for example, terminated by insertion of a stop codon at nucleotide 7740, 7745, 7747, 7750, 7755, 7760, 7765, 7770, 7775, 7780, or any nucleotide in between.

Furthermore, *env* expression cassettes are described which contain a heterologous promoter, a heterologous leader sequence and/or heterologous polyadenylation sequence. As utilized herein, "heterologous" promoters, leaders or polyadenylation sequences refers to sequences which are from a different source from which the *gag*/*pol* gene (and preferably the *env* gene and retroviral vector construct) is derived from. Representative examples of suitable promoters include the CMV IE promoter, the HSVTK promoter, the RSV promoter, the Adenovirus major-late promoter and the SV 40 promoters. Representative examples of suitable polyadenylation signals include the SV 40 late polyadenylation signal and the SV40 early polyadenylation signal, and the bovine growth hormone termination/polyadenylation sequence. Preferably any such termination/polyadenylation sequence will not have any 10 bp stretch which has more than 80% homology to a retroviral construct.

Envelope expression cassettes that contain no MoMLV noncoding sequences can also be created. For example, analogous to the 3' end modifications described in example 12, noncoding bases on the 5' of envelope prior to the start AUG codon can be deleted as described in Example 4. Another method of 5' end modification is to substitute the 5' untranslated RNA leader of MoMLV envelope with an alternate leader. The 5' untranslated RNA sequence can be a leader from another protein or an entirely synthetic leader. The leader may also contain one or more introns. The only requirements for the leader are that it contains a Kozak sequence sufficient for efficient translation of the amphotropic envelope. Representative leader sequences may also include untranslated RNA leaders for envelope proteins from other viruses. Example of these include Vesicular Stomatitis Virus -G protein (VSV-g). Herpes Simplex Virus(HSV) gB protein, or HSV-gD protein. The 5' untranslated leader sequence is inserted so that it spans the sequence between the eucaryotic promoter start site and the amphotropic envelope start codon.

### HETEROLOGOUS SEQUENCES

As noted above, the retroviral vector constructs, *gag*/*pol* expression cassettes, and *env* expression cassettes used in the present invention may contain (and express) one or more heterologous sequences. As utilized within the context of the present invention, it should be understood that the heterologous sequence need not code for a particular protein, but may be merely included in order to improve efficiency of viral particle production. In this regard, heterologous sequences of at least 1 kb or greater are particularly preferred:

A wide variety of heterologous sequences may be utilized within the context of the present invention, including for example, cytotoxic genes, antisense sequences, sequences which encode gene products that activate a compound with little or no cytotoxicity (*i.e.,* a "prodrug") into a toxic product, sequences which encode immunogenic portions of disease-associated antigens and sequences which encode immune accessory molecules. Representative examples of cytotoxic genes include the genes which encode proteins such as ricin (Lamb et al., Eur. J. Biochem. 148:265-270, 1985), abrin (Wood et al., Eur. J. Biochem. 198:723-732, 1991; Evensen, et al., J. of Biol. Chem. 266:6848-6852, 1991: Collins et al., J. of Biol Chem. 265:8665-8669, 1990; Chen et al., Fed. of Eur. Biochem Soc. 309:115-118, 1992), diphtheria toxin (Tweten et al., J. Biol. Chem. 260:10392-10394, 1985), cholera toxin (Mekalanos et al., Nature 306:551-557, 1983: Sanchez & Holmgren, PNAS 86:481-485, 1989), gelonin (Stirpe et al., J. Biol. Chem. 255:6947-6953, 1980), pokeweed (Irvin. Pharmac. Ther. 21:371-387, 1983), antiviral protein (Barbieri et al., Biochem. J. 203:55-59, 1982; Irvin et al., Arch Biochem. & Biophys. 200:418-425, 1980; Irvin, Arch Biochem. & Biophys. 169:522-528, 1975), tritin, Shigella toxin (Calderwood et al., PNAS 84:4364-4368, 1987; Jackson et al., Microb. Path 2:147-153, 1987), and Pseudomonas exotoxin A (Carroll and Collier, J. Biol. Chem. 262:8707-8711, 1987).

Antisense RNA may be utilized as a cytotoxic gene in order to induce a potent Class 1 restricted response. Briefly, in addition to binding RNA and thereby preventing translation of a specific mRNA, high levels of specific antisense sequences may be utilized to induce the increased expression of interferons (including gamma-interferon), due to the formation of large quantities of double-stranded RNA. The increased expression of gamma interferon, in turn, boosts the expression of MHC Class 1 antigens. Preferred antisense sequences for use in this regard include actin RNA, myosin RNA, and histone RNA. Antisense RNA which forms a mismatch with actin RNA is particularly preferred.

Herewith antisense sequences are described which inhibit, for example, tumor cell growth, viral replication, or a genetic disease by preventing the cellular synthesis of critical proteins needed for cell growth. Examples of such antisense sequences include antisense thymidine kinase, antisense dihydrofolate reductase (Maher and Dolnick, Arch. Biochem. & Biophys. 253:214-220, 1987; Bzik et al.. PNAS 84:8360-8364, 1987), antisense HER2 (Coussens et al., Science 230:1132-1139. 1985), antisense ABL (Fainstein, et al.. Oncogene 4:1477-1481, 1989), antisense Myc (Stanton et al., Nature 310:423-425, 1984) and antisense *ras*, as well as antisense sequences which block any of the enzymes in the nucleotide biosynthetic pathway.

Herewith retroviral vector constructs, *gag*/*pol* expression cassettes and *env* expression cassettes are described which direct the expression of a gene product that activates a compound with little or no cytotoxicity *(i.e..* a "prodrug") into a toxic product. Representative examples of such gene products include varicella zoster virus thymidine kinase (V7.VTK), herpes simplex virus thymidine kinase (HSVTK) (Field et al., J. Gen. Virol. 49:115-124, 1980; Munir et al.. Protein Engineering 7(1):83-89, 1994; Black and Loeb. Biochem 32(43):11618-11626. 1993), and *E. coli.* guanine phosphoribosyl transferase (*see* U.S. Patent Application Serial No. 08/155.944, entitled "Compositions and Methods for Utilizing Conditionally Lethal Genes," filed November 18, 1993; *see also* WO 93/10218 entitled "Vectors Including Foreign Genes and Negative Selection Markers". WO 93/01281 entitled "Cytosine Deaminase Negative Selection System for Gene Transfer Techniques and Therapies", WO 93/08843 entitled "Trapped Cells and Use Thereof as a Drug", WO 93/08844 entitled "Transformant Cells for the Prophylaxis or Treatment of Diseases Caused by Viruses, Particularly Pathogenic Retroviruses", and WO 90/07936 entitled "Recombinant Therapies for Infection and Hyperproliferative Disorders.") Some of, the retroviral vector constructs direct the expression of a gene product that activates a compound with little or no cytotoxicity into a toxic product in the presence of a pathogenic agent, thereby affecting localized therapy to the pathogenic agent (see WO 94/13304).

Furthermore, retroviral vector constructs are described which direct the expression of a HSVTK gene downstream, and under the transcriptional control of an HIV promoter (which is known to be transcriptionally silent except when activated by HIV tat protein). Briefly, expression of the tat gene product in human cells infected with HIV and carrying the vector construct causes increased production of HSVTK. The cells (either *in vitro* or *in vivo*) are then exposed to a drug such as ganciclovir, acyclovir or its analogues (FIAU, FIAC, DHPG). Such drugs are known to be phosphorylated by HSVTK (but not by cellular thymidine kinase) to their corresponding active nucleotide triphosphate forms. Acyclovir and FIAU triphosphates inhibit cellular polymerases in general, leading to the specific destruction of cells expressing HSVTK in transgenic mice (*see* Borrelli et al., Proc. Natl. A cad. Sci. USA 85:7572. 1988). Those cells containing the recombinant vector and expressing HIV tat protein are selectively killed by the presence of a specific dose of these drugs.

Retroviral vector constructs, *gag*/*pol* expression cassettes and *env* expression cassettes described herein may also direct the expression of one or more sequences which encode immunogenic portions of disease-associated antigens. As utilized within the context of the present invention, antigens are deemed to be "disease-associated" if they are either associated with rendering a cell (or organism) diseased, or arc associated with the disease-state in general but are not required or essential for rendering the cell diseased. In addition, antigens are considered to be "immunogenic" if they are capable, under appropriate conditions, of causing an immune response (either cell-mediated or humoral). Immunogenic "portions" may be of variable size, but are preferably at least 9 amino acids long, and may include the entire antigen.

A wide variety of "disease-associated" antigens are described herein including for example immunogenic, non-tumorigenic forms of altered cellular components which are normally associated with tumor cells *(see* WO 93/10814). Representative examples of altered cellular components which are normally associated with tumor cells include ras* (wherein "*" is understood to refer to antigens which have been altered to be non-tumorigenic), p53*, Rb*, altered protein encoded by Wilms' tumor gene, ubiquitin*, mucin, protein encoded by the DCC. APC, and MCC genes, as well as receptors or receptor-like structures such as neu, thyroid hormone receptor, Platelet Derived Growth Factor ("PDGF") receptor, insulin receptor, Epidermal Growth Factor ("EGF") receptor, and the Colony Stimulating Factor ("CSF") receptor.

"Disease-associated" antigens should also be understood to include all or portions of various eukaryotic, prokaryotic or viral pathogens. Representative examples of viral pathogens include the Hepatitis B Virus ("HBV") and Hepatitis C Virus ("HCV"; *see* WO 93/15207), Human Papiloma Virus ("HPV"; *see* WO 92/05248; WO 90/10459; EPO 133,123), Epstein-Barr Virus ("EBV"; *see* EPO 173,254: JP 1,128,788; and U.S. Patent Nos. 4,939,088 and 5,173,414), Feline Leukemia Virus ("FeLV"; *see* WO 93/09070; EPO 377,842; WO 90/08832; WO 93/09238), Feline. Immunodeficiency Virus ("FIV"; U.S. Patent No. 5,037,753; WO 92/15684; WO 90/13573; and JP 4,126,085), HTLV I and II, and Human Immunodeficiency Virus ("HIV"; *see* WO 93/02805).

The retroviral vector constructs, *gag*/*pol* expression cassettes and *env* expression cassettes described above may also direct the expression of one or more immune accessory molecules. As utilized herein, the phrase "immune accessory molecules" refers to molecules which can either increase or decrease the recognition, presentation or activation of an immune response (either cell-mediated or humoral). Representative examples of immune accessory molecules include a interferon, b interferon, IL-1, IL-2. IL-3, IL-4, IL-5, IL-6, IL-7 (U.S. Patent No.4,965,195), IL-8, IL-9, IL-10, IL-11, IL-12 (Wolf et al., J. Immun. 46:3074, 1991; Gubler et al., PNAS 88:4143, 1991; WO 90/05147; EPO 433,827), IL-13 (WO 94/04680), IL-14, IL-15, GM-CSF, M-CSF-1, G-CSF, CD3 (Krissancn et al., Immunogenetics 26:258-266, 1987). CD8. ICAM-1 (Simmons ct al., Nature 331:624-627, 1988), ICAM-2 (Singer, Science 255: 1671, 1992), h-microglobulin (Parnes et al., PNAS 78:2253-2257, 1981), LFA-1 (Altmann et al., Nature 338: 521, 1989), LFA3 (Wallner et al., J. Exp. Med 166(4):923-932, 1987), HLA Class 1, HLA Class II molecules, B7 (Freeman et al., J. Immun. 143:2714, 1989), and B7-2. For example, the heterologous gene encodes gamma-interferon.

The retroviral vector constructs described herein may direct the expression of more than one heterologous sequence. Such multiple sequences may be controlled either by a single promoter, or preferably, by additional secondary promoters (*e.g.*, Internal Ribosome Binding Sites or "IRBS" also known as Internal Ribosome Entry Sites or "IRES"). Retroviral vector constructs are described herein which direct the expression of heterologous sequences which act synergically. For example, retroviral vector constructs are described which direct the expression of a molecule such as IL-15, IL-12, IL-2, gamma interferon, or other molecule which acts to increase cell-mediated presentation in the T_{H}1 pathway, along with an immunogenic portion of a disease-associated antigen. In such cases, immune presentation and processing of the disease-associated antigen will be increased due to the presence of the immune accessory molecule.

Furthermore, retroviral vector constructs are described which direct the expression of one or more heterologous sequences which encode "replacement" genes. As utilized herein, it should be understood that the term "replacement genes" refers to a nucleic acid molecule which encodes a therapeutic protein that is capable of preventing, inhibiting, stabilizing or reversing an inherited or noninherited genetic defect. Representative examples of such genetic defects include disorders in metabolism, immune regulation, hormonal regulation, and enzymatic or membrane associated structural function. Representative examples of diseases caused by such defects include Cystic Fibrosis ("CF"; *see* Dorin et al., Nature 326:614,), Parkinson's Disease, Adenosine Deaminase deficiency ("ADA"; Hahma et al., J. Bact. 173:3663-3672, 1991), b-globin disorders, Hemophilia A & B (Factor VIII-deficiencies; *see* Wood et al., Nature 312:330, 1984), Gaucher disease, diabetes, forms of gouty arthritis and Lesch-Nylan disease (due to "HPRT" deficiencies; *see* Jolly et al., PNAS 80:477-481. 1983) and Familial Hypercholesterolemia (LDL Receptor mutations; *see* Yamamoto et al., Cell 39:27-38, 1984).

Sequences which encode the above-described heterologous genes may be readily obtained from a variety of sources. For example, plasmids which contain sequences that encode immune accessory molecules may be obtained from a depository such as the American Type Culture Collection (ATCC, Rockville, Maryland), or from commercial sources such as British Bio-Technology Limited (Cowley. Oxford England). Representative sources sequences which encode the above-noted immune accessory molecules include BBG 12 (containing the GM-CSF gene coding for the mature protein of 127 amino acids), BBG 6 (which contains sequences encoding gamma interferon), ATCC No. 39656 (which contains sequences encoding TNF), ATCC No. 20663 (which contains sequences encoding alpha interferon). ATCC Nos. 31902, 31902 and 39517 (which contains sequences encoding beta interferon), ATCC No 67024 (which contains a sequence which encodes lnterieukin-1). ATCC Nos. 39405. 39452, 39516, 39626 and 39673 (which contains sequences encoding Interleukin-2), ATCC Nos. 59399, 59398, and 67326 (which contain sequences encoding Interleukin-3), ATCC No. 57592 (which contains sequences encoding Interleukin-4), ATCC Nos. 59394 and 59395 (which contain sequences encoding Interleukin-5), and ATCC No. 67153 (which contains sequences encoding Interleukin-6). It will be evident to one of skill in the art that one may utilize either the entire sequence of the protein, or an appropriate portion thereof which encodes the biologically active portion of the protein.

Alternatively, known cDNA sequences which encode cytotoxic genes or other heterologous genes may be obtained from cells which express or contain such sequences. Briefly, within one embodiment mRNA from a cell which expresses the gene of interest is reverse transcribed with reverse transcriptasc using oligo dT or random primers. The single stranded cDNA may then be amplified by PCR *(see* U.S. Patent Nos. 4,683.202, 4,683,195 and 4,800,159. *See also* PCR Technology: Principles and Applications for DNA Amplification, Erlich (ed.). Stockton Press, 1989 all of which are incorporated by reference herein in their entirety) utilizing oligonucleotide primers complementary to sequences on either side of desired sequences. In particular, a double stranded DNA is denatured by heating in the presence of heat stable Taq polymerase, sequence specific DNA primers, ATP. CTP. GTT and TTP. Double-stranded DNA is produced when synthesis is complete. This cycle may be repeated many times, resulting in a factorial amplification of the desired DNA.

Sequences which encode the above-described genes may also be synthesized, for example, on an Applied Biosystems Inc. DNA synthesizer (*e.g.*, ABI DNA synthesizer model 392 (Foster City, California)).

### PREPARATION OF RETROVIRAL PACKAGING CELL LINES, AND GENERATION OF RECOMBINANT VIRAL PARTICLES

As noted above, the *gag*/*pol* expression cassettes and env expression cassettes may be used to generate transduction competent retroviral vector particles by introducing them into an appropriate parent cell line in order to create a packaging cell line, followed by introduction of a retroviral vector construct, in order to create a producer cell line (*see* WO 92/05266). Such packaging cell lines, upon introduction of an N2-type vector construct (Armentano et al., J. of Vir. 61(5):1647-1650, 1987) produce a titer of greater than 10⁵ cfu/ml, and preferably greater than 10-fold. 20-fold, 50-fold, or 100-fold higher titer than similar transduced PA317 cells (Miller and Buttimore. Mol. and Cell. Biol. 6(8):2895-2902. 1986).

Herewith methods for creating packaging cell lines are described, comprising the steps of (a) introducing a *gag*/*pol* expression cassette according into an animal cell, (b) selecting a cell containing a *gag*/*pol* expression cassette which expresses high levels of *gag*/*pol,* (c) introducing an *env* expression cassette into the selected cell, and (d) selecting a cell which expresses high levels of *env,* and thereby creating the packaging cell. Methods for creating packaging cell lines are described comprising the steps of (a) introducing an env expression cassette into an animal cell (b) selecting a cell which expresses high levels of *env,* (c) introducing a *gag*/*pol* expression cassette into the selected cell, and (d) selecting a cell containing a *gag*/*pol* expression cassette which expresses high levels of *gag*/*pol,* and thereby creating the packaging cell. As utilized herein, it should be understood that "high" levels of gag/pol or env refers to packaging cells which produce at least z times greater gag/pol or env protein than PA317 cells, wherein z is selected from the group consisting of 1, 2, 3, 4, 5, 6, 7, 8, 9 or 10.

A wide variety of animal cells may be utilized to prepare the packaging cells useful in the context of the present invention, including for example cells obtained from vertebrates, warm-blooded animals, or, mammals such as human, feline, goat, bovine, sheep, caprine, macaque, dog, rat and mouse cells. Particularly preferred cell lines for use in the preparation of packaging cell lines useful in the context of the present invention are those that lack genomic sequences which are homologous to the retroviral vector construct, *gag*/*pol* expression cassette and *env* expression cassette to be utilized. Methods for determining homology may be readily accomplished by, for example, hybridization analysis *(see* Martin el al., PNAS 78:4892-4896, 1981; *see also* WO 92/05266).

Expression cassettes used in the present invention may be introduced into cells by numerous techniques, including for example, transfection by various physical methods, such as electroporation, DEAE dextran, lipofection (Felgner et al., Proc. Natl. Acad. Sci. USA 84:7413-7417, 1989), direct DNA injection (Acsadi et al., Nature 352:815-818, 1991); microprojectile bombardment (Williams et al., PNAS 88:2726-2730, 1991), liposomes of several types (*see e.g.,* Wang et al., PNAS 84:7851-7855. 1987); CaPO₄ (Dubensky et al., PNAS 81:7529-7533, 1994), DNA ligand (Wu et al. J. of Biol. Chem. 264:16985-16987, 1989), administration of nucleic acids alone (WO 90/11092), or administration of DNA linked to killed adenovirus (Curiel et al., Hum. Gene Ther. 3: 147-154, 1992).

Producer cell lines (also called vector-producing lines or "VCLs") may then be readily prepared by introducing a retroviral vector construct into a packaging cell line via transfection as described above, or, via transduction . Producer cell lines are described comprising a *gag*/*pol* expression cassette, an *env* expression cassette, and a retroviral vector construct, wherein the *gag*/*pol* expression cassette, *env* expression cassette and retroviral vector construct lack a consecutive sequence of greater than 20, preferably 15, more preferably 10, and most preferably 10 or 8 nucleotides in common.

### PHARMACEUTICAL COMPOSITIONS

Pharmaceutical compositions are described comprising a recombinant viral particle as described above, in combination with a pharmaceutically acceptable carrier or diluent. Such pharmaceutical compositions may be prepared either as a liquid solution, or as a solid form (*e.g.,* lyophilized) which is suspended in a solution prior to administration. In addition, the composition may be prepared with suitable carriers or diluents for topical administration, injection, or oral, nasal, vaginal, sub-lingual, inhalant or rectal administration.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH (such as phosphate-buffered saline or Tris-buffered saline), mannitol, dextrose, glycerol, and ethanol, as well as polypeptides or proteins such as human serum albumin. A particularly preferred composition comprises a retroviral vector construct or recombinant viral particle in 1 mg/ml HSA, 18.75 mM Tris, pH 7.2, 37.5 mM NaCl and 40.0 mg/ml lactose. In this case, since the recombinant vector represents approximately 1 mg of material, it may be less than 1% of high molecular weight material, and less than 1/100,000 of the total material (including water). This composition is stable at -70°C for at least six months.

Pharmaceutical compositions useful in the context of the present invention may also additionally include factors which stimulate cell division, and hence, uptake and incorporation of a recombinant retroviral vector. Representative examples include Melanocyte Stimulating Hormone (MSH), for melanomas or epidermal growth factor for breast or other epithelial carcinomas.

Particularly preferred methods and compositions for preserving recombinant viruses are described in U.S. applications entitled "Methods for Preserving Recombinant Viruses" (*see* WO 94/11414).

### METHODS OF ADMINISTRATION

Methods are described for inhibiting or destroying pathogenic agents in a warm-blooded animal, comprising administering to a warm-blooded animal a recombinant viral particle as described above, such that the pathogenic agent is inhibited or destroyed. Recombinant viral particles may be administered *in vivo,* or ex vivo. Representative routes for *in vivo* administration include intradermally ("i.d."), intracranially ("i.c."), intraperitoneally ("i.p."), intrathecally ("i.t."), intravenously ("i.v."), subcutaneously ("s.c."), intramuscularly ("i.m.") or even directly into a tumor.

Alternatively, the cytotoxic genes, antisense sequences, gene products, retroviral vector constructs or viral particles used in the present invention may also be administered to a warm-blooded animal by a variety of other methods. Representative examples include transfection by various physical methods, such as lipofection (Felgner et al., Proc. Natl. Acad Sci. USA 84:7413-7417, 1989), direct DNA injection (Acsadi et al., Nature 352:815-818, 1991); microprojectile bombardment (Williams et al., PNAS 88:2726-2730, 1991); liposomes of several types (*see e.g.,* Wang et al., PNAS 84:7851-7855, 1987); CaPO₄ (Dubensky et al., PNAS 81:7529-7533, 1984); DNA ligand (Wu et al, J. of Biol. Chem. 264:16985-16987, 1989); administration of nucleic acids alone (WO 90/11092); or administration of DNA linked to killed adenovirus (Curiel et al., Hum. Gene Ther. 3: 147-154, 1992).

Furthermore retroviral particles (or retroviral vector constructs alone) may be utilized in order to directly treat pathogenic agents such as a tumor. Some of the retroviral particles or retroviral vector constructs described above may be directly administered to a tumor, for example, by direct injection into several different locations within the body of tumor. Alternatively, arteries which serve a tumor may be identified, and the vector injected into such an artery, in order to deliver the vector directly into the tumor. In another example a tumor which has a necrotic center may be aspirated, and the vector injected directly into the now empty center of the tumor. Within another example, the retroviral vector construct may be directly administered to the surface of the tumor, for example, by application of a topical pharmaceutical composition containing the retroviral vector construct, or preferably, a recombinant retroviral particle.

Furthermore methods are described for inhibiting the growth of a selected tumor in a warm-blooded animal, comprising the steps of (a) removing tumor cells associated with the selected tumor from a warm-blooded animal, (b) infecting the removed cells with a retroviral vector construct which directs the expression of at least one anti-tumor agent, and (c) delivering the infected cells to a warm-blooded animal, such that the growth of the selected tumor is inhibited by immune responses generated against the gene-modified tumor cell. Within the context of the present invention, "inhibiting the growth of a selected tumor" refers to either (1) the direct inhibition of tumor cell division, or (2) immune cell mediated tumor cell lysis, or both, which leads to a suppression in the net expansion of tumor cells. Inhibition of tumor growth by either of these two mechanisms may be readily determined by one of ordinary skill in the art based upon a number of well known methods (*see* U.S. Serial No. 08/032,846). Examples of compounds or molecules which act as anti-tumor agents include immune accessory molecules, cytotoxic genes, and antisense sequences as discussed above (*see also* U.S. Serial No. 08/032.846).

Cells may be removed from a variety of locations including, for example, from a selected tumor. In addition, a vector construct may be inserted into non-tumorigenic cells, including for example, cells from the skin (dermal fibroblasts), or from the blood (*e.g.*, peripheral blood leukocytes). If desired, particular fractions of cells such as a T cell subset or stem cells may also be specifically removed from the blood (*see*, for example, PCT WO 91/16116, an application entitled "Immunoselection Device and Method"). Vector constructs may then be contacted with the removed cells utilizing any of the above-described techniques, followed by the return of the cells to the warm-blooded animal, preferably to or within the vicinity of a tumor. Subsequent to removing tumor cells from a warm-blooded animal, a single cell suspension may be generated by, for example, physical disruption or proteolytic digestion. In addition, division of the cells may be increased by addition of various factors such as melanocyte stimulating factor for melanomas or epidermal growth factor for breast carcinomas, in order to enhance uptake, genomic integration and expression of the recombinant viral vector.

Within the context of the present invention, it should be understood that the removed cells may not only be returned to the same animal, but may also be utilized to inhibit the growth of selected tumor cells in another, allogeneic, animal. In such a case it is generally preferable to have histocompatibility matched animals (although not always, *see*, *e.g.* Yamamoto et al., "Efficacy of Experimental FIV Vaccines," Ist International Conference of FIV Researcher, University of California at Davis, September 1991).

The above-described methods may additionally comprise the steps of depleting fibroblasts or other non-contaminating tumor cells subsequent to removing tumor cells from a warm-blooded animal, and/or the step of inactivating the cells, for example, by irradiation.

As noted above, several anti-tumor agents may be administered either concurently or sequentially, in order to inhibit the growth of a selected tumor in accordance with the methods of the present invention. For example an anti-tumor agent such as g-IFN may be co-administered or sequentially administered to a warm-blooded animal along with other anti-tumor agents such as IL-2, or IL-12, in order to inhibit or destroy a pathogenic agent. Such therapeutic compositions may be administered directly utilizing a single vector construct which directs the expression of at least two anti-tumor agents, or, within other embodiments, expressed from independent vector constructs. Alternatively, one anti-tumor agent (*e.g.*, g-IFN) may be administered utilizing a vector constructs, while other tumor agents (*e.g.*. IL-2) arc administered directly (*e.g.*, as a pharmaceutical composition intravenously).

Retroviral vector constructs which deliver and express both a g-IFN gene and another gene encoding IL-2, may be administered to the patient. In such constructs, one gene may be expressed from the retrovector LTR and the other may utilize an additional transcriptional promoter found between the LTRs, or may be expressed as a polycistronic mRNA, possibly utilizing an internal ribosome binding site. After *in vivo* gene transfer, the patient's immune system is activated due to the expression of g-IFN. Infiltration of the dying tumor with inflammatory cells, in turn, increases immune presentation and further improves the patient's immune response against the tumor.

Furthermore methods are described for generating an immune response against an immunogenic portion of an antigen, in order to prevent or treat a disease (*see, e.g.,* U.S. Serial Nos. 08/104,424; 08/102,132, 07/948.358; 07/965,084), for suppressing graft rejection, (*see* U.S. Serial No. 08/116,827), for suppressing an immune response (*see* U.S. Serial No. 08/116,828), and for suppressing an autoimmune response *(see* U.S. Serial No. 08/116,983).

As will be understood by one of ordinary skill in the art given the disclosure provided herein, any of the retroviral vector constructs described herein may be delivered not only as a recombinant viral particle, but as direct nucleic acid vectors. Such vectors may be delivered utilizing any appropriate physical method of gene transfer including for example, those which have been discussed above.

The following examples are offered by way of illustration, and not by way of limitation.

### EXAMPLE 1

### CONSTRUCTION OF RETROVECTOR BACKBONES

### A. Preparation of a Retroviral vector construct That Does Not Contain an Extended Packaging Sequence (Y)

This example describes the construction of a retroviral vector construct using site-specific mutagenesis. Within this example, a MoMLV retroviral vector construct is prepared wherein the packaging signal "Y" of the retrovector is terminated at basepair 617 of SEQ ID NO: 1, thereby eliminating the ATG start of *gag.* Thus, no crossover can occur between the retroviral vector construct and the *gaglpol* expression cassette which is described below in Example 3.

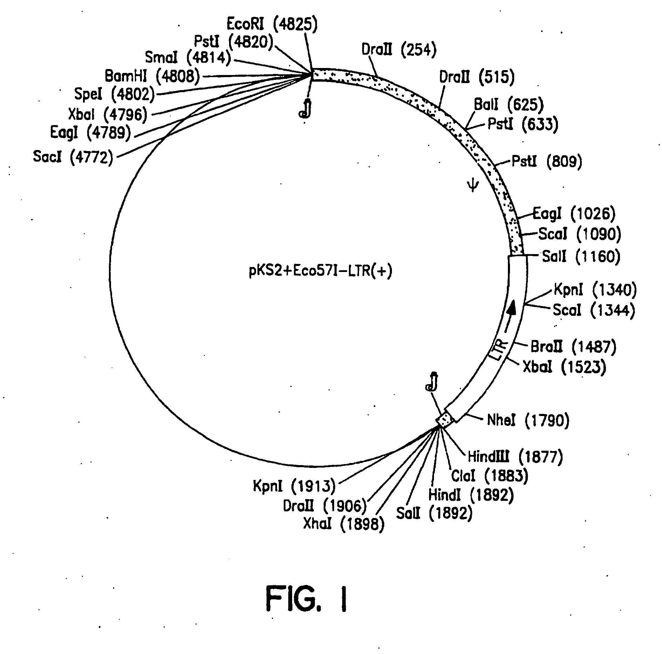

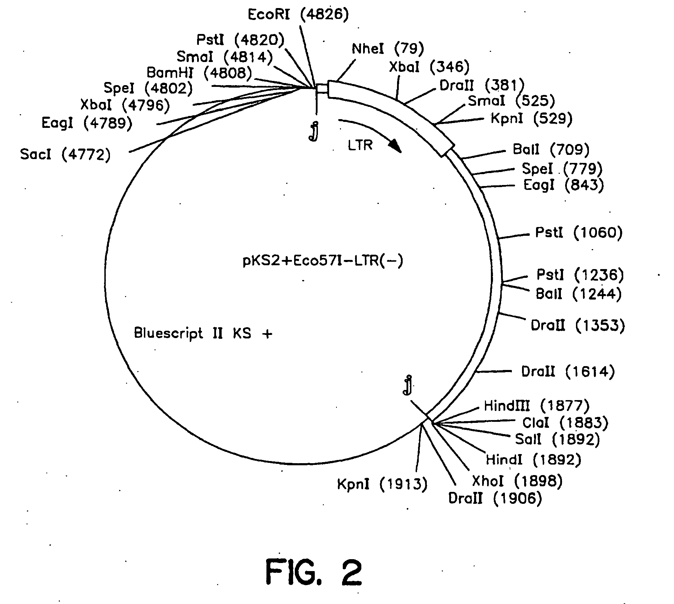

Briefly, pMLV-K (Miller, J. Virol 49:214-222, 1984 - an infectious clone derived from pMLV-1 Shinnick et al., Nature, 293:543-548, 1981) is digested with *Eco*57I, and a 1.9kb fragment is isolated: (*Eco*571 cuts upstream from the 3' LTR, thereby removing all env coding segments from the retroviral vector construct.) Then fragment is then blunt ended with T4 polymerase (New England Biolabs), and all four deoxynucleotides, and cloned into the EcoRV site of phagemid pBluescript II KS+ (Stratagene, San Diego, Calif.). This procedure yields two constructs, designated pKS2+Eco57I-LTR(+) (Figure 1) and pKS2+Eco57I-LTR(-) (Figure 2), which are screened by restriction analysis. When the (+) single stranded phagemid is generated, the sense sequence of MoMLV is isolated.

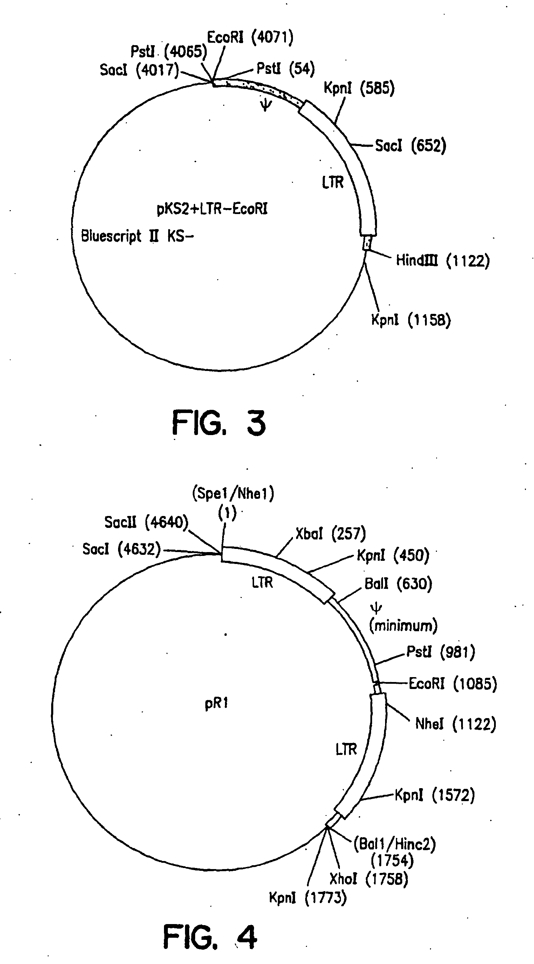

A new *EcoRI* site is then created in construct pKS2+*Eco*57I-L TR(+) in order to remove the ATG start codon of *gag.* In particular, an *Eco*RI site is created using the single stranded mutagenesis method of Kunkle (PNAS 82:488, 1985). pKS2+*Eco*57I-LTR(+) is a pBluescript^{™} II + phagemid (Strategene, San Diego, Calif.) containing an Eco57I fragment from pMLV-K. It includes the MoMLV LTR and downstream sequence to basepair 1378. When single stranded phagemid is generated the sense sequence of MoMLV is isolated. The oligonucleotide, 5'-GGT AAC AGT CTG GCC CGA ATT CTC AGA CAA ATA CAG (SEQ ID NO: 2), is created and used to generate an *Eco*RI site at basepairs 617-622. This construct is designated pKS2+LTR-EcoRI (Figure 3).

### B. Substitution of Nonsense Codons in the Extended Packaging Sequence (Y+)

This example describes modification of the extended packaging signal (Y+) by site-specific mutagenesis. In particular, the modification will substitute a stop codon, TAA, at the normal ATG start site of *gag* (position 621-623 of SEQ ID NO: 1), and an additional stop codon TAG at position 637-639 of SEQ ID NO: 1.

Briefly, an *Eco*57I - *Eco*RI fragment (MoMLV basepairs 7770 to approx. 1040) from pN2 (Amentano et al., J. Virol. 61:1647-1650, 1987) is first cloned into pBluescript II KS+ phagemid at the *Sac*II and *Eco*RI sites (compatible). Single stranded phagemid containing antisense MoMLV sequence, is generated using helper phage M13K07 (Stratagene, San Diego, Calif.). The oligonucleotide 5'-CTG TAT TTG TCT GAG AAT TAA GGC TAG ACT GTT ACC AC (SEQ ID NO: 3) is synthesized, and utilize according to the method of Kunkle as described above, in order to modify the sequence within the Y region to encode stop codons at nucleotides 621-623 and 637-639.

### C. Removal of Retroviral Packaging Sequence Downstream from the 3' LTR

Retroviral packaging sequence which is downstream from the 3' LTR is deleted essentially as described below. Briefly, pKS2+*Eco*57I-LTR(-) (Figure 2) is digested with *Ball* and *Hinc*II, and relegated excluding the *Ball* to *Hinc*II DNA which contains the packaging region of MoMLV.

### D. Construction of Vector Backbones

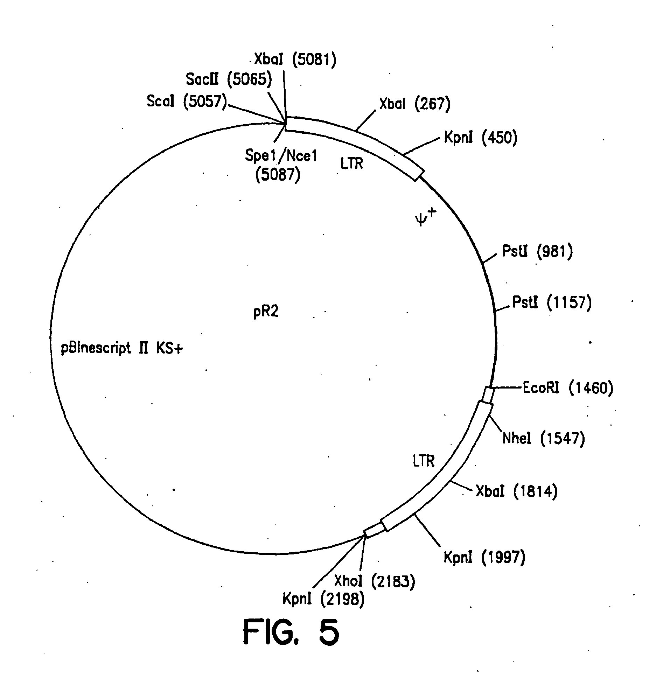

Constructs prepared in sections A arid C above, or alternatively from sections B and C above, are combined with a plasmid vector as described below, in order to create a retrovector backbone containing all elements required *in cis,* and excluding all sequences of 8 nucleic acids or more contained in the retroviral portion of the *gag-pol* and *env* expression elements *(see* Examples 3 and 4).
1. Parts A and C are combined as follows: The product of A is digested with *Nhe*I and *Eco*RI*,* and a 1034 basepair fragment containing the LTR and minimal Y is isolated. The fragment is ligated into the product of part C at the unique (compatible) restriction sites *Spe*I and *Eco*RI. The resultant construct is designated pRI (Figure 4)
2. Parts B and C are combined as follows: The product of B is digested with *Nhe*I and *Eco*RI and a 1456 basepair fragment containing the LTR and modified Y+ region is isolated. The fragment is ligated into the product of C at the unique (compatible) restriction sites *Spe*I and *Eco*RI*.* The resultant construct is designated pR2 (Figure 5).

### EXAMPLE 2

### INSERTION OF A GENE OF INTEREST INTO PR1 AND PR2

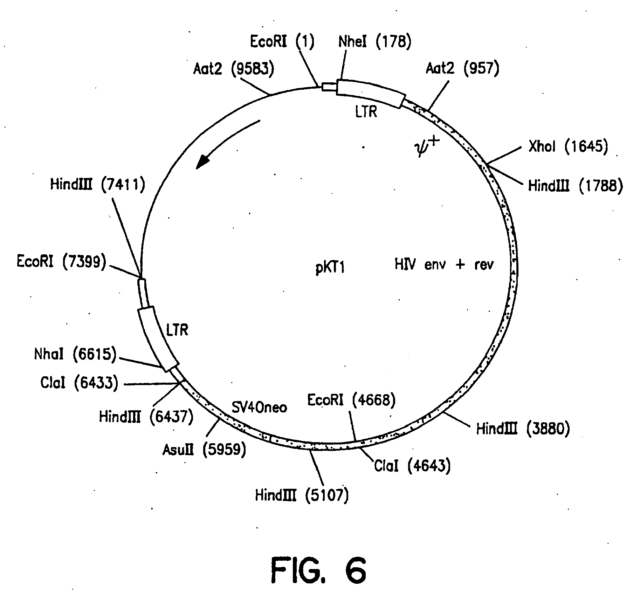

This example describes the insertion of a gene of interest, gp120, gp41, and rev along with a selectable marker into either pR1 or pR2. Briefly, the sequence encoding gp120, gp41 and rev is taken from construct pKT1 (Figure 6; *see also* Chada et al., J. Vir. 67:3409-3417, 1993); note that this vector is also referred to as N2IIIBenv. In particular, pKT1 is first digested at the unique *Asu*II site (position 5959). The ends are blunted, and an *Xho* I linker is ligated at that site. (New England Biolabs). The construct is then digested with *Xho* I, and a 4314 bp fragment containing HIV envelope (gp120 and gp41), rev, SV40 early promoter and G418 resistance genes is isolated.

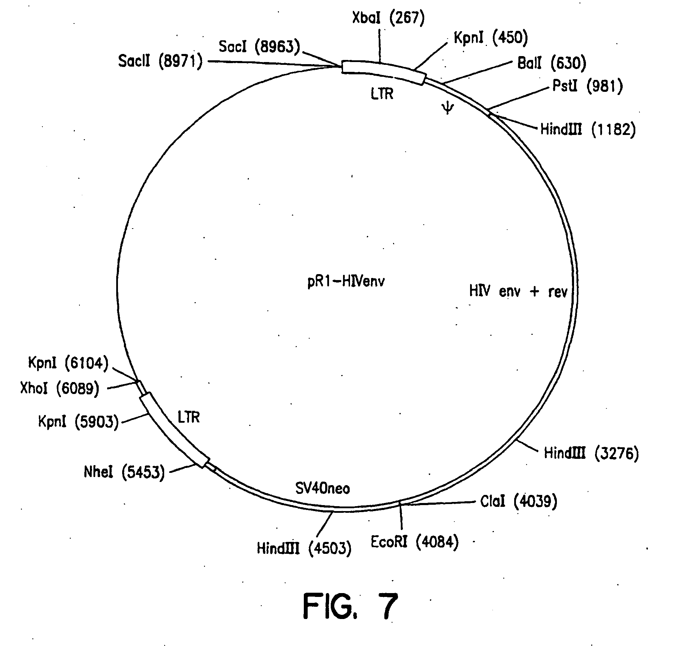

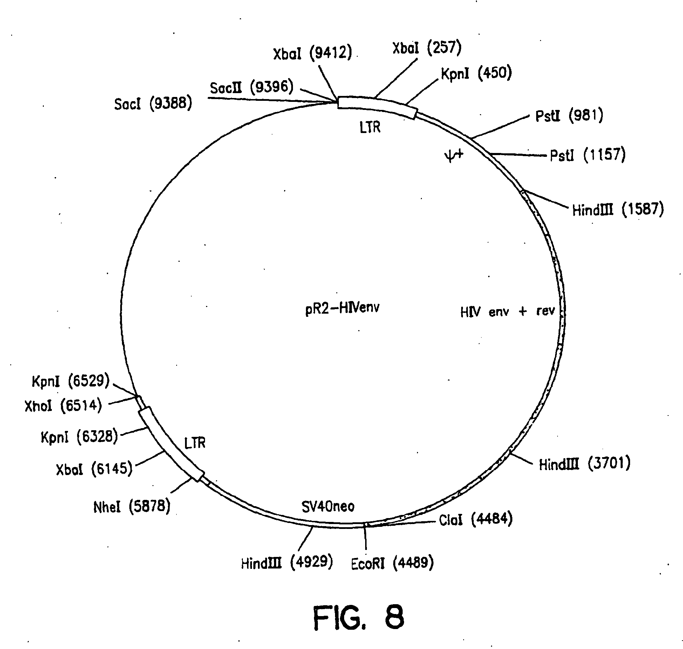

pR1 or pR2 is digested at the unique *Eco* R1 restriction site, blunted, and *Sal* I linkers (New England Biolabs) are ligated in. The 4314 bp KT1 fragment, is then ligated into pR1 or pR2 at the new *Sal* I sites, and the correct orientation is determined *(see* Figures 7 and 8). In both of these constructs, (pR1-HIVenv and pR2-HIVenv) the HIV genes are expressed from the MLV LTR, and G418 resistance is expressed from the SV40 promoter.

### EXAMPLE 3

### CONSTRUCTION OF GAG-POL EXPRESSION CASSETTES

### A. Construction of an Expression Cassette Backbone, pHCMU-PA

A vector is first created in order to form the backbone for both the *gag*/*pol* and *env* expression cassettes. Briefly, pBluescript SK- phagemid (Stratagene, San Diego, Calif.; GenBank accession number 52324; referred to as "SK-") is digested with *Spe*I and blunt ended with Klenow. A blunt end *Dra*I fragment of SV40 (Fiers et al., "Complete nucleotide sequence of SV40 DNA" Nature 273:113-120, 1978) from DraI (bp 2366) to DraI (bp2729) is then inserted into SK-, and a construct isolated in which the SV40 late polyadenylation signal is oriented opposite to the LacZ gene of SK-. This construct is designated SK-SV40A.

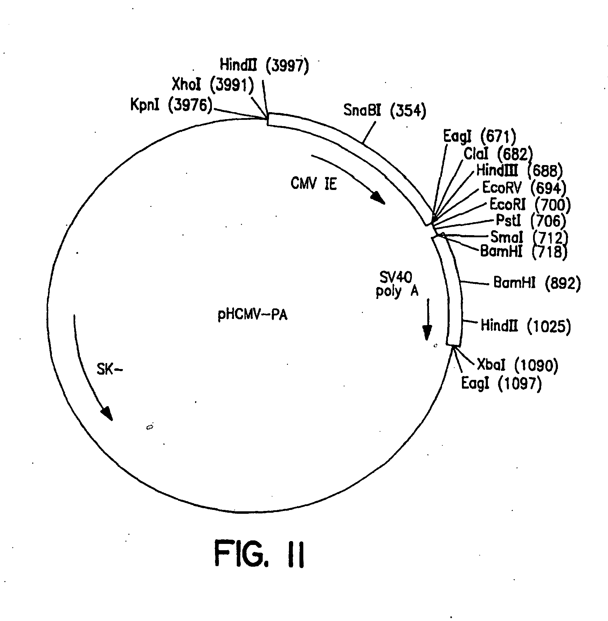

A Human Cytomegalovirus Major Immediate Early Promoter ("HCMV-IE"; Boshart et al., Cell 41:521-530, 1985) (*Hinc*II, bp 140, to *Eag*I*,* bp814) is isolated after digestion with *Hinc*II and *Eag*I*,* and the *Eag*I site blunt ended. The 674 blunt ended fragment, is ligated into SK-SV40A. The final construct, designated pHCMV-PA is then isolated *(see* Figure 11). This construct contains the HCMV promoter oriented in opposite orientation to the LacZ gene, and upstream from the late polyadenylation signal of SV40.

### B. Creation of New Codons for the 5' Gag

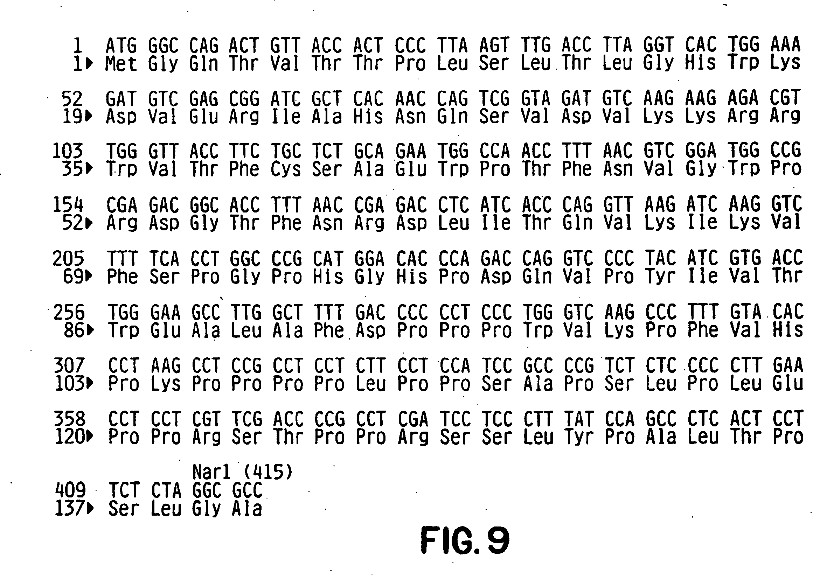

This example describes *gag*/*pol* expression cassettes that lack non-coding sequences upstream from the *gag* start, thereby reducing recombination potential between the *gag-pol* expression element and Y+ sequence of a retroviral vector construct, and inhibiting co-packaging of the *gag-pol* expression element along with the retrovector. In order to construct such an expression cassette, 448 bp of DNA is synthesized with the following features: 5' ATATATATAT ATCGAT(ClaI site) ACCATG(start codon, position 621) (SEQ ID NO: 4), followed by 410 bp encoding 136+ amino acid residues using alternative codons *(see* Figures 9 and 10), followed by GGCGCC(*Nar*I site)AAACCTAAAC 3' (SEQ ID NO: 5).

Briefly, each of oligos 15 through 24 (set forth below in Table 1) are added to a PCR reaction tube such that the final concentration for each is 1 µM. Oligos 25 and 26 are added to the tube such that the final concentration for each is 3 µM. 1.2 uL of 2.5 mM stock deoxynucleotide triphosphates (dG, dA, dT, dC) are added to the tube. 5 µL of 10X PCR buffer (Perkin Elmer). Water is added to a final volume of 50 µL. Wax beads are added and melted over the aqueous layer at 55°C and then cooled to 22°C. A top aqueous layer is added as follows: 5 µL 10X PCR buffer, 7.5 µL dimethylsulfoxide, 1.5 µL Taq polymerase (Perkin-Elmer) and 36 µL water. Forty cycles of PCR are then performed as follows: 94_C, 30 seconds; 56_C, 30 seconds; and 72_C, 30 seconds. The PCR product is stored at -20_C until assembly of the *gag*/*pol* expression cassette.

**Table 1**

| **SEQ. ID. No.** | **Sequence** |
|---|---|
| **15** | |
| **16** | |
| **17** | |
| **18** | |
| **19** | |
| **20** | |
| **21** | |
| **22** | |
| **23** | |
| **24** | |
| **25** | 5' ATA TAT ATA TAT CGA TAC C 3' |
| **26** | 5' GTT TAG GTT TGG CGC CGA GG 3' |

### C. Creation of a New 3' End for Pol

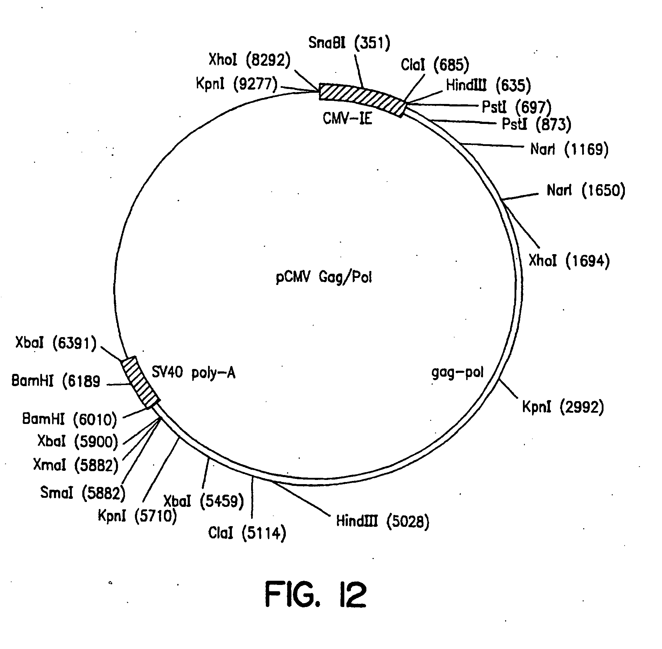

In order to prepare a *gag*/*pol* expression cassette which expresses full length *gag*/*pol,* pCMV *gag*/*pol* is constructed. Briefly, MoMLV sequence from *Pst*1 (BP567) to *Nhe*1 (bp 7847) is cloned into the *Pst*1*-Xba*1 sites of pUC19 (New England Biolabs). The resultant intermediate is digested with *Hin*dIII and *Xho*I, and a 1008 bp fragment containing the *gag* leader sequence is isolated. The same intermediate is also digested with *XhoI* and *Sca*I*,* and a 4312 bp fragment containing the remaining *gag* and *pol* sequences is isolated. The two isolated fragments are then cloned into the HindIII and *Sma*I sites of pHCMV-PA, described above. The resultant construct, designated CMV *gag*/*pol* (Figure 12) expresses MoMLV *gag* and *pol* genes.

In order to truncate the 3' end of the *pol* gene found in pCMV *gag-pol,* a 5531 basepair *Sna*BI - *Xma*I fragment containing a portion of the CMV IE promoter and all of *gag-pol* except the final 28 codons, is isolated from pCMV *gag-pol.* This fragment is cloned into the *Sna*BI and *Xma*I sites of pHCMV-PA. This construct expresses five new amino acids at the carboxy-terminus (Ser-Lys-Asn-Tyr-Pro) (SEQ ID NO: 6) (pCMV gpSma).

Alternatively, these five amino acids may be eliminated by digesting pCMVgp *Sma*I with *Sma*I and adding an *Nhe*I (termination codons in three phases) linker (5' - CTA GCT AGC TAG) (SEQ ID NO: 14; New England Biolabs) at the end of the truncated *pol* sequence. This construct is designated pCMV gp Nhe. Both of these constructs eliminates potential crossover between *gag*/*pol* and *env* expression cassettes.

### D. Gag-Pol Expression Cassette

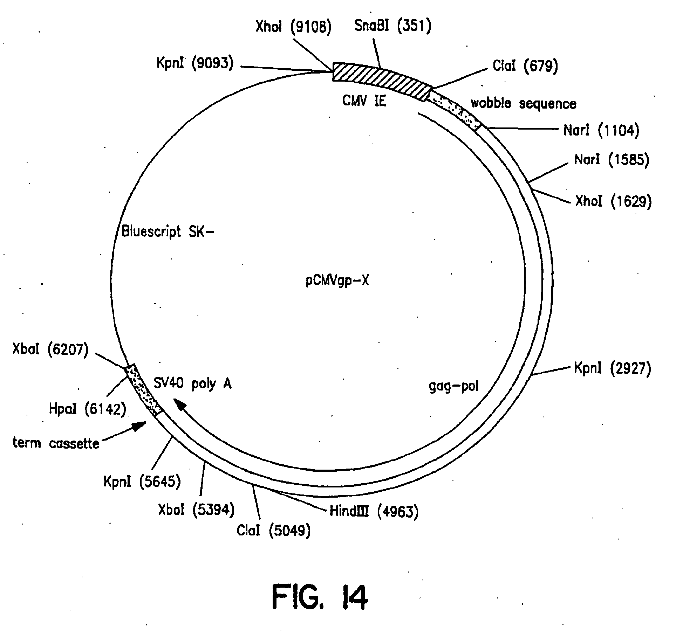

Parts B and C from above are combined to provide an expression vector containing a CMV IE promoter, *gag-pol* sequence starting from the new *Cla*I site (followed by ACC ATG and 412 bp of alternative or "wobble" *gag* coding sequence) and terminating at the *Sma*I site (MoMLV position 5750) followed by an SV40 polyadenylation signal, essentially as described below. Briefly, the approximately 451 bp double stranded wobble fragment from part A is ligated into pCR'II TA cloning vector (Invitrogen Corp.). The wobble PCR product naturally contains a 3' A-overhang at each end, allowing for cloning into the 3' T-overhang of pCR'II. The 422 bp *Cla*I *- Nar*I wobble fragment from the pCR'II clone is removed and is ligated into the *Cla*I (Position 679, Figure pCMV gp Sma) and *Nar*I (Position 1585) sites of pCMVgp *Sma*I (Part B) (or pCMV gp Nhe). (The *Cla*I site at position 5114 is methylated and not cut with *Cla*I)*.* The product of that ligation is digested with *Nar*I, and the MLV-K *Nar*I fragment (positions 1035 to 1378) is inserted (SEQ ID NO: 1). This construct is designated pCMVgp -X (Figure 14).

### EXAMPLE 4

### CONSTRUCTION OF ENV EXPRESSION CASSETTES

### A. Creation of a New 5' EagI Restriction Site

Starting with an *Eag*I- *Eco*RI 626 bp subfragment from a 4070A amphotropic envelope (Chattopodhyay et al., J Vir. 39:777, 1981; GenBank accession # MLV4070A, and #MLVENVC; SEQ ID NO: 13) cloned in a pBluescript II Ks+ vector (containing the start codon), site directed mutagenesis is performed upstream of the translation start site in order to change ACCATCCTCT GGACGGACAT G... (SEQ ID NO: 7; positions 19 - 39 of Genebank sequence # MLVENVC) to ACCCGGCCGT. GGACGGACAT G... (SEQ ID NO: 8) and create a new *Eag*I site at position 23. This modification allows cloning of the amphotropic envelope sequence into an expression vector eliminating upstream 4070A sequence homologous to the *gag-pol* expression element as described in Example 2A.

### B. Creation of a New 3' End for Env

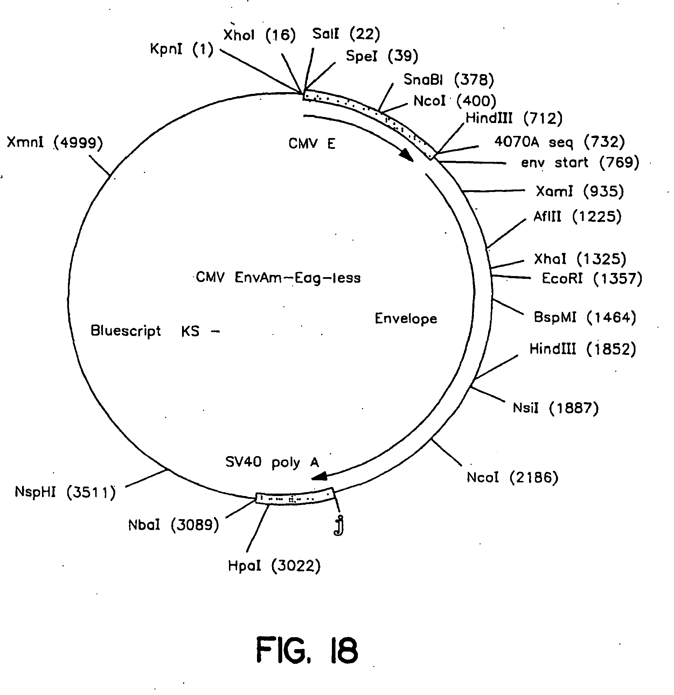

A new 3' end of the envelope expression element is created by terminating the sequence which encodes the R-peptide downstream from the end of the transmembrane region (pulse). Briefly, construct pHCMV-PA, described above, is first modified by digestion with *Not*I (position 1097), blunted and relegated to obliterate the overlapping Bluescript *Eag*I site at the same position. pCMV Envam-Eag-X-less is then constructed by digesting the modified pHCMV-PA with *Eag*I (position 671 and *Sma*1 (position 712) and ligating in two fragments. The first is an *EagI*-*NcoI* fragment from 4070A (positions 1-1455) (SEQ ID NO: 13). The second is an MLV-K envelope fragment, *Nco*I *- Pvu*II (positions 7227-7747) (SEQ ID NO: 1). The resultant construct from the three-way ligation contains the HCMV promoter followed by the SU (GP70) coding sequence of the 4070A envelope, the TM (p15e) coding sequence of MoMLV, and sequence encoding 8 residues of the R-peptide. In addition, this envelope expression cassette (pCMV Env am-Eag-X-less) (Figure 18) shares no sequence with crossless retrovector backbones described in Example 1.

### C. Envelope Expression Element

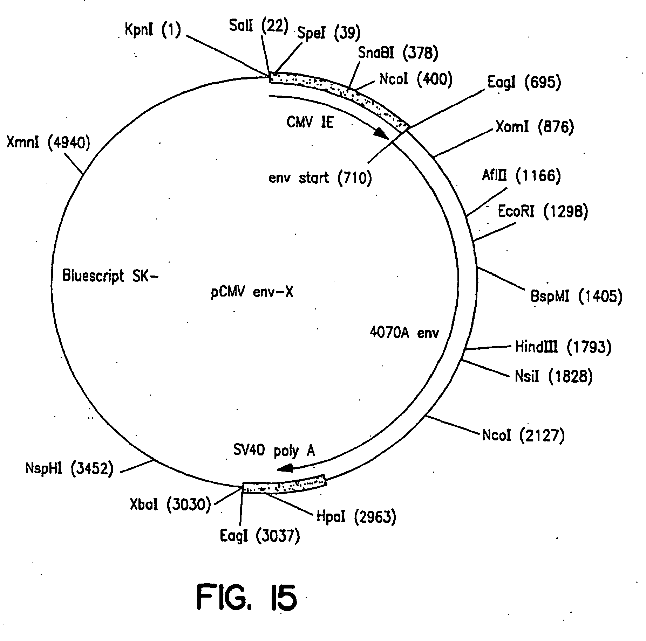

Parts A and B from above are combined to complete an amphotropic expression element containing the CMV promoter, 4070A SU, MoMLV TM and SV40 polyadenylation signal in a Bluescript SK- plasmid vector. This construct is called pCMVenv-X (Figure 15). Briefly, the construct described in part A with a new *Eagl* restriction site is digested with *Eag*1 and *XhoI,* and a 571 bp fragment is isolated. pCMV Envam-Eag-X-less (from part B) is digested with *Kpn*I and *Eag*I and the 695 bp fragment is reserved. pCMV Envam-Eag-X-less (from part B) is digested with *Kpn*I and *Xho*I and the 4649 bp fragment is reserved. These two fragments are ligated together along with the 571 bp *Eag*I to *Xho*I fragment digested from the PCR construct from part A. pCMVenv-X shares no sequence with crossless retrovector backbones nor the *gag-pol* expression element pCMVgp-X.

### EXAMPLE 5

### FUNCTIONALITY TESTS FOR GAG-POL AND ENV EXPRESSION CASSETTES

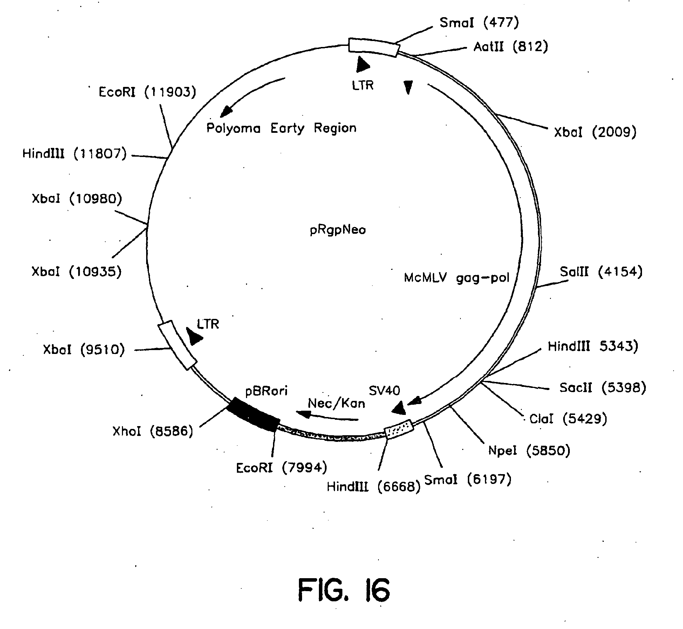

Rapid tests have, been developed in order to ensure that the *gag-pol* and env expression cassettes are biologically active. The materials for these tests consist of a cell line used for transient expression (typically 293 cells. ATCC #CRL 1573), a target cell line sensitive to infection (typically HIT 1080 cells, ATCC #CCL 121) and either pRgpNeo (Figure 16) or pLARNL (Emi et al., J. Virol 65:1202-1207, 1991). The two later plasmids express rescuable retrovectors that confer G418 resistance and also express *gag-pol,* in the case of RgpNeo or *env,* in the case of pLARNL. For convenience, the organization of RgpNeo (Figure 16) is set forth below.

In order to test expression cassettes such as pCMVgp-X for functionality of *gag*/*pol,* the plasmid is co-transfected with pLARNL at a 1: ratio into 293 cells. After 12 hours, the media is replaced with normal growth media. After an additional 24 hours, supernatant fluid is removed from the 293 cells, filtered through a 0.45 mm filter, and placed on HT 1080 target cells. Twenty-four hours after that treatment, the media is replaced with growth media containing 800 µg/ml G418. G418 resistant colonies are scored after one week. The positive appearance of colonies indicates that all elements are functional and active in the original co-transfection.

For convenience, the organization of RgpNeo (Figure 16) is set forth below:
Position 1 = left end of 5' LTR; Positions 1-6320 = MoMLV sequence from 5'LTR to Sca 1 restriction site; Positions 6321 - 6675 = SV40 early promoter; Positions 6676-8001 = Neo resistance gene from Tn 5 (including prokaryotic promoter); and Positions 8002 - 8606 = pBR origin of replication.

### EXAMPLE 6

### PACKAGING CELL LINE AND PRODUCER CELL LINE DEVELOPMENT

This example describes the production of packing and producer cell lines utilizing the above described retroviral vector constructs, *gag*/*pol* expression cassettes, and env expression cassettes, which preclude the formation of replication competent virus.

Briefly, for amphotropic MoMLV-based retroviral vector constructs, a parent cell line is selected which lacks sequences which are homologous to Murine Leukemia Viruses, such as the dog cell line D-17 (ATCC No. CCL 183). The *gaglpol* expression cassettes are then introduced into the cell by electroporation, along with a selectable marker plasmid such as DHFR (Simonsen et al., PNAS 80:2495-2499, 1983). Resistant colonies are then selected, expanded in 6 well plates to confluence, and assayed for expression of gag/pol by Western Blots. Clones are also screened for the production of high titer vector particles after transduction with pLARNL.

The highest titer clones are then electroporated with an *env* expression cassette and a selectable marker plasmid such as hygromycin *(see* Gritz and Davies, Gene 25:179-188, 1983). Resistant colonies are selected, expanded in 6 well plates to confluency, and assayed for expression of env by Western Blots. Clones are also screened for the production of high titer vector particles after transduction with a retroviral vector construct.

Resultant packaging cell lines may be stored in liquid Nitrogen at 10 x 10⁶ cells per vial, in DMEM containing 10% irradiated Fetal Bovine Serum, and 8% DMSO. Further testing may be accomplished in order to confirm sterility, and lack of helper virus production. Preferably, both an S+L- assay and a *Mus dunni* marker rescue assay should be performed in order to confirm a lack of helper virus production.

In order to construct a producer cell line, retroviral vector construct as described above in Example 1 is electroporated into a xenotropic packaging cell line made utilizing the methods described above. After 24-48 hours, supernatant fluid is removed from the xenotropic packaging cell line, and utilized to transduce a second packaging cell line, thereby creating the final producer cell line.

### EXAMPLE 7

### HELPER DETECTION ASSAY COCULTIVATION, AND MARKER RESCUE

This example describes a sensitive assay for the detection of replication competent retrovirus ("RCR"). Briefly, 5 x 10⁵ vector-producing cells are cocultivated with an equal number of *Mus dunni* cells (Lander and Chattopadhyay, *J Virol. 52*:695, 1984). *Mus dunni* cells are particularly preferred for helper virus detection because they are sensitive to nearly all murine leukemia-related viruses, and contain no known endogenous viruses. At three, six, and nine days after the initial culture, the cells are split approximately 1 to 10, and 5 x 10⁵ fresh *Mus dunni* cells are added. Fifteen days after the initial cocultivation of *Mus dunni* cells with the vector-producing cells, supernatant fluid is removed from cultures, filtered through a 0.45 mm filter, and subjected to a marker rescue assay.

Briefly, culture fluid is removed from a MdH tester cell line (*Mus dunny* cells containing pLHL (a hygromycin resistance marker retroviral vector; *see* Palmer et al., PNAS 84(4):1055-1059, 1987) and replaced with the culture fluid to be tested. Polybrene is added to a final concentration of 4 mg/ml. On day 2, medium is removed and replaced with 2 ml of fresh DMEM containing 10% Fetal Calf Serum. On day 3, supernatant fluid is removed, filtered, and transferred to HT1080 cells. Polybrene is added to a final concentration of 4mg/ml. On day 4, medium in the HT1080 cells is replaced with fresh DMEM containing 10% Fetal Calf Serum, and 100 mg/ml hygromycin. Selection is continued on days 5 through 20 until hygromycin resistant colonies can be scored, and all negative controls (e.g., mock infected MdH cells) are dead.

### EXAMPLE 8

### ASSAY FOR ENCAPSIDATION OF WOBBLE RNA SEQUENCE

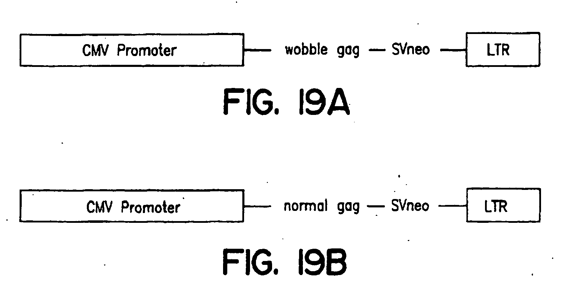

This example describes a sensitive assay for the detection of encapsidation of RNA from constructs containing wobble or normal gag sequence. Briefly, a fragment of DNA from a "wobble" *gag*/*pol* expression cassette (Example 3), containing the CMV promoter and gag sequence to the *Xho*I site (MoMLV position 1561) is ligated to a SV40 neo-3' LTR DNA fragment from N2 (Armentano et al., *supra)* or KT-3 *(see* WO 91/02805 or WO 92/05266). This construct is diagrammatically illustrated in Figure 19A, and is not expected to be encapsidated in packaging cell lines such as DA or HX (*see* WO 92/05266) because it lacks a 5' LTR and primer binding site.

A second construct is also made, similar to the first except that the wobble sequence is replaced by normal *gag* sequence. Similar to the first construct; the RNA transcribed from this DNA is not expected to be encapsidated. This construct is diagrammatically illustrated in Figure 19B.

The above constructs are separately transfected into a packaging cell line. The culture is then assayed for the ability to generate transducible G418-resistant retrovector. Neither construct results in transducible vector.

Cell cultures containing the above constructs are then transduced with the retrovector LHL *(see* Example 7). The cell cultures, after selection, will now generate retrovector conferring hygromycin resistance to target cells. Further, if co-encapsidation is allowed by interaction between LHL RNA and the transcripts from the above constructs, statistically significant RT-mediated recombination can occur resulting in the transfer of G418 resistance to target cells.

### EXAMPLE 9

### CONSTRUCTION OF RETROVIRAL BACKBONES

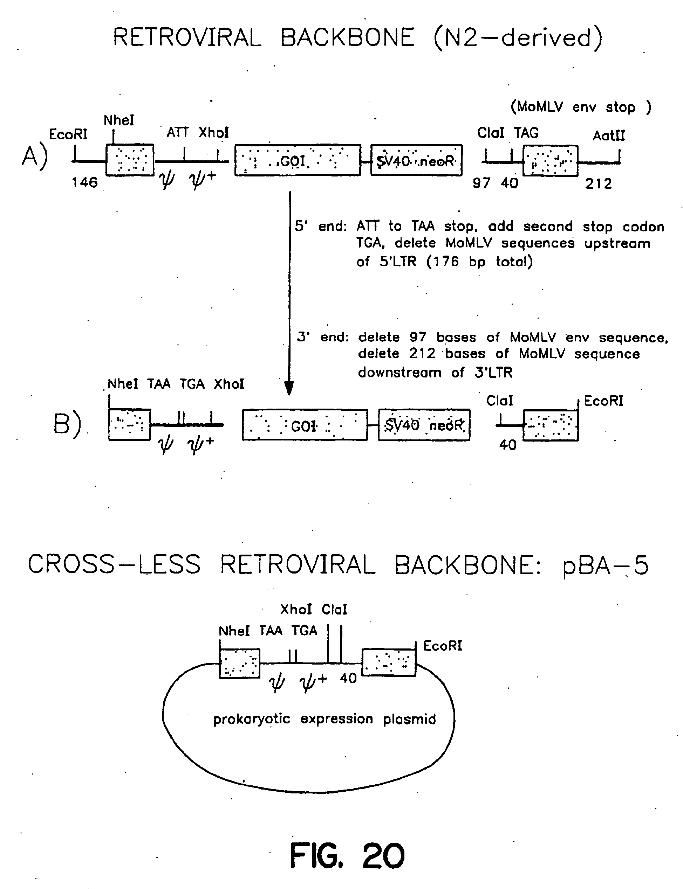

This example describes several modifications of the retroviral vector put (Figure 6) resulting in decreased sequence homology to the retroviral gag/pol and envelope expression constructs. In addition, two stop codons were introduced in the DNA sequence of the packaging signal sequence in order to increase the safety of these vectors. All modifications are summarized in Fig. 20 and the resulting retroviral backbone is called pBA-5b.

### A. Substitution of Nonsense Codons in the Extended Packaging Sequence (ψ+)

This example describes modification of the extended packaging signal (ψ+) by PCR on the template KT-1 using primers that introduce two stop codons in the extended packaging signal sequence. -In particular, the template pKT-1 contains the modification ATT at the normal ATG start site of *gag* (position 621-623 of SEQ ID NO: 1). Here the start site was further modified to the stop codon, TAA, and an additional stop codon TGA was added to replace the codon TTA at position 645-647 of SEQ ID NO: 1.

Briefly, two sets of PCR reactions were carried out on pKT1, each introducing one stop codon. The primers for the PCR were designed such that the two PCR products had overlapping regions and a splice-overlap extension PCR (SOE-PCR) was carried out with the two PCR products in order to combine the two introduced stop codons on one strand. The first set of oligonucleotides introducing the change from ATT to TAA were 5'-GGG-AGT-GGT-AAC-AGT-CTG-GCC-TTA-ATT-CTC-AG (SEQ ID NO: 27) and 5'-CGG-TCG-ACC-TCG-AGA-ATT-AAT-TC (SEQ ID NO: 28) and the second set of oligonucleotides introducing the change from TTA to TGA were 5'CTG-GGA-GAC-GTC-CCA-GGG-ACT-TC (SEQ ID NO: 29) and 5'GGC-CAG-ACT-GTT-ACC-ACT-CCC-TGA-AGT-17G-AC (SEQ ID NO: 30). The flanking primers of the final 708 base pair PCR product introduced the *Aat*II and the *Xho*I sites, at the 5' and 3', respectively.

The ends of the 708 base pair product were blunted and phosphorylated and the product introduced into the *Sma*I and *Eco*RV digested vector pBluescript SK-(Stratagene, San Diego, Calif.). The resulting plasmid was designated pBA-2, and is shown diagramatically in Figure 20.

### B. Removal of Retroviral Sequences Upstream and Downstream from the 3' LTR and Upstream and within the 5' LTR

Retroviral envelope sequence was removed upstream of the 3' LTR between the *Cla*I site and the TAG stop codon of the envelope coding sequence. The DNA sequence was modified by PCR such that the TAG stop codon was replaced by a *Cla*I site and the 97 nucleotides upstream from this new *Cla*I site to the original *Cla*I site were deleted, as well as the 212 base pairs of retroviral sequence downstream of the 3' LTR.

Briefly, the following two oligonucleotides were used for the PCR: 5'-CATCGATAAA ATAAAAGATT TTATTTAGTC (SEQ ID NO: 31) and 5'-CAAATGAAAG ACCCCCGCTG AC (SEQ ID NO: 32) and the template was pKT1. The PCR product was cloned into pPCRII (Invitrogen, San Diego, Calif.) using the TA cloning kit (Invitrogen, San Diego, Calif.) and called pBA-1.

Subsequently, pBA-2 (described in section A above) was digested with *Xba*I and *Aat*II which deleted a part of the multiple cloning site and into this linearized vector the 780 base pair fragment from *Nhe*I to *Aat*II from pKT1 was cloned, resulting in the plasmid pBA-3. This plasmid pBA-3 combined the shortened 5' LTR with the above described packaging region including the two introduced stop codons.

Subsequently, pBA-1 was digested with *Cla*I and *Apa*I resulting in a 640 base pair fragment that was cloned into the *Cla*I and *Apa*I digested pBA-3 resulting in the plasmid pBA-4. This plasmid combines the above described 5' LTR and the packaging signal with the 3' LTR.

Subsequently, pBA-4 was digested with *Apa*I and *Eco*RI, ends blunted and religated in order to remove extraneous 3' polylinker sites, resulting in plasmid pBA-5a.

Subsequently, pBA-5a was cut with NotI (blunted) and *EcoRI* and introduced into *Sma*I and *Eco*RI digested pUC 18 (GIBCO/BRL, Gaithersburg, MD) resulting in pBA-5b. This construct moved the retroviral vector from a pBluescript into an alternate pUC18 vector.

### EXAMPLE 10

### INSERTION OF GENES OF INTEREST INTO CROSS-LESS RETROVIRAL VECTOR BACKBONE PBA-5B

This example describes the insertion of two genes of interest, gp120(HIVenv/rev) and HSV-TK along with the neomycin gene into pBA-5b. Briefly, the sequence encoding gp120 was taken from construct pKT1 (Figure 6; see also Chada et al., J. Vir. 67:3409-3417, 1993). This vector is also referred to as N2IIIBenv.

### A. Introduction of HSV-TK and Neomycin into the Retroviral Vector pBA-5b

The HSV-TK gene was retrieved by digesting pBH-1 (PCT#UU 091-02805) with *Xho*I and *Eco*RI resulting in a 1.2 kb fragment. The neomycin gene driven by the SV40 promoter was retrieved by digesting pKT1 with *Eco*RI and BstBI resulting in a 1.3 kb fragment. Both fragments were cloned into a *Xho*I and *Cla*I digested pBA-5b resulting in the retroviral vector pMO-TK.

### B. Introduction of HIVenv/rev and neomycin into the retroviral vector pBA-Sb

The HIVenv/rev and neomycin genes were retrieved by digesting pKT1 with *Xho*I and BstBI resulting in a 4.4 kb fragment which was cloned into the *Xho*I and *Cla*I digested pBA-5b resulting in the retroviral vector pBA-6b.

### EXAMPLE 11

### FUNCTIONALITY TESTS FOR THE CROSS-LESS RETROVIRAL BACKBONES PBA-6B AND PMO-TK

Rapid tests are described in more detail below which ensure that the retroviral vectors coding for HIVenv/rev and neomycin (pBA-6b) and HSV-Tk and neomycin (pMO-TK) are comparable to the original retroviral vectors with regard to expression levels the genes of interest (HIVenv/rev and HSV-TK) and titers.

### A. Comparison of Transient and Stable Neo titer from pKT1 Versus pBA-6 in Transfected Non-clonal Vector Producing Pools

Retroviral vectors pKT1 or pBA-6 were transfected into DA packaging cells via CaPO₄-precipitation using the ProFection kit from Promega according to manufacturer's protocol (Promega, Madison, WI). The transient supernatant was collected 48 hours posttransfection, sterile-filtered (0.45 mm) and placed on HT 1080 target cells (HT 1080 cells, ATCC #CCL 121) in the presence of 8 mg polybrene/ml. Twenty-four hours after that treatment, the media is replaced with growth media containing 800 µg/ml G418. G418 resistant colonies are scored after one week The positive appearance of colonies indicates that all elements are functional and active in the original co-transfection.

For the stable neo titer, the transfected DA cells were cultured in selection media containing 800 µg/ml G418 for two weeks or until the untransfected control cells were dead. Titer of the supernatants from the confluent vector producing pools was determined as described above.

Results of transient and stable neo titers are presented in Table 2.

**Table 2:**

| Transient and stable neo titer of pKT-1 versus pBA-6b retroviral vectors in transfected and selected non-clonal DA vector producing pools. | |
|---|---|
| Retroviralvector coding tor HlVenv/rev plus neo | Transient neo titer in CFU/ml |
| pKT-1 | 5.0x10⁴ |
| pBA-6b (cross-less retroviral vector) | 2.5x10⁴ |
| | Stable neo titer in CFU/ml |
| pKT-1 | 5.6x10⁶ |
| pBA-6b (cross-less retroviral vector) | 6.7x10⁶ |

### B. Comparison of gp120 and rev expression levels of pKT1 versus pBA-6b in vector producing pools and target cells.

The supernatants from the above described selected non-clonal pools DA/KT1 and DA/BA-6b were used to transduced HT 1080 target cells as described above. G-418 resistant colonies were selected as described above and the pools were named HT 1080/kit1 and HT 1080/BA-6b.

The DA/KT1, DA/BA-6b vector producer pools as well as the HT 1080/KT1 and HT 1080/BA-6b pools were lysed and gp120 and rev protein levels were estimated by Western Blot analysis according to standard procedures.

Results are presented in Table 3.

**Table 3:**

| Comparison of pKTI and pBA-6b retroviral vector with regard to gp120 and rev expression levels in transduced and selected non-clonal DA vector producing pools and transduced and selected target cells. | | |
|---|---|---|
| G-418 selected pools analyzed | gp120 expression levels | Rev expression levels |
| DA | - | - |
| DA/KT1 | + | + |
| DA/BA-6b | ++ | + |
| HT-1080 | - | - |
| HT-1080/KTI | +++ | + |
| HT-1080/BA-6b | +++ | + |

### C. Comparison of Stable Neo Titer from pKT1 Versus pBA-6 in Transduced Non-Clonal Vector Producing Pools

The retroviral vectors pBH1 or pMO-TK were transduced into various packaging cell lines using transient transfection produced VSV-G pseudotyped vectors as described in PCT/US91/06852 entitled "Packaging Cells" and PCT/US91/05699 entitled "Viral Particles Having Altered Host Range." The following packaging cell lines were used: DA, HA, HP, HX, 2A, 2X, as described in PCT/US91/06852 and PCT/U S91 /05699.

For the stable neo titer, the transduced packaging cell line pools were cultured in selection media containing 800 µg/ml G418 for two weeks or until the untransfected control cells were dead. Titers of the supernatants from the confluent vector producing pools were determined as described above.

TK expression levels were determined by Western Blot analysis of lysates of the specified vector producing pools.

Results of stable neo titers as well as TK expression levels in the various vector producing pools are presented in Table 4.

**Table 4:**

| Comparison of pBH-1 and pMO-TK in various packaging cell lines with regard to neo titers and TK expression levels in the transduced and selected vector producing pools. | | |
|---|---|---|
| G-418 selected-pools analyzed | Stable neo titers (CFU/ml) | TK expression levels |
| DA/BH-I | 6.0x10⁵ | ++ |
| DA/MO-TK | 1.3x10⁶ | ++ |
| HA/BH-I | 3.7x10⁵ | +++ |
| HA/MO-TK | 1.6x10⁵ | +++ |
| HP/BH-I | < 1x10³ | ++ |
| HP/MO-TK | < 1x10³ | ++ |
| HX/BH-T | 3.5x10³ | ++ |
| HX/MO-TK | 1.0x10⁵ | ++ |
| 2A/BH-I | 1.3x10⁵ | + |
| 2A/MO-TK | 1.7x10⁵ | + |
| 2X/BH-I | 3.2x10⁵ | + |
| 2X/MO-TK | 5.2x10⁵ | + |

### EXAMPLE 12

### CONSTRUCTION OF ENV EXPRESSION CASSETTES

### A. Clowning of Long and Short Bovine Growth Hormone Termination-Polyadenylation Sequences

The Long Bovine Growth Hormone (BGH) termination-polyadenylation sequence (positions 2330-2551 of Genebank sequence # BOVGHGH) was PCR amplifies from the plasmid pCDNA3 (Invitrogen Corp, San Diego CA) using the forward primer 5'CCTATGAGCT CGCCTTCTAG TTGCCAGC (SEQ ID NO: 33) (positions 2330-2346 of Genebank sequence # BOVGHGH) containing a restriction site for *Sac I* restriction endonuclease (underlined) and the reverse primer 5' CCTATGAATT CGCGGCCGCC ATAGAGCCCA CCGCATCC (SEQ ID NO: 34) (positions 2551-2531 of Genebank sequence # BOVGHGH) containing restriction sites for *EcoR* I and Not I(underlined). The PCR fragment was digested with *Sac* I and *Eco*R I and inserted into *Sac* I/*Eco*R I digested pBGS131 vector (American Type Culture Collection #37443) to create pBGS-long BGH. Similarly, the short BGH termination-polyadenylation sequence (positions 2415-2463 of Genebank sequence # BOVGHGH) was PCR amplified using the forward primer 5'TATATATGAG CTCTAATAAA ATGAGGAAAT TGCATCGCAT TGTC (SEQ ID NO: 35) (positions 2415-2445 of Genebank sequence # BOVGHGH) containing a restriction site for *Sac I* restriction endonuclease (underlined) and the reverse primer 5'CCTATGAATT CGCGGCCGCA TAGAATGACA CCTACTCAGA CAATGCGA (SEQ ID NO: 36) (positions 2463-2436 of Genebank sequence # BOVGHGH) containing the restriction sites for *EcoR I* and *Not I* (underlined). A template was not required because the primer sequences overlap. The PCR fragment was digested with *Sac I* and *EcoR I* and inserted into *Sac I*/*EcoR I* digested pBGS 131 vector to create pGBS-short BGH.

### B. Creation of a New 3' End for Env

The entire MoMLV amphotropic envelope coding region was PCR amplified from the plasmid pCMVenvAmDra (described in PCT/US91/06852 example 2) using the forward primer GCTCGTTTAG TGAACCGTCA, G (SEQ ID NO: 37) (positions 606-631 of pCMVenvAmDra) and the reverse primer TATCCGAGCT CATGGCTCGT ACTCTATGG (SEQ ID NO: 38) (positions 3136-3118 of pCMVenvAmDra). The reverse primer contains the restriction site for Sac I restriction endonuclease (underlined) directly after the stop codon of amphotropic envelope (bold). The PCR fragment was digested with *Sac I* and *Bgl II* and inserted into *Sac I*/*Bgl II* digested pBGS-long BGH and pBGS-short BGH vector to create pBGSAmEnv-long BGH and pBGSAmENV-short BGH respectively. These constructs contain amphotropic envelope with no MoMLV sequence past the termination codon, followed by the BGH termination-polyadenylation sequence.

### C. Insertion of Env-BGH into an Expression Plasmid

The plasmid pCMVenvAmDra (described in PCT/US91/06852, Example 2) was digested with *BstB I* and *Not I* restriction endonucleases. This digest removes approximately 210 bases of the 3' coding region of amphotropic envelope, approximately 75 MoMLV 3'noncoding bases, and the SV40 termination-polyadenylation sequence. The small BstB I/Not I fragment of the plasmids pBGSAmEnv-long BGH and pBGSAmENV-short BGH was inserted into the *BstB I*/*Not I* digested pCMVenvAmDra expression plasmid to create pCMVenvAmDra/LBGH and pCMVenvAmDra/SBGH respectively. The small *BstB I*/*Not I* fragment of the plasmids pBGSAmEnv-long BGH and pBGSAmENV-short BGH was also inserted into *BstB IlNot I* digested plasmid pCMVenv-X (Example 4) to create the plasmids pCMVenvX-long BGH and plasmids pCMVenvX-short BGH.

### D. Construction of the envelope gene truncated in the 5' and 3' non-coding regions of pCI

The entire MoMLV amphotropic envelope coding region was PCR amplified from the plasmid pCMVenvAmDra (described in PCT/US91/06852, Example 2) using the forward primer 5' CACCTATGCT AGCCACCATG GCGCGTTCAA CGCTCTC (SEQ ID NO: 39) containing a restriction site for *NheI* restriction endonuclease (underlined) and the reverse primer 5' CACCTATGCG GCCGCTCATG GCTCGTACTC TATGGG (SEQ ID NO: 40) containing a restriction site for *NotI* restriction endonuclease (underlined). The PCR fragment was digested with *NotI* and *NheI* and inserted into a *NotI*/*NheI* digested pCI vector (Promega Corp, Madison WI) to create pCI/envam. The PCR fragment contains the entire coding region of the envelope gene including the *NheI* site followed by the Kozak sequence CACC upstream of the ATG start codon and the TCA stop codon followed by the *NotI* site.

### E. Construction of the envelope gene truncated in the 5' and 3' non-coding regions in pCMVb

Similarly to pCI/envam, the entire MoMLV amphotropic envelope coding region was PCR amplified from the plasmid pCMVenvAmDra (described in PCT/US91/06852, Example 2) using the forward primer 5' CACCTATGCG GCCGCCACCA TGGCGCGTTC AACGCTCTC (SEQ ID NO: 41) containing a restriction site for *NotI* restriction endonuclease (underlined) and the reverse primer 5' CACCTATGCG GCCGCTCATG GCTCGTACTC TATGGG (SEQ ID NO: 40) containing a restriction site for *NotI* restriction endonuclease (underlined). The PCR fragment was digested with *NotI* and inserted into the *NotI* digested pCMVb vector (Clontech Laboratories Inc., Palo Alto, CA) deleting the b-galactosidase gene from pCMVb to create pCMV-b/envarn. The PCR fragment contains the entire coding region of the envelope gene including the *NotI* site followed by the Kozak sequence CACC upstream of the ATG start codon and the TCA stop codon followed by the *NotI* site.

### F. Construction of the envelope gene truncated in the 5' and 3' non-coding regions in pCMVenvAmDra/LBGH/ EAG del.

The plasmid pCMVenvAmDra/LBGH/EAG del was constructed from the plasmid pCMVenvAmDra/LBGH (described in example 12-c) by deletion of 441 base pairs from the *agI* site at position 695 to the *EagI* site at position at 1136 just upstream of the env start codon. This was accomplished by digesting pCMVenvAmDra/LBGH with *agI* and gel purifying the resulting bands of 2,227 and 3,573 base pairs. These two fragments were then ligated together and screened for correct orientation, such that the env start site was positioned downstream of the CMV promoter. The resulting construct was named pCMVenvAmDra/LBGH/EAG del.

### EXAMPLE 13

### CONSTRUCTION OF VARIOUS GAG/POL EXPRESSION PLASMIDS WITH PARTIALLY OR COMPLETELY REDUCED SEQUENCE OVERLAP TO THE CROSS-LESS RETROVIRAL BACKBONE AND ENVELOPE

This example describes several modifications of the MoMLV gag/pol expression plasmid pSCV10 (PCT/US91/06852, WO 92/05266) resulting in decreased or eliminated sequence homology to the retroviral backbone and envelope expression constructs.

### A. Creation of New Codons for the 5'Gag

This example describes the *gag*/*pol* expression plasmid cassette that contains wobbled non-coding sequences upstream from the gag start site, thereby reducing recombination potential between the *gag*/*pol* expression element and the extended packaging signal of a retroviral vector construct, and inhibiting co-packaging of the *gag*/*pol* expression element along with the retrovector. In order to construct such an expression cassette a 406 bp DNA fragment with a *ClaI* site at the 5' end (underlined) and a *NarI* site at the 3' end (underlined) was synthesized by Operon (Operon Technologies Inc, Alameda CA). The sequence of the 406 bp DNA fragment was verified and is provided in Table 5. The synthesized DNA was transferred to a shuttle plasmid as a *ClaI-NarI* fragment to create the plasmid pWOB.

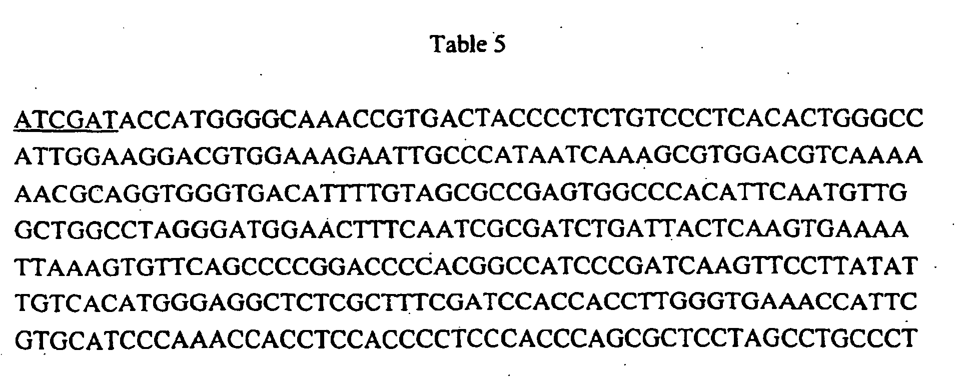

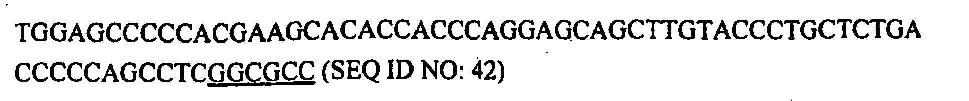

The *ClaI-NarI* fragment from pWOB was isolated by *ClaI-NarI* digest, and the 406 bp fragment cloned into the *ClaI-NarI* site of pSCV10 to create the plasmid pSCV10/wob (-NarI fragment) which resulted in the loss of the 481 bp *NarI-NarI* fragment just downstream of the wobbled *ClaI-NarI* fragment.

### B. Creation of a 5' truncated gag/pol construct without MoMLV sequence upstream of the start codon in pSCV10

This example describes the *gag*/*pol* expression plasmid cassette that eliminated the MoMLV sequence upstream of the ATG start codon in order to prevent sequence overlap to the retroviral backbone.

Briefly, a new *ClaI* site followed by an ideal Kozak translational start sequence was introduced upstream of the start codon of the *gaglpol* construct pSCV 10 by PCR using the forward primer 5' CGAATCGATA CCATGGGCCA GACTGTTACC AC (SEQ ID NO: 43) (the *ClaI* site is underlined) and the reverse primer 5' CATTCTGCAG AGCAGAAGGT AAC (SEQ ID NO: 44) containing a restriction site for *PstI* (underlined). The PCR fragment was digested with *ClaI* and *PstI* and the 131 bp fragment cloned into pSCV 10 replacing the existing *ClaI-PstI* DNA fragment to create the plasmid pSCV10/5'truncated g/p.

### C. Creation of a 5' truncated gaglpol construct without MoMLV sequence upstream of the start codon in pCI

This example describes the construction of the 5' truncated *gaglpol* construct analogous to that described under section B above in the pCI (Promega Corp, Madison, WI) vector backbone.

Briefly, fragments were prepared for a three-way ligation as follows: pCI was digested with *SmaI* and *NotI* and the 4 kb fragment was isolated. pSCV10 was digested with *XhoI* and *NotI* and the 4.7 kb fragment was isolated. pSCV10/5' truncated g/p as described in section B was digested with *ClaI,* filled in with Klenow to blunt, then digested with *XhoI* and the 0.95 kb fragment was isolated. These three fragments were then ligated together to give the final plasmid pCI/5'truncated g/p.

### D. Creation of a 5' truncated and wobbled gag/pol construct in pCI

This example describes the construction of the 5' truncated and wobbled *gaglpol* construct in the pCI vector where the 5' truncation as described in section C and the wobbled gag sequences as described in section A were combined.

Briefly, the wobbled *gaglpol* sequence (0.47 kb) was retrieved from plasmid pSCV10/wob (-NarI fragment) as described in section A above by digestion with *ClaI* and *XhoI.* This fragment was cloned into the *ClaI-XhoI* digested pBluescript SK- (Stratagene, San Diego, CA) to create pBluescript/wob (- NarI fragment). This plasmid was digested with *EcoRI* and *NarI* to retrieve the wobbled gag sequence and the *EcoRI-NarI* fragment cloned into *the EcoRI-NarI* digested pCI/5' truncated g/p described in section C above in order to create pCI/5'truncated wob g/p.

### E. Creation of a 5' and 3' truncated gag/pol construct in pCI and pSCV 10

This example describes the construction of the 5' and 3' truncated *gaglpol* construct in the pCI vector where the 5' truncation as described in section C is combined with the following 3'truncation upstream of the stop codon eliminating the DNA sequence coding for the last 28 amino acids of the pol protein.

Briefly, the plasmid pCI/5'truncated g/p described in section C was linearized with the restriction enzyme *SmaI* which is located 84 bases upstream of the *gaglpol* termination codon in the open reading frame of *gaglpol.* The linearized plasmid was ligated to the oligonucleotide 5' TAAGCGGCCG CTTA (SEQ ID NO: 45). This oligonucleotide is self-complementary and forms a palindromic duplex containing a TAA termination codon and a *NotI* restriction endonclease site. After ligation of 100ng vector and 5µM oligo in the presence of T4 DNA ligase, the reaction was purified by GeneClean and digested with *SmaI* to recut any vector that did not contain an insert. The reaction was used to transform XL1 Blue *E. coli* (Stratagene, San Diego, CA) and plasmid DNA from a correct clone was then digested with *NotI. NotI* cuts in the inserted oligonucleotide as well as just upstream of the SV40 termination/polyadenylation site of the pCI vector. The digested plasmids were purified by Geneclean and religated to recircularize. Bacteria were transformed and clones were identified which deleted the sequences between the *NotI* site introduced by the oligonucleotide and the *NotI* site in the pCI vector. These sequences include sequences encoding the last 28 amino acids of gag/pol as well as MoMLV sequences and vector sequences carried over from pSCV 10. The resulting *gaglpol* construct was named pCI-GPM. The identically shortened *gaglpol* region was cloned by standard techniques into a pSCV10 background expression cassette. This expression plasmid was named pSCV10/5',3'tr.

### F. Creation of a 5' and 3' truncated and wobbled gaglpol construct in pCI

This example describes the construction of the 5' and 3' truncated and wobbled *gaglpol* construct in the pCI vector combining the 5' truncation and wobbled *gaglpol* sequence from section D above with the 3'truncation described in section E above.

Briefly, pCI/5'truncated wob g/p was linearized with *SmaI* and all further steps leading to the 3'truncation of *gaglpol* were carried out as described in section E above, leading to the 5' and 3' truncated and wobbled *gaglpol* construct in pCI named pCI-WGPM.

### EXAMPLE 14

### CONSTRUCTION AND TESTING TITER POTENTIAL OF PCLS WITH VARIOUS COMBINATIONS OF GAG/POL AND ENV EXPRESSION CASSETTES RESULTING IN PCLs WITH VARIOUS DEGREES OF DNA SEQUENCE OVERLAP BETWEEN THE RETROVIRAL COMPONENTS: GAG/POL, ENV AND RETROVIRAL VECTOR

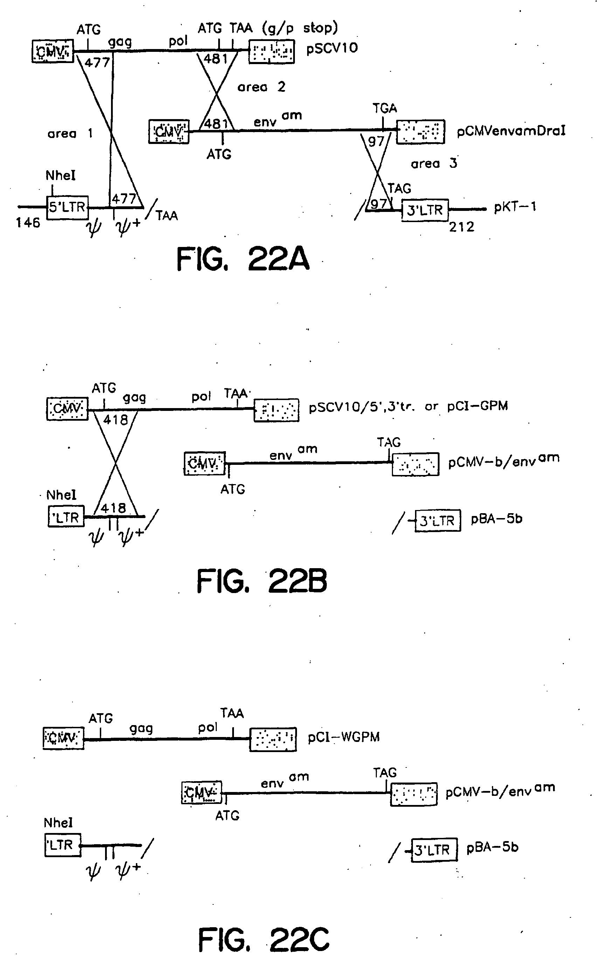

This example describes the production of PCLs based on various combinations of the *gaglpol* and *env* expression cassettes described above and in Examples 12 and 13. The three unmodified retroviral components *gaglpol, env* and retroviral vector (PCT Application No. WO 92/05266) result in three areas of sequence overlap (area 1-3) in a VCL as shown in Figure 22A. PCLs/VCLs with reduced sequence overlap can be produced with elimination of any combination of these three sequence overlap areas for example, a PCL/VCL may eliminate sequence overlap of area 1, area 2 or area 3 only, a combination of any two or all three areas. Production and potential titer analysis of PCLs with all three overlaps eliminated (Figure 22 C) as well as PCLs with the first area of overlap reduced and area 2 and 3 eliminated (Figure 22 B) are described below. A critical issue in the production of PLCs with reduced sequence overlap is the maintenance of high titer potential. Therefore, the titer potential of the PCLs with reduced sequence overlap were analyzed and compared extensively to existing PCLs with unmodified PCL components such as the DA and HA PCLs described in PCT Application No. WO 92/05266.

### A. Production of PCLs with one area of sequence overlap between PCL components

This example describes the production of PCLs with the *gaglpol* expression plasmid cassette pSCV10/5',3'tr. or pCI-GPM described in Example 13 E and the *env* expression plasmid pCMV-b/envam described in Example 12 E. PCLs with these *gaglpol* and *env* expression plasmids in conjunction with a retroviral vector derived from pBA-5b (Example 9) result in VCLs where sequence overlap areas 2 and 3 are eliminated and area 1 is reduced (Figure 22 B). The cell lines HT 1080 (ATCC #CCL 121) and D17 (ATCC #CCL 183) were used as parent cell lines to establish the PCLs.

Briefly, either *gaglpol* plasmid pSCV10/5',3'tr. or pCI-GMP was co-transfected together with a phleomycin^{r} expressing marker plasmid into HT 1080 and D17 cells, respectively, via CaPO₄-precipitation using the ProFection kit from Promega according to manufacturer's protocol (Promega, Madison, WI). The transfected cells were selected with media containing zeocin^{™} (Invitrogen, Carlsbad, CA) at a concentration of 150 mg/ml for HT 1080 cells and 170 mg/ml for D17 cells until untransfected control cells were dead. HT 1080 and D17 gag/pol pools were dilution cloned into 96-well microtiter plates according to standard protocols where clonality was ensured by seeding cell densities that yield a maximum of 30% of wells with cell growth. HT 1080 and D17 derived gag/pol intermediate clones were isolated and analyzed for intracellular p30 expression levels in a standard Western blot using primary p30-specific goat antibodies and secondary, HRP-labeled rabbit anti-goat antibodies. These gag/pol clones were compared to HTSCV21 and D17 4-15 which are the HT 1080 and D17 gag/pol intermediate clones for the PCLs HA and DA, respectively (PCT #WO 92/05266). DA and HA have all three areas of sequence overlap (Figure 22 A). Results of the p30 Western are shown below in Table 6.

**Table 6:**

| HT 1080 and D 17 derived clones screened for intracellular p30 levels after introduction of *gaglpol* expression cassettes pSCV 10/5',3'tr. or pCI-GPM | | | |
|---|---|---|---|
| **Gag/pol intermediates** | **#clones screened for p30** | **#clones positive for p30 - (%)** | **p30 expression levels** |
| D17gag/pol (pCI-GPM) | 80 | 32 (40%) | 3-4 clones have p30 levels comparable to D17 4-15 |
| HT 1080gag/pol (pSCV10/5',3'tr.) | 100 | 24 (24%) | 15 clones have p30 levels comparable or higher than HTSCV21 |

The 18 HT 1080 and 12 D17 gag/pol intermediates with the highest p30 expression levels were analyzed for titer potential.

Briefly, a retroviral ecotropic *env* expressing vector and a retroviral vector coding for β-gal and neo^{r} were co-introduced into the gag/pol intermediate clones, transient and stable supernatants harvested and β-gal titers determined on 3T3 target cells using either a standard blue X-gal staining procedure or a "Galactolight assay." Briefly, this assay allows rapid determination of β-gal vector titers by chemiluminescent detection of transfer of β-gal expression to HT 1080 target cells. (Tripix, New Bedford, MA). Expression was compared to a standard curve for transfer of expression versus titer generated by serial dilutions of a known titer reference β-gal vector.

The titer results for the HT 1080 gag/pol intermediates are shown below in Table 7.

**Table 7:**

| Transient β-gal titers from transduced pools of HT 1080 gag/pol intermediates | | |
|---|---|---|
| **Crone#** | **Transient β-gal titer (CFU/ml)** | **x-fold titer decrease (HTSCV21:HT 1080 gag/pol intermediate)** |
| | | |
| 1 | 178 | 11 |
| 2 | 750 | 3 |
| 6 | 345 | 6 |
| 12 | 728 | 3 |
| 14 | 545 | 4 |
| 18 | 113 | 18 |
| 38 | 263 | 8 |
| 41 | 1100 | 2 |
| 42 | (3) | (660) |
| 47 | 83 | 24 |
| 53 | 573 | 3 |
| 57 | 95 | 21 |
| 59 | 850 | 2 |
| 62 | 518 | 4 |
| 67 | 440 | 5 |
| 69 | 0 | - |
| 70 | 375 | 5 |
| 90 | 1300 | 2 |
| | | |
| HTSCV21 1975 | | |

11 out of 18 clones gave a titer potential that was 2-6 fold lower in comparison to HTSCV21. The titer results for the D17 gag/pol intermediate clones are shown below in Table 8.

**Table 8:**

| Transient β-gal titers from transduced pools of D17gag/pol intermediates and stable β-gal titers from transduced and G-418 selected D17gag/pol intermediates | | | | |
|---|---|---|---|---|
| **Clone#** | **Transient β-gal titer (CFU/ml)** | **x-fold titer decrease (D17 4-15: D17 gag/pol intermediates)** | **Stable β-gal titer (light units)** | **x-fold titer decrease** |
| 2 | 165 | 473 | 80.1 | 14 |
| 10 | 95 | 821 | 44.3 | 25 |
| 42 | 270 | 289 | 27.8 | 40 |
| 55 | 990 | 79 | 246.7 | 5 |
| 60 | 220 | 354 | 67.8 | 16 |
| 65 | 495 | 158 | 280.5 | 4 |
| 71 | 605 | 129 | 77.0 | 14 |
| 72 | 0 | - | 95.9 | 12 |
| 74 | 1.7E4 | 5 | 1497.3 | no decrease |
| 75 | 2100 | 37 | 1180.3 | no decrease |
| 84 | 3400 | 23 | 300.3 | 4 |
| 92 | 1600 | 49 | 2013.7 | no decrease |
| | | | | |
| D17 4-15 | 7.8E4 | 1112.3 | | |

The transient titers show a strong decrease in titer potential when compared to D 17 4-15, but for the stable titers, six out of the 12 gag/pol intermediates show 0-6 fold decrease in comparison to D17 4-15.

A total of 6 D17 and 4 HT 1080 gag/pol intermediates with the highest titer potential were co-transfected with the *env* expression plasmid pCMV-b/envam described in Example 12 and a methotrexate^{r} expressing marker plasmid into the HT 1080 and D17gag/pol intermediate clones via CaPO₄-precipitation using the ProFection kit from Promega according to manufacturer's protocol (Promega, Madison, WI). The transfected cells were selected with media containing methotrexate at a concentration of 2x10⁻⁷ M until untransfected control cells were dead. HT 1080 and D 17 derived PCL pools were dilution cloned into 96-well microtiter plates according to standard protocols where clonality was ensured by seeding cell densities that yield a maximum of 30% of wells with cell growth. Several hundred HT 1080 and D 17 derived PCL clones named HAII and DAII, respectively, were isolated and analyzed for titer potential.

Briefly, five rounds of titer potential analysis were carried out using various retroviral vectors. The DA or HA PCL controls (PCT #WO 92/05266) were included as a reference for high titer potential PCLs. In the first round, the PCL clones were transduced in 24-well plates with the β-gal coding retroviral vector DX/ND7 (WO 95/16852) at an moi of 5-10 in the presence of 8 µg/ml polybrene, transient supernatants harvested, filtered (0.45 µm), HT 1080 target cells transduced and transient β-gal titer determined using a standard Galactolight procedure following manufacturer's instructions (Tropix, Bedford, MA). In the second round, the same transduction assay as described for the first round was repeated with the top clones from round one using standardized PCL cell numbers. In the following titer potential analysis rounds, the top clones from round two were used to transduce with several retroviral vectors, supernatant from transient and stable pools were harvested, filtered, HT 1080 target cells transduced, and transient and stable titers determined.

Data on the titer potential analysis of the second round of screening is shown below in Table 9 on a small selection of representative DAII and HAII PCL clones.

**Table 9:**

| Transient β-gal titer on VCL pools from transduced HAII and DAII PCLs determined by Galactolight readout. | | |
|---|---|---|
| **Clone#** | **Transient β-gal titer (Galactolight, light units)** | **x-fold decrease in titer potential (DA:DAII, HA:HAII)** |
| **D-17 based PCLs called DAII** | | |
| DAII (based on pCI-GPM#74 intermediate): | | |
| 20 | 3 | - |
| 30 | 69 | 14 |
| 47 | 19 | 51 |
| 49 | 1 | - |
| 55 | 145 | 7 |
| 60 | 1 | - |
| 67 | 8 | - |
| 70 | 45 | 22 |
| DA | 978 | |
| | | |
| DAII (based on pCI-GPM#75 intermediate): | | |
| 7 | 47 | 19 |
| 32 | 202 | 5 |
| 40 | 27 | 33 |
| 60 | 15 | 61 |
| 70 | 7 | - |
| 72 | 1 | - |
| DA | 901 | |
| | | |
| **HT-1080 based PCLs called HAII** | | |
| HAII (based on pSCV 10/5'3'tr.intermediate #12): | | |
| 6 | 147 | 10 |
| 11 | 8 | - |
| 12 | 56 | 27 |
| 18 | 45 | 34 |
| 44 | 113 | 13 |
| 51 | 2 | - |
| 54 | 2 | - |
| 56 | 83 | 18 |
| 57 | 115 | 13 |
| 65 | 133 | 11 |
| 66 | 104 | 15 |
| 78 | 195 | 8 |
| 86 | 125 | 12 |
| 87 | 77 | 20 |
| 88 | 259 | 6 |
| 90 | 196 | 8 |
| 91 | 91 | 17 |
| HA | 1508 | |
| | | |
| HAII(based on pSCV10/5'3'tr.#41): | | |
| 4 | 48 | 31 |
| 9 | 84 | 18 |
| 15 | 157 | 10 |
| 18 | 174 | 9 |
| 37 | 111 | 14 |
| 55 | 357 | 4 |
| 58 | 140 | 11 |
| 75 | 164 | 9 |
| 92 | 57 | 28 |
| HA | 1570 | |

The top DAII and HAII PCL clones gave a 4-5 fold reduced titer potential when measured as a transient β-gal pool. A large percentage of these DAII and HAII PCL clones gave a 10-15 fold decrease in titer potential.

The top DAII and HAII clones were further tested for their titer potential using various retroviral vectors. Table 10 below shows a summary of titer potentials on VCL pools of the top HAII clones, with HAII#41#55 as the overall best PCL.

**Table 10:**

| PCL clone# | transient factor VIII | stable PLAP (BAAP) | stable neo (BAAP) | stable neo (KT-1) | transient hGH | transient factor VIII |
|---|---|---|---|---|---|---|
| | | | | | | |
| HAII: | | | | | | |
| 12#78 | 1.1E5 | 5.0E4 | 1.6E4 | 5.3E5 | 36,000 | 7.7E4 |
| 12#86 | *8.8E4* | 6.6E4 | *1.1E4* | *8.4E5* | *29,200* | 7. 1 E4 |
| 12#88 | *1.1E4* | 3.2E4 | 8.6E4 | *8.2E5* | *34,500* | 1.1E5 |
| 12#90 | 9.4E4 | 1.0E5 | 6.4E4 | 9.4E5 | 37,700 | 1.1E5 |
| 41#18 | 7.0E4 | 6.2E4 | 2.0E4 | 1.3E5 | 34,400 | 1.8E5 |
| 41#55 | *9.9E4* | 6.3E5 | *1.7E5* | *1.1E6* | *44,000* | 1.3E5 |
| 41#58 | 8.9E4 | 4.8E4 | 8.8E3 | 5.1E5 | 38,700 | 4.3E4 |
| 41#75 | 1.1E5 | 1.4E5 | 3.2E4 | 8.7E5 | 34,300 | 1.5E5 |
| | | | | | | |
| HA | 5.3E5 | 4. 1 E4 | 2.1E4 | 5.0E5 | 38,300 | 3.7E5 |
| | *1.3E5* | | *1.6E4* | *1.1E7* | *39,300* | |
| | | | | | | |
| DA | 3.9E4 | 1.0E6 | 4.0E5 | 5.9E5 | 45,700 | 1.9E5 |
| | *7.3E3* | | *8.4E6* | *4.8E6* | *52,500* | |

| | | | | | | |
|---|---|---|---|---|---|---|
| Values in italics must be compared to the control PCL values (DA, HA) in italics | | | | | | |

Differences in titer potential were observed, depending not only on which PCL clone was used but also which gene of interest was expressed in the retroviral vector.

Comparison of the 8 top HAII clones with titers on VCL pools from various rounds of titer potential assays. The B-domain deleted factor VIII, human placental alkaline phosphatase plus neo^{r} (BAAP), human growth hormone (hGH) and HIVenv/rev plus neo' (KT-1) expressing retroviral vectors were used to transduce the HAII PCLs. Transient and stable supernatants were tested on HT 1080 target cells. The readout for hGH is in units and the other titers are in CFU/ml.

### B. Production of PCLs without any sequence overlap between PCL components

This example describes the production of PCLs with the *gaglpol* expression plasmid cassette pCI-WGPM described in Example 13 F and the *env* expression plasmid pCMV-b/envam described in Example 12 E. PCLs with these *gaglpol* and *env* expression plasmids in conjunction with the retroviral vector derived from pBA-5b (Example 9) result in producer cell lines where sequence overlap between all three areas of homology is completely eliminated (Figure 22 C). The cell lines HT 1080 (ATCC #CCL 121) and D17 (ATCC #CCL 183) were used as parent cell lines to establish the PCLs.

Briefly, *gaglpol* plasmid pCI-WGPM was co-transfected together with a phleomycin^{r} expressing marker plasmid into HT 1080 and D17 cells, selected and dilution cloned as described above. HT 1080 and D17 derived clones were isolated and analyzed for intracellular p30 expression levels as described above. Results of the p30 Western are shown below in Table 11.

**Table 11:**

| HT 1080 and D 17 derived clones screened for intracellular p30 levels after introduction of *gaglpol* expression cassette pCI-WGPM | | | |
|---|---|---|---|
| **Gag/pol intermediates** | **#clones screened for p30** | **#clones positive for p30 - (%)** | **p30 expression levels** |
| D-17g/p (pCI-WGPM) | 82 | 36 (44%) | 3-4 clones have p30 levels comparable to D17 4-15 |
| HT-1080g/p (pCI-WGPM) | 96 | 26 (27%) | 3-4 clones have p30 levels that are comparable to HTSCV21 |

The 12 HT 1080 and 22 D17 gag/pol intermediates with the highest p30 expression levels were analyzed for titer potential as described above. The titer results for the HT 1080 gag/pol intermediates are shown below in Table 12.

**Table 12:**

| Transient β-gal titers from transduced pools of HT 1080 gag/pol intermediates (pCI-WGPM) | | |
|---|---|---|
| **Clone#** | **Transient β-gal titer (CFU/ml)** | **x-fold titer decrease (HTSCV21:HT 1080 gag/pol intermediate)** |
| | | |
| 10 | 217 | >9 |
| 12 | 28 | >71 |
| 23 | 670 | >3 |
| 29 | 565 | >4 |
| 34 | 950 | >2 |
| 35 | 398 | >5 |
| 45 | 280 | >7 |
| 52 | 670 | >3 |
| 53 | 600 | >3 |
| 71 | 590 | >3 |
| 86 | 480 | >4 |
| 87 | 55 | >36 |
| | | |
| HTSCV21 | >2000 | |

The Galactolight readout for HTSGV10 was out of the range with >2000, therefore the above shown decrease in titer potential is likely to be higher. The titer results for the D17 gag/pol intermediates are shown below in Table 13.

**Table 13:**

| Transient β-gal titers from transduced pools of D17gag/pol (D17 g/p) intermediates and stable β-gal titers from transduced and G-418 selected pools of D17gag/pol intermediates | | | | | | |
|---|---|---|---|---|---|---|
| Clone# | Transient β-gal titer (CFU/ml) | x-fold decrease (D17 4-15:D17g/ p inter.) | Stable β-gal titer (CFU/ml) | x-fold decrease (D17 4-15: D17g/p inter.) | Transient β-gal (CFU/ml) | x-fold decrease (D17 4-15:D17g/p inter.) |
| 1 | 0 | - | | | | |
| 3 | 40 | >100 | | | | |
| 6 | 20 | >200 | | | | |
| 14 | 10 | >400 | | | | |
| 21 | 10 | >400 | | | | |
| 22 | 1380 | >3 | 800 | >5 | 2.1E4 | 9 |
| 27 | 30 | >133 | | | | |
| 41 | 100 | >40 | | | | |
| 47 | 30 | >133 | 730 | >6 | 90 | 2111 |
| 48 | 30 | >133 | | | | |
| 49 | 500 | >8 | 680 | >6 | 9.4E3 | 20 |
| 50 | 140 | >29 | | | | |
| 51 | 10 | >400 | | | | |
| 56 | 600 | >7 | 320 | >13 | 1.8E3 | 105 |
| 57 | 230 | >17 | | | 1.3E3 | 146 |
| 60 | 380 | >11 | 580 | >7 | 1.0E4 | 190 |
| 65 | 0 | - | | | | |
| 66 | 470 | >9 | 330 | >12 | 1.1E3 | 172 |
| 70 | 30 | >133 | | | | |
| 73 | 320 | >13 | 1.05E4 | 0 | | |
| 76 | 40 | >100 | | | | |
| 79 | 20 | >200 | | | | |
| D1.7 4-15 | >4000* | | >4000* | | 1.9E5 | |

| | | | | | | |
|---|---|---|---|---|---|---|
| =out of range | | | | | | |

The titer potential measured within the range indicates decreases in titer potential of 10-200 fold in most clones.

A total of 6 D17 and 4 HT 1080 gag/pol intermediates with the highest titer potential were co-transfected with the env expression plasmid pCMV-b/envam, pools selected and dilution cloned as described above. Several hundred HT 1080 and D17 derived PCL clones named HAIIwob and DAIIwob, respectively, were isolated and analyzed for titer potential.

Briefly, several rounds of titer potential analysis were carried out using various retroviral vectors. The DA or HA PCL (PCT #WO 92/05266) controls were included as a reference for high titer potential PCLs. In the first round, the PCL clones were transduced in 24-well plates with the β-gal coding retroviral vector DX/ND7 (WO 95/16852) at an moi of 5-10 in the presence of 8 µg/ml polybrene, transient supernatants harvested, filtered (0.45 µm), HT 1080 target cells transduced and transient β-gal titer determined using a standard Galactolight transfer of expression procedure described previously. In the second round, the same transduction assay as described for the first round was repeated with the top clones from round one using standardized PCL cell numbers. In the third round, the top clones from round two were used to transduce with several retroviral vectors, supernatant from transient and stable pools harvested, filtered, HT 1080 target cells transduced and titers determined.

Data on the titer potential analysis of the first and second round of screening is shown below in Table 14 on a small selection of representative DAII and HAII PCL clones.

**Table 14:**

| Transient β-gal titer on VCL pools from transduced HAII and DAII PCLs determined by Galactolight readout. | | |
|---|---|---|
| **Clone#** | **Transient β-gal titer (Galactolight, light units)** | **x-fold decrease in titer potential (DA:DAIIwob or HA:HAIIwob)** |
| | | |
| **D-17 based PCLs called DAIIwob** | | |
| DAIIwob (pCI-WGPM#60): | | |
| 7 | 21 | 27 |
| 11 | 6 | 93 |
| 21 | 2 | 279 |
| | | |
| 30 | 14 | 40 |
| 33 | 51 | 11 |
| 41 | 30 | 19 |
| DA | 558 | |
| | | |
| DAIIwob (pCI-WGPM#22): | | |
| 5 | 148 | 0 |
| 8 | 28 | 5 |
| 28 | 14 | 11 |
| 56 | 15 | 10 |
| 78 | 39 | 4 |
| 97 | 10 | 15 |
| DA | 153 | |
| | | |
| **HT-1080 based PCLs called HAIIwob** | | |
| HAIIwob (pCI-WGPM)#34: | | |
| 4 | 8 | 128 |
| 7 | 9 | 114 |
| 35 | 7 | 147 |
| 43 | 4 | 257 |
| 53 | 5 | 205 |
| 65 | 9 | 114 |
| 66 | 10 | 103 |
| 77 . | 19 | 54 |
| 79 | 6 | 171 |
| 80 | 4 | 257 |
| 95 . | 4 | 257 |
| 105 | | 500 |
| 115 | 9 | 114 |
| 118 | 6 | 171 |
| | | |
| HA | 1026 | |

The best DAIIwob PCL clone shows a 4-fold reduction in titer but most clones show >10-fold reduction. The best HAIIwob PCL clone shows a 50-fold reduced titer potential and most HAIIwob clones have >100-fold reduced titer potential. In general, the DAII wob and HAIIwob PCL clones gave in average about a 5-50 fold lower titer potential when compared to DAII and HAII PCLs.

## Claims

1. A producer cell line, comprising a *gag*/*pol* expression cassette, an *env* expression cassette and a retroviral vector construct, wherein there is no sequence overlap between the *gag*/*pol* expression cassette and the *env* expression cassette, and no sequence overlap between the *env* expression cassette and the retroviral vector construct, with the proviso that said retroviral vector construct overlaps with at least 4 nucleotides of a 5' terminal end of a *gag*/*pol* gene encoded within said *gag*/*pol* expression cassette.

2. The producer cell line according to claim 1, wherein said retroviral vector construct is a retroviral vector construct comprising a 5' LTR, a tRNA binding site, a packaging signal, an origin of second strand DNA synthesis and a 3' LTR, wherein said vector construct contains *gag*/*pol* coding sequences which have been modified to contain one, two or more stop codons.

3. The producer cell line according to claim 2 wherein one of the stop codons eliminates the start site.

4. The producer cell line according to claim 2 or 3 wherein one of the stop codons has two or three base pair substitutions.

5. The producer cell line according to claim 2 wherein said retroviral vector construct lacks an extended packaging signal.

6. The producer cell line according to claim 2 wherein said retroviral vector construct lacks a retroviral nucleic acid sequence upstream of said 5' LTR.

7. The producer cell line according to claim 6 wherein said retroviral vector construct lacks an *env* coding sequence upstream of said 5' LTR.

8. The producer cell line according to claim 2 wherein said retroviral vector construct lacks an *env* coding and/or untranslated *env* sequence upstream of said 3' LTR.

9. The producer cell line according to claim 2 wherein said retroviral vector construct lacks a retroviral packaging signal sequence downstream of said 3' LTR.

10. The producer cell line according to claim 2 wherein said retroviral vector construct is constructed from a retrovirus selected from the group consisting of amphotropic, ecotropic, xenotropic or polytropic viruses.

11. The producer cell line according to claim 2 wherein said retroviral vector construct is constructed from a Murine Leukemia Virus.

12. The producer cell line according to claim 2 wherein said retroviral vector construct is constructed from a lentivirus.

13. The producer cell line according to claim 2 wherein said retroviral vector construct further comprises a heterologous sequence.

14. The producer cell line according to claim 13 wherein said heterologous sequence is a gene encoding a cytotoxic protein.

15. The producer cell line according to claim 14 wherein said cytotoxic protein is selected from the group consisting of ricin, abrin, diptheria toxin, cholera toxin, gelonin, pokeweed, antiviral protein, tritin, Shigella toxin and Pseudomonas exotoxin A.

16. The producer cell line according to claim 13 wherein said heterologous sequence is an antisense sequence.

17. The producer cell line according to claim 13 wherein said heterologous sequence encodes an immune accessory molecule.

18. The producer cell line according to claim 17 wherein said immune accessory molecule is selected from the group consisting of IL-1, IL-2, IL-3, IL-4, IL-5, IL-6, IL-7, IL-8, IL-9 IL-10, 1L-11, IL-12, IL-13 and IL-15

19. The producer cell line according to claim 17 wherein said immune accessory molecule is selected from the group consisting of ICAM-1, ICAM-2, b-microglobin, LFA3, HLA class I and HLA class II molecules.

20. The producer cell line according to claim 13 wherein said heterologous sequence encodes a gene product that activates a compound with little or no cytotoxixity into a toxic product.

21. The producer cell line according to claim 20 wherein said gene product is selected from group consisting of HSVTK, VZVTK and cytosine deaminase.

22. The producer cell line according to claim 13 wherein said heterologous sequence is a ribozyme.

23. The producer cell line according to claim 13 wherein said heterologous sequence is a replacement gene.

24. The producer cell line according to claim 23 wherein said replacement gene encodes a protein selected from the group consisting of Factor VIII, ADA, HPRT, CF and the LDL Receptor.

25. The producer cell line according to claim 13 wherein said heterologous sequence encodes an immunogenic portion of a virus selected from the group consisting of HBV, HCV, HPV, EBV, FeLV, FIV and HIV.

26. The producer cell line according to claim 1 wherein said *gag*/*pol* expression cassette comprises a promoter operably linked to a *gag*/*pol* gene, and a polyadenylation sequence, wherein a 3' terminal end of said *gag*/*pol* gene has been delcted-without affecting the biological activity of

27. The producer cell line according to claim 26 wherein said 3' terminal end has been deleted upstream of nucleotide 5751 of Sequence ID No.1.

28. The producer cell line according to claim 26 wherein said promoter is a heterologous promoter.

29. The producer cell line according to claim 26 wherein said promoter is selected from the group consisting of CMV IE, the HSVTK promoter, RSV promoter, Adenovirus major-latter promoter and the SV40 promoter.

30. The producer cell line according to claim 26 wherein said polyadenylation sequence is a heterologous polyadenylation sequence.

31. The producer cell line according to claim 30 wherein said heterologous polydenylation sequence is selected from the group consisting of the SV40 late poly A signal, the SV40 early poly A signal and a bovine growth hormone poly A signal.

32. The producer cell line according to claim 1 wherein said *env* expression cassette comprises a promoter operably linked to an *env* gene, and a polyadenylation sequence, wherein no more than 6 consecutive retroviral nucleotides are included upstream of said *env* gene.

33. The producer cell line according to claim 1 wherein said *env* expression cassette comprises a promoter operably linked to an *env* gene, and a polyadenylation sequence, wherein said *env* expression cassette does not contain a consecutive sequence of more than 8 nucleotides which are found in a *gag*/*pol* gene.

34. The producer cell line according to claim 1 wherein said *env* expression cassette comprises a promoter operably linked to an *env* gene, and a polyadenylation sequence, wherein a 3' terminal end of said *env* gene has been deleted without effecting the biological activity of *env.*

35. The produce cell line according to claim 34 wherein a 3' terminal end of said *env* gene has been deleted such that a complete R peptide is not produced by said expression cassette.

36. The producer cell line according to claim 34 wherein said *env* gene is derived form a type C retrovirus, and wherein the 3' terminal end has been deleted such that said *env* gene includes less that 18 nucleic acids which encode said R peptide.

37. The producer cell line according to claim 34 wherein said promoter is a heterologus promoter.

38. The producer cell line according to claim 37 wherein said promoter is selected from the group consisting of CMV IE, the HSVTK, promoter, RSV promoter, Adenovirus major-latter promoter and the SV40 promoter.

39. The producer cell line according to claim 34 wherein said polyadenylation sequence is a heterologous polyadenylation sequence.

40. The producer cell line according to claim 39 wherein said heterologous polyadmylation is selected-from the group consisting of the SV40 late poly A signal, the SV40 early poly A signal and a bovine growth hormone polyadenylation sequence.

## Patentansprüche

1. Produzentenzellinie, welche eine *gag*/*pol*-Expressionskassette, eine *env*-Expressionskassette und ein retrovirales Vektorkonstrukt umfaßt, wobei es keine Sequenzüberlappung zwischen der *gag*/*pol*-Expressionskassette und der *env*-Expressionskassette und keine Sequenzüberlappung zwischen der *env*-Expressionskassette und dem retroviralen Vektorkonstrukt gibt, mit der Maßgabe, daß das retrovirale Vektorkonstrukt mit wenigstens 4 Nukleotiden eines 5'-terminalen Endes eines *gag*/*pol*-Gens, welches innerhalb der *gag*/*pol*-Expressionskassette codiert ist, überlappt.

2. Produzentenzellinie nach Anspruch 1, wobei das retrovirale Vektorkonstrukt ein retrovirales Vektorkonstrukt ist, welches eine 5'-LTR, eine tRNA-Bindungsstelle, ein Verpackungssignal, einen Ursprung der Zweitstrang-DNA-Synthese und eine 3'-LTR umfaßt, wobei das Vektorkonstrukt *gag*/*pol* codierende Sequenzen umfaßt, die so modifiziert wurden, daß sie ein, zwei oder mehr Stopcodon(s) enthalten.

3. Produzentenzellinie nach Anspruch 2, wobei eines der Stopcodons die Startstelle eliminiert.

4. Produzentenzellinie nach Anspruch 2 oder 3, wobei eines der Stopcodons zwei oder drei Basenpaarsubstitutionen aufweist.

5. Produzentenzellinie nach Anspruch 2, wobei dem retroviralen Vektorkonstrukt ein verlängertes Verpackungssignal fehlt.

6. Produzentenzellinie nach Anspruch 2, wobei dem retroviralen Vektorkonstrukt eine retrovirale Nukleinsäuresequenz aufstromig von der 5'-LTR fehlt.

7. Produzentenzellinie nach Anspruch 6, wobei dem retroviralen Vektorkonstrukt eine *env* codierende Sequenz aufstromig von der 5'-LTR fehlt.

8. Produzentenzellinie nach Anspruch 2, wobei dem retroviralen Vektorkonstrukt eine *env* codierende und/oder untranslatierte *env-*Sequenz aufstromig von der 3'-LTR fehlt.

9. Produzentenzellinie nach Anspruch 2, wobei dem retroviralen Vektorkonstrukt eine retrovirale Verpackungssignalsequenz abstromig von der 3'-LTR fehlt.

10. Produzentenzellinie nach Anspruch 2, wobei das retrovirale Vektorkonstrukt aus einem Retrovirus konstruiert ist, der aus der Gruppe ausgewählt ist, bestehend aus amphotropen, ecotropen, xenotropen oder polytropen Viren.

11. Produzentenzellinie nach Anspruch 2, wobei das retrovirale Vektorkonstrukt aus einem Maus-Leukämievirus konstruiert ist.

12. Produzentenzellinie nach Anspruch 2, wobei das retrovirale Vektorkonstrukt aus einem Lentivirus konstruiert ist.

13. Produzentenzellinie nach Anspruch 2, wobei das retrovirale Vektorkonstrukt weiterhin eine heterologe Sequenz umfaßt.

14. Produzentenzellinie nach Anspruch 13, wobei die heterologe Sequenz ein Gen ist, welches ein zytotoxisches Protein codiert.

15. Produzentenzellinie nach Anspruch 14, wobei das zytotoxische Protein aus der Gruppe ausgewählt ist, bestehend aus Ricin, Abrin, Diphtherietoxin, Choleratoxin, Gelonin, Kermesbeere, antiviralem Protein, Tritin, Shigellatoxin und Pseudomonas-Exotoxin A.

16. Produzentenzellinie nach Anspruch 13, wobei die heterologe Sequenz eine Antisense-Sequenz ist.

17. Produzentenzellinie nach Anspruch 13, wobei die heterologe Sequenz ein immunakzessorisches Molekül codiert.

18. Produzentenzellinie nach Anspruch 17, wobei das immunakzessorische Molekül aus der Gruppe ausgewählt ist, bestehend aus IL-1, IL-2, IL-3, IL-4, IL-5, IL-6, IL-7, IL-8, IL-9, IL-10, IL-11, IL-12, IL-13 und IL-15.

19. Produzentenzellinie nach Anspruch 17, wobei das immunakzessorische Molekül aus der Gruppe ausgewählt ist, bestehend aus ICAM-1, ICAM-2, b-Mikroglobin, LFA3, HLA-Klasse I- und HLA-Klasse II-Molekülen.

20. Produzentenzellinie nach Anspruch 13, wobei die heterologe Sequenz ein Genprodukt codiert, welches eine Verbindung mit wenig oder keiner Zytotoxizität zu einem toxischen Produkt aktiviert.

21. Produzentenzellinie nach Anspruch 20, wobei das Genprodukt aus der Gruppe ausgewählt ist, bestehend aus HSVTK, VZVTK und Cytosindesaminase.

22. Produzentenzellinie nach Anspruch 13, wobei die heterologe Sequenz ein Ribozym ist.

23. Produzentenzellinie nach Anspruch 13, wobei die heterologe Sequenz ein Ersatzgen ist.

24. Produzentenzellinie nach Anspruch 23, wobei das Ersatzgen ein Protein codiert, welches aus der Gruppe ausgewählt ist, bestehend aus Faktor VIII, ADA, HPRT, CF und dem LDL-Rezeptor.

25. Produzentenzellinie nach Anspruch 13, wobei die heterologe Sequenz einen immunogenen Abschnitt eines Virus codiert, ausgewählt aus der Gruppe, bestehend aus HBV, HCV, HPV, EBV, FeLV, FIV und HIV.

26. Produzentenzellinie nach Anspruch 1, wobei die gag/pol-Expressionskassette einen Promotor, der funktionsfähig mit einem *gag*/*pol*-Gen verknüpft ist, und eine Polyadenylierungssequenz umfaßt, wobei ein 3'-terminales Ende des *gag*/*pol*-Gens deletiert wurde, ohne die biologische Aktivität von Integrase zu beeinflussen.

27. Produzentenzellinie nach Anspruch 26, wobei das 3'-terminale Ende aufstromig von Nukleotid 5751 von Sequenz ID Nr. 1 deletiert wurde.

28. Produzentenzellinie nach Anspruch 26, wobei der Promotor ein heterologer Promotor ist.

29. Produzentenzellinie nach Anspruch 26, wobei der Promotor aus der Gruppe ausgewählt ist, bestehend aus CMV IE, dem HSVTK-Promotor, dem RSV-Promotor, dem starken späten Adenovirus-Promotor und dem SV40-Promotor.

30. Produzentenzellinie nach Anspruch 26, wobei die Polyadenylierungssequenz eine heterologe Polyadenylierungssequenz ist.

31. Produzentenzellinie nach Anspruch 30, wobei die heterologe Polyadenylierungssequenz aus der Gruppe ausgewählt ist, bestehend aus dem späten SV40-poly A-Signal, dem frühen SV40-poly A-Signal und einem Rinderwachstumshormon-poly A-Signal.

32. Produzentenzellinie nach Anspruch 1, wobei die *env-*Expressionskassette einen Promotor, der funktionsfähig mit einem *env*-Gen verknüpft ist, und eine Polyadenylierungssequenz umfaßt, wobei nicht mehr als 6 aufeinanderfolgende retrovirale Nukleotide aufstromig von dem *env-*Gen aufgenommen sind.

33. Produzentenzellinie nach Anspruch 1, wobei die *env*-Expressionskassette einen Promotor, der funktionsfähig mit einem *env-*Gen verknüpft ist, und eine Polyadenylierungssequenz umfaßt, wobei die *env-*Expressionskassette keine aufeinanderfolgende Sequenz von mehr als 8 Nukleotiden, die in einem *gag*/*pol*-Gen zu finden sind, enthält.

34. Produzentenzellinie nach Anspruch 1, wobei die *env*-Expressionskassette einen Promotor, der funktionsfähig mit einem *env*-Gen verknüpft ist, und eine Polyadenylierungssequenz umfaßt, wobei ein 3'-terminales Ende des *env*-Gens deletiert wurde, ohne die biologische Aktivität von *env* zu beeinflussen.

35. Produzentenzellinie nach Anspruch 34, wobei ein 3'-terminales Ende des *env-*Gens deletiert wurde, so daß durch die Expressionskassette ein vollständiges R-Peptid nicht erzeugt wird.

36. Produzentenzellinie nach Anspruch 34, wobei das *env*-Gen von einem Typ C-Retrovirus abgeleitet ist und wobei das 3'-terminale Ende deletiert wurde, so daß das *env*-Gen weniger als 18 Nukleinsäuren, die das R-Peptid codieren, enthält.

37. Produzentenzellinie nach Anspruch 34, wobei der Promotor ein heterologer Promotor ist.

38. Produzentenzellinie nach Anspruch 37, wobei der Promotor aus der Gruppe ausgewählt ist, bestehend aus CMV IE, dem HSVTK-Promotor, dem RSV-Promotor, dem starken späten Adenovirus-Promotor und dem SV40-Promotor.

39. Produzentenzellinie nach Anspruch 34, wobei die Polyadenylierungssequenz eine heterologe Polyadenylierungssequenz ist.

40. Produzentenzellinie nach Anspruch 39, wobei die heterologe Polyadenylierungssequenz aus der Gruppe ausgewählt ist, bestehend aus dem späten SV40-poly A-Signal, dem frühen SV40-poly A-Signal und einer Rinderwachstumshormon-Polyadenylierungssequenz.

## Revendications

1. Lignée cellulaire productrice, comprenant une cassette d'expression *gag*/*pol,* une cassette d'expression *env* et un vecteur rétroviral construit, dans laquelle il n'y a pas de chevauchement de séquences entre la cassette d'expression *gag*/*pol* et la cassette d'expression *env*, ni de chevauchement de séquences entre la cassette d'expression *env* et le vecteur rétroviral construit, à condition que ledit vecteur rétroviral construit chevauche au moins 4 nucléotides d'une extrémité 5' terminale d'un gène *gag*/*pol* codé dans ladite cassette d'expression *gag*/*pol.*

2. Lignée cellulaire productrice selon la revendication 1, dans laquelle ledit vecteur rétroviral construit est un vecteur rétroviral construit comprenant une 5'-LRT, un site de liaison d'ARNt, un signal d'encapsidation, une origine de synthèse de second brin d'ADN et une 3'-LRT, dans laquelle ledit vecteur construit contient des séquences codantes *gag*/*pol* qui ont été modifiées pour contenir un, deux ou plus de deux codons d'arrêt.

3. Lignée cellulaire productrice selon la revendication 2, dans laquelle l'un des codons d'arrêt élimine le site d'initiation.

4. Lignée cellulaire productrice selon la revendication 2 ou 3, dans laquelle l'un des codons d'arrêt comporte deux ou trois substitutions de paires de bases.

5. Lignée cellulaire productrice selon la revendication 2, dans laquelle ledit vecteur rétroviral construit est dépourvu d'un signal d'encapsidation étendu.

6. Lignée cellulaire productrice selon la revendication 2, dans laquelle ledit vecteur rétroviral construit est dépourvu d'une séquence d'acide nucléique rétroviral en amont de ladite 5'-LRT.

7. Lignée cellulaire productrice selon la revendication 6, dans laquelle ledit vecteur rétroviral construit est dépourvu d'une séquence codante *env* en amont de ladite 5'-LRT.

8. Lignée cellulaire productrice selon la revendication 2, dans laquelle ledit vecteur rétroviral construit est dépourvu d'une séquence codante *env* et/ou *env* non traduite en amont de ladite 3'-LRT.

9. Lignée cellulaire productrice selon la revendication 2, dans laquelle ledit vecteur rétroviral construit est dépourvu d'une séquence signal d'encapsidation rétrovirale en aval de ladite 3'-LRT.

10. Lignée cellulaire productrice selon la revendication 2, dans laquelle ledit vecteur rétroviral construit est construit à partir d'un rétrovirus choisi dans le groupe formé par les virus amphotropes, écotropes, xénotropes ou polytropes.

11. Lignée cellulaire productrice selon la revendication 2, dans laquelle ledit vecteur rétroviral construit est construit à partir du Virus de la Leucémie Murine.

12. Lignée cellulaire productrice selon la revendication 2, dans laquelle ledit vecteur rétroviral construit est construit à partir d'un lentivirus.

13. Lignée cellulaire productrice selon la revendication 2, dans laquelle ledit vecteur rétroviral construit comprend de plus une séquence hétérologue.

14. Lignée cellulaire productrice selon la revendication 13, dans laquelle ladite séquence hétérologue est un gène codant pour une protéine cytotoxique.

15. Lignée cellulaire productrice selon la revendication 14, dans laquelle ladite protéine cytotoxique est choisie dans le groupe formé par la ricine, l'abrine, la toxine diphtérique, la toxine cholérique, la gélonine, le pokeweed, une protéine antivirale, la tritine, la toxine de *Shigella* et l'exotoxine A de *Pseudomonas.*

16. Lignée cellulaire productrice selon la revendication 13, dans laquelle ladite séquence hétérologue est une séquence antisens.

17. Lignée cellulaire productrice selon la revendication 13, dans laquelle ladite séquence hétérologue code pour une molécule immunitaire accessoire.

18. Lignée cellulaire productrice selon la revendication 17, dans laquelle ladite molécule immunitaire accessoire est choisie dans le groupe formé par IL-1, IL-2, IL-3, IL-4, IL-5, IL-6, IL-7, IL-8, IL-9, IL-10, IL-11, IL-12, IL-13 et IL-15.

19. Lignée cellulaire productrice selon la revendication 17, dans laquelle ladite molécule immunitaire accessoire est choisie dans le groupe formé par ICAM-1, ICAM-2, β-microglobine, LFA3, molécules de la classe I du HLA et de la classe II du HLA.

20. Lignée cellulaire productrice selon la revendication 13, dans laquelle ladite séquence hétérologue code pour un produit de gène qui active en un produit toxique un composé ayant peu ou n'ayant pas de cytotoxicité.

21. Lignée cellulaire productrice selon la revendication 20, dans laquelle ledit produit de gène est choisi dans le groupe formé par HSVTK, VZVTK et la cytosine-désaminase.

22. Lignée cellulaire productrice selon la revendication 13, dans laquelle ladite séquence hétérologue est un ribozyme.

23. Lignée cellulaire productrice selon la revendication 13, dans laquelle ladite séquence hétérologue est un gène de remplacement.

24. Lignée cellulaire productrice selon la revendication 23, dans laquelle ledit gène de remplacement code pour une protéine choisie dans le groupe formé par le Facteur VIII, ADA, HPRT, CF et le récepteur des LDL.

25. Lignée cellulaire productrice selon la revendication 13, dans laquelle ladite séquence hétérologue code pour une portion immunogène d'un virus choisi dans le groupe formé par HBV, HCV, HPV, EBV, FeLV, FIV et HIV.

26. Lignée cellulaire productrice selon la revendication 1, dans laquelle ladite cassette d'expression *gag*/*pol* comprend un promoteur enchaîné fonctionnellement à un gène *gag*/*pol,* et une séquence de polyadénylation, dans laquelle une extrémité 3'-terminale dudit gène *gag*/*pol* a été délétée sans affecter l'activité biologique d'intégrase.

27. Lignée cellulaire productrice selon la revendication 26, dans laquelle ladite extrémité 3'-terminale a été délétée en amont du nucléotide 5751 de la Séquence ID N° 1.

28. Lignée cellulaire productrice selon la revendication 26, dans laquelle ledit promoteur est un promoteur hétérologue.

29. Lignée cellulaire productrice selon la revendication 26, dans laquelle ledit promoteur est choisi dans le groupe formé par CMV-IE, le promoteur de HSVTK, le promoteur de RSV, le promoteur majeur tardif de l'adénovirus et le promoteur de SV40.

30. Lignée cellulaire productrice selon la revendication 26, dans laquelle ladite séquence de polyadénylation est une séquence de polyadénylation hétérologue.

31. Lignée cellulaire productrice selon la revendication 30, dans laquelle ladite séquence de polyadénylation hétérologue est choisie dans le groupe formé par le signal poly-A tardif de SV40, le signal poly-A précoce de SV40 et le signal poly-A de l'hormone de croissance bovine.

32. Lignée cellulaire productrice selon la revendication 1, dans laquelle ladite cassette d'expression *env* comprend un promoteur enchaîné fonctionnellement à un gène *env,* et une séquence de polyadénylation, dans laquelle pas plus de 6 nucléotides rétroviraux consécutifs ne sont inclus en amont dudit gène *env*.

33. Lignée cellulaire productrice selon la revendication 1, dans laquelle ladite cassette d'expression *env* comprend un promoteur enchaîné fonctionnellement à un gène *env*, et une séquence de polyadénylation, dans laquelle ladite cassette d'expression *env* ne contient pas une séquence consécutive de plus de 8 nucléotides qui se trouvent dans un gène *gag*/*pol.*

34. Lignée cellulaire productrice selon la revendication 1, dans laquelle ladite cassette d'expression env comprend un promoteur enchaîné fonctionnellement à un gène *env*, et une séquence de polyadénylation, dans laquelle une extrémité 3'-terminale dudit gène *env* a été délétée sans affecter l'activité biologique de *env*.

35. Lignée cellulaire productrice selon la revendication 34, dans laquelle une extrémité 3'-terminale dudit gène *env* a été délétée en sorte qu'un peptide R complet ne soit pas produit par ladite cassette d'expression.

36. Lignée cellulaire productrice selon la revendication 34, dans laquelle ledit gène *env* est dérivé d'un rétrovirus de type C, et dans laquelle l'extrémité 3'-terminale a été délétée en sorte que ledit gène *env* comprenne moins de 18 acides nucléiques codant pour ledit peptide R.

37. Lignée cellulaire productrice selon la revendication 34, dans laquelle ledit promoteur est un promoteur hétérologue.

38. Lignée cellulaire productrice selon la revendication 37, dans laquelle ledit promoteur est choisi dans le groupe formé par CMV-IE, le promoteur de HSVTK, le promoteur de RSV, le promoteur majeur tardif de l'adénovirus et le promoteur de SV40.

39. Lignée cellulaire productrice selon la revendication 34, dans laquelle ladite séquence de polyadénylation est une séquence de polyadénylation hétérologue.

40. Lignée cellulaire productrice selon la revendication 39, dans laquelle ladite séquence de polyadénylation hétérologue est choisie dans le groupe formé par le signal poly-A tardif de SV40, le signal poly-A précoce de SV40 et une séquence de polyadénylation d'hormone de croissance bovine.
